# EUROPEAN PATENT SPECIFICATION

(11) **EP 1 648 906 B1**
(45) Date of publication and mention of the grant of the patent: **03.10.2007**
(21) Application number: 04742977.4
(22) Date of filing: 18.06.2004
(51) Int. Cl.: C07F 7/08, A61K 31/695, A61P 35/00, A61P 31/00, A61P 9/00

(54) **ORGANOSILICON COMPOUNDS AND THEIR USE**
ORGANOSILIZIUMVERBINDUNGEN UND DEREN VERWENDUNG
COMPOSES ORGANOSILICIES ET LEUR UTILISATION

(30) Priority: 09.07.2003 GB 0316090; 10.02.2004 GB 0402903; 18.05.2004 GB 0411071
(43) Date of publication of application: 26.04.2006
(73) Proprietor: Takeda Cambridge Limited, Milton Road Cambridge Cambridgeshire CB4 0PA (GB)
(72) Inventor: MILLER, David John, Paradigm Therapeutics Ltd., Cambridge CB4 0GP (GB); SHOWELL, Graham Andrew, Paradigm Therapeutics Ltd., Cambridge CB4 0GP (GB); CONROY, Richard, Paradigm Therapeutics, Ltd., Cambridge CB4 0GP (GB); DAISS, Juergen, Julius-Maximilians-Universitaet, 97070 Wuerzburg (DE); TACKE, Reinhold, Julius-Maximilians-Universitaet, 97070 Wuerzburg (DE); TEBBE, David, Julius-Maximilians-Universitaet, 97070 Wuerzburg (DE)
(74) Representative: Perry, Robert Edward
(86) International application number: PCT/GB2004/002622
(87) International publication number: WO 2005/005443

(56) References cited:
- WO-A-00/20358
- WO-A-03/106446
- WO-A-20/04045625

## Description

### Field of the Invention

This invention relates to compounds and their use in therapy.

### Background to the Invention

Gonadotropin-Reieasing Hormone (GnRH) plays a key role in the biology of reproduction. GnRH is also known as luteinizing hormone-releasing hormone (LH-RH).

The GnRH decapeptide (pyro-Glu-His-Trp-Ser-Tyr-Gly-Leu-Art-Pro-Gly-NH₂ or p-EHWSYGLRPG-NH₂) is formed in neurons of the medial basal hypothalamus from a larger precursor via enzymatic processing. The peptide is released in a pulsatile manner into the pituitary portal circulation system, where GnRH interacts with high-affinity receptors (7-transmembrane G-protein coupled receptors) in the anterior pituitary gland located at the base of the brain. Here, GnRH triggers the release of luteinizing hormone (LH) and follicle-stimulating hormone (FSH), both of which are gonadotropic hormones (gonadotropins). LH stimulates the production of testosterone and estradiol in the testes and ovaries respectively, whilst FSH stimulates follicle growth in women and sperm formation in men. When correctly functioning, the pulsatile release and concentration levels of GnRH are critical for the maintaining of gonadal steroidogenesis and for normal functions of reproduction related to growth and sexual development.

The pituitary response to GnRH varies greatly throughout life. GnRH and the gonadotropins first appear in the foetus at about ten weeks of gestation. Sensitivity to GnRH reduces until the onset of puberty. There is, however, a brief rise during the first three months after birth. Prior to puberty, the FSH response to GnRH is greater than that of LH. Once puberty begins, sensitivity to GnRH increases, and pulsatile LH secretion ensues. Later in puberty and throughout the reproductive years, pulsatile release of GnRH occurs throughout the day, with responsiveness to LH being greater than that of FSH. Pulsatile GnRH release results in pulsatile LH and FSH release and thus testosterone and estradiol release from the gonads. Post-menopause, the concentration of FSH an LH rise, and the post-menopausal levels of FSH are higher than those of LH.

Chronic administration of GnRH agonists and antagonists results in decreased circulating levels of both LH and FSH. GnRH agonists are compounds that mimic endogenous GnRH to stimulate receptors on the pituitary gland, resulting in release of LH and FSH. After a transient rise in gonadal hormone production ("flare" response), the chronic administration of GnRH agonists results in down-regulation of the GnRH receptors. This down-regulation and desensitization results in a reduction in the circulating levels of LH and FSH. In spite of the symptom-exacerbating hormonal flare experienced, GnRH agonists have been the preferred treatment for sex-steroid-dependent pathophysiologies. GnRH agonists have been used to reduce testosterone production, thereby reducing prostate volume in benign prostatic hyperplasia (BPH) and slowing tumour growth in prostate cancer. Such compounds have also been used in the treatment of breast and ovarian cancers.

In recent years, GnRH antagonists have become available for clinical evaluation, and have been shown to have an immediate effect on the pituitary but without the observed flare associated with agonists. Use of GnRH antagonists has been reported for the treatment of ovarian, breast and prostate cancers.

Other uses of antagonists include endometriosis (including endometriosis with pain), uterine myoma, ovarian and mammary cystic diseases (including polycystic ovarian disease), prostatic hypertrophy, amenorrhea (e.g. secondary amenorrhea), and precocious puberty. These compounds may also be useful in the symptomatic relief of premenstrual syndrome (PMS). Antagonists may also be useful to regulate the secretion of gonadotropins in male mammals to arrest spermatogenesis (e.g. as male contraceptives), and for treatment of male sex offenders. GnRH antagonists and agonists have been shown to have utility in treatments where a reversible suppression of the pituitary-gonadal axis is desired.

The presence of GnRH receptors on anterior pituitary cells and several tumour cell types offers the opportunity to develop drugs that act upon receptors to treat both hormone-dependent and hormone-independent cancers.

Conventionally, androgen deprivation has been the most effective systematic therapy for the treatment of metastatic carcinoma of the prostate. The prostate gland requires androgens for normal growth, maintenance, and function. Prostate cancer and benign prostate hyperplasia, however, are common in men and develop in an environment of continuous exposure to androgen. Utilizing a GnRH antagonist to interrupt the pituitary-gonadal axis reduces androgen production and results in tumour growth modulation.

GnRH antagonists may have a direct effect on tumour growth by blocking receptors on the tumour cells. For those cancer types that respond both to sex hormones and to GnRH directly, antagonists should be effective in slowing tumour growth by two mechanisms. Since GnRH receptors are present on many prostate and breast cancer cells, it has recently been proposed that GnRH antagonists may also be effective in treating non-hormone-dependent tumours. Recent literature examples indicate that GnRH receptors are present on a number of cancer cell lines. In particular, prostate, ovarian and breast cancers (see for example Montagnani et al., Arch. Ital, Urol. Androl. 1997, 69(4), 257-263; Jungwirth et al., Prostate 1997, 32(3), 164-172; Srkalovic et al., Int. J. Oncol. 1998, 12(3), 489-498; Kottler et al., Int. J. Cancer 1997, 71(4), 595-599.

Available GnRH antagonists have primarily been peptide analogues of GnRH (see, for example, WO93/03058). Peptide antagonists of peptide hormones have some potency but, the use of current peptide antagonists is often associated with problems because peptides are degraded by physiological enzymes and often poorly distributed within the organism being treated. They thus have a limited effectiveness as drugs.

WO00/20358 discloses non-peptide analogues of GnRH.

Sila-substitution (C/Si-exchange) of drugs is a relatively recent approach for searching for organo-silicon compounds which have beneficial biological properties. The approach involves the replacement of specific carbon atoms in compounds by silicon, and monitoring how the biological properties of the compounds have changed. A review of this approach is provided in Tacke and Zilch, Endeavour, New Series, 10, 191-197 (1986).

### Summary of the Invention

A first aspect of the invention is a compound of formula (I) or formula (II): 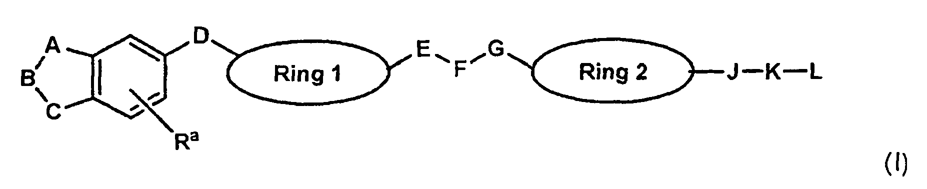 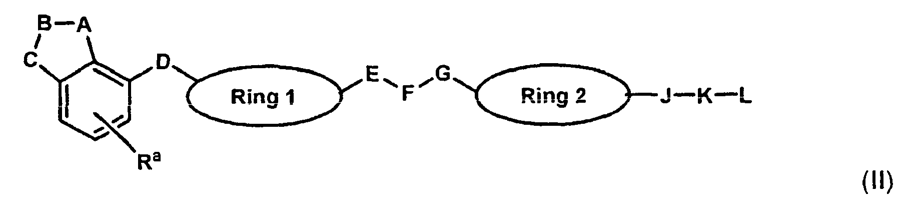 wherein
A and C are the same or different and are each a bond, -(CH₂)ₙ-, -C(R^{b})₂-, -Si(R^{c})₂-, -O-, -S(O)ₘ-, -N=, -N(R^{b})-, -N(R^{e})C(=X)- or -C(=X)-;
B is -(CH₂)ₙ-, -O-, -C(R^{b})₂-, -Si(R^{c})₂-, -C(R^{b})=C(R^{b})-, -C(R^{b})=, -(CH₂)ₙC(R^{g})₂-, -C(R⁹)₂(CH₂)ₙ- or -CH(R^{b})CH(R^{b})-;
wherein any of A, B and C is optionally substituted with -Si(R^{c})₃;
D is -(CH₂)ₙ-, -C(=X)-, -O-, -S(O)ₘ-, -C(=X)N(R^{e})-, -C(R^{b})₂-,-C(R^{b})=C(R^{b})-, -CH(R^{b})CH(R^{b})-;
E is optionally present and is -(CH₂)ₙ-, -N(R^{d})-, -(CH₂)ₙN(R^{d})- or -N(R^{d})(CH₂)ₙ-;
F is -C(=X)- or -N(R^{d})-;
G is -(CH₂)ₙ-, -N(R^{d})-, -(CH₂)ₙN(R^{d})- or -N(R^{d})(CH₂)ₙ;
J is optionally present and is -O-, -N(R^{c})C(=X)-, -C(=X)N(R^{c})-, -S(O)ₘ-, -N(R^{c})S(O)ₘ-, -S(O)ₘN(R^{c})- or -N(R^{e})-;
K is optionally present and is alkylene optionally substituted with R^{b}; or K is cycloalkylene, cycloalkenylene, arylene, heterocycloalkylene, heterocycloalkylene or heteroarylene, any of which is optionally substituted with R^{a};
L is hydrogen, halogen, -N(R^{f})₂, cycloalkyl, cycloalkenyl, aryl, heterocycloalkyl, heterocycloalkenyl or heteroaryl, any of which is optionally substituted with R^{a}, -C(=X)OR^{d}, -OH, -OR^{c}, -C(=X)N(R^{b})(R^{c}), -S(O)ₘN(R^{b})(R^{c}) or -CN;
each R^{a} is the same or different and is hydrogen, halogen, alkyl, aryl, hydroxy, alkoxy, -alkoxy-(CH₂)ₙC(O)₂R^{b}, -O-aryl, -C(=X)R^{c}, -NO₂, -CN, -N(R^{c})C(=X)R^{c}, -C(=X)N(R^{c})₂, -S(O)₂N(R^{c})₂ or -N(R^{e})₂;
each R^{b} is the same or different and is hydrogen or alkyl;
each R^{c} is the same or different and is alkyl, cycloalkyl, -alkyl-aryl, -alkyl-cycloalkyl or aryl optionally substituted with R^{a};
each R^{d} is the same or different and is hydrogen, alkyl or aryl optionally substituted with R^{a};
each R^{e} is the same or different and is hydrogen or alkyl; or R^{e} is aryl or heteroaryl, either of which is optionally substituted with R^{a};
each R^{f} is the same or different and is hydrogen or alkyl; or R^{f}-N-R^{f} taken together form heterocycloalkyl, heterocycloalkenyl or heteroaryl;
R^{g}-C-R^{g} taken together form heterocyclyl;
each X is the same or different and is oxygen or sulphur;
Rings 1 and 2 are the same or different and are each arylene or heteroarylene, either of which is optionally substituted with R^{a};
each m is the same or different and is 0, 1 or 2; and
each n is the same or different and is 0, 1, 2, or 3;
with the provisos that at least one of A, B and C comprises a silicon atom; A and C are not each a bond; and the compound does not comprise a Si-Si bond, a N-N single bond, a Si-O bond, a Si-N bond or a N-O-N linkage;
or a pharmaceutically acceptable salt thereof.

Compounds of the invention may act as GnRH antagonists and, as a result, may have therapeutic utility in cancer therapy or in the treatment or prevention of endometriosis, uterine myoma, an ovarian disease, a mammary cystic disease, prostatic hypertrophy, amenorrhea, precocious puberty, premenstrual syndrome, a sex-steroid-dependent pathophysiology, benign prostatic hyperplasia, Alzheimer's disease, HIV infection, AIDS or a disease caused by thyroid malfunction, or to arrest spermatogenesis.

Accordingly, another aspect of the invention is the use of a compound of the invention for the manufacture of a medicament for cancer therapy or for the treatment or prevention of endometriosis, uterine myoma, an ovarian disease, a mammary cystic disease, prostatic hypertrophy, amenorrhea, precocious puberty, premenstrual syndrome, a sex-steroid-dependent pathophysiology, benign prostatic hyperplasia, Alzheimer's disease, HIV infection, AIDS or a disease caused by thyroid malfunction, or to arrest spermatogenesis.

Another aspect of the invention is a pharmaceutical composition comprising a compound of the invention and a pharmaceutically acceptable diluent or carrier.

### Description of the Invention

Certain compounds and combinations of substituents are preferred; in particular see the subclaims.

The term "alkyl" as used herein refers to an optionally substituted straight or branched chain alkyl moiety having from one to six carbon atoms. The term includes, for example, methyl, ethyl, propyl, isopropyl, butyl, tert-butyl, pentyl, hexyl and the like. The substituents may be the same or different in each occurrence and selected from halogen and the like. "C₁₋₆ alkyl" has the same meaning. "Alkylene" refers to a similar, divalent group.

The term "alkoxy" as used herein refers to an optionally substituted straight or branched chain alkoxy group containing one to six carbon atoms. The term includes, for example, methoxy, ethoxy, propoxy, isopropoxy, butoxy, tert-butoxy, pentoxy, hexoxy and the like. The substituents may be the same or different in each occurrence and selected from halogen and the like. "C₁₋₆ alkoxy" has the same meaning.

The term "halogen" as used herein refers to F, Cl, Br or I.

The term "aryl" as used herein refers to optionally substituted aromatic ring systems comprising six to ten ring atoms, and optionally substituted polycyclic ring systems having two or more cyclic rings at least one of which is aromatic. This term includes, for example, phenyl and naphthyl. The group may be optionally substituted with the substituents being the same or different in each occurrence and selected from R^{a} and the like. "Arylene" refers to a similar, divalent group.

The term "cycloalkyl" as used herein refers to a saturated alicyclic moiety having from three to six carbon atoms. The term includes, for example, cyclopropyl, cyclobutyl, cyclopentyl, cyclohexyl and the like. The group may be optionally substituted by any substituent described herein. "Cycloalkylene" refers to a similar, divalent group.

The term "cycloalkenyl" as used herein refers to an alicyclic moiety having from three to six carbon atoms and having in addition at least one double bond. The term includes, for example, cyclopentenyl, cyclohexenyl and the like. The group may be optionally substituted by any substituent described herein. "Cycloalkenylene" refers to a similar, divalent group.

The term "heterocycloalkyl" as used herein refers to a saturated heterocyclic moiety having from three to seven carbon atoms and one or more heteroatoms selected from the group N, O, S, P and Si. The term includes, for example, azetidinyl, pyrrolidinyl, tetrahydrofuranyl, piperidinyl and the like. The group may be optionally substituted by any substituent described herein. "Heterocycloalkylene" refers to a similar, divalent group.

The term "heteroaryl" as used herein refers to aromatic ring systems of five to ten atoms at least one atom of which is selected from O, N and S. The term includes, for example, furanyl, thiophenyl, pyridyl, indolyl, quinolyl and the like. The group may be optionally substituted with R^{a} and the like. "Heteroarylene" refers to a similar, divalent group.

The term "heterocyclyl" as used herein refers to a saturated or unsaturated heterocyclic ring moiety having from three to seven carbon atoms and one or more heteroatoms selected from N, O, S, P and Si. The term includes, for example, piperidinyl, pyrrolidinyl, morpholinyl and the like. The group may be polycyclic (e.g. a fused ring system), the group comprising two or more rings, at least one of which comprises a heteroatom.

Preferred compounds of the invention include:
5-(1,1,4,4,7-pentamethyl-1,4-disila-1,2,3,4-tetrahydronaphthalen-6-yloxy)-*N*-(2,6-dimethoxyphenyl)furan-2-carboxamide;
5-(1,1,4,4,7-pentamethyl-1,4-disila-1,2,3,4-tetrahydronaphthalen-6-yloxy)-*N*-(2,4,6-trimethoxyphenyl)furan-2-carboxamide;
5-(1,1,4,4,7-pentamethyl-1,4-disila-1,2,3,4-tetrahydronaphthalen-6-yloxy)-*N*-(2,4,6-trimethoxy-1,3-pyrimidin-5-yl)furan-2-carboxamide;
5-(1,1,4,4,7-pentamethyl-1,4-disila-1,2,3,4-tetrahydronaphthalen-6-yloxy)-*N*-(2-methylamino-4,6-dimethoxy-1,3-pyrimidin-5-yl)furan-2-carboxamide;
5-(1,1,4,4,7-pentamethyl-1,4-disila-1,2,3,4-tetrahydronaphthalen-6-yloxy)-*N*-{2-[3-(4-methylpiperazin-1-yl)propylamino]-4,6-dimethoxy-1,3-pyrimidin-5-yl}furan-2-carboxamide;
5-(1,1,4,4,7-pentamethyl-1,4-disila-1,2,3,4-tetrahydronaphthalen-6-yloxy)-*N*-{2-[3-(*N,N*-dimethylamino)propylamino]-4,6-dimethoxy-1,3-pyrimidin-5-yl}furan-2-carboxamide;
5-(1,1,4,4,7-pentamethyl-1,4-disila-1,2,3,4-tetrahydronaphthalen-6-yloxy)-*N*-{2-[3-(morpholin-4-yl)propylamino]-4,6-dimethoxy-1,3-pyrimidin-5-yl}furan-2-carboxamide;
5-(1,1,6-trimethyl-1-sila-1,2,3,4-tetrahydronaphthalen-7-yloxy)-*N*-(2,6-dimethoxyphenyl)furan-2-carboxamide;
5-(1,1,6-trimethyl-1-sila-1,2,3,4-tetrahydronaphthalen-7-yloxy)-*N*-(2,4,6-trimethoxyphenyl)furan-2-carboxamide;
5-(1,1,6-trimethyl-1-sila-1,2,3,4-tetrahydronaphthalen-7-yloxy)-*N*-(2,4,6-trimethoxy-1,3-pyrimidin-5-yl)furan-2-carboxamide;
5-(1,1,6-trimethyl-1-sila-1,2,3,4-tetrahydronaphthalen-7-yloxy)-*N*-(2-methylamino-4,6-dimethoxy-1,3-pyrimidin-5-yl)furan-2-carboxamide;
5-(1,1,6-trimethyl-1-sila-1,2,3,4-tetrahydronaphthalen-7-yloxy)-*N*-{2-[3-(4-methylpiperazin-1-yl)propylamino]-4,6-dimethoxy-1,3-pyrimidin-5-yl}furan-2-carboxamide;
5-(1,1,6-trimethyl-1-sila-1,2,3,4-tetrahydronaphthalen-7-yloxy)-*N*-{2-[3-(*N,N*-dimethylamino)propylamino]-4,6-dimethoxy-1,3-pyrimidin-5-yl}furan-2-carboxamide;
5-(1,1,6-trimethyl-1-sila-1,2,3,4-tetrahydronaphthalen-7-yloxy)-*N*-{2-[3-(morpholin-4-yl)propylamino]-4,6-dimethoxy-1,3-pyrimidin-5-yl}furan-2-carboxamide;
5-(1,1,7-trimethyl-1-sila-1,2,3,4-tetrahydronaphthalen-6-yloxy)-*N*-(2,6-dimethoxyphenyl)furan-2-carboxamide;
5-(1,1,7-trimethyt-1-sita-1,2,3,4-tetrahydronaphthalen-6-ytoxy)-*N*-(2,4,6-trimethoxyphenyl)furan-2-carboxamide;
5-(1,1,7-trimethyl-1-sila-1,2,3,4-tetrahydronaphthalen-6-yloxy)-*N*-(2,4,6-trimethoxy-1,3-pyrimidin-5-yl)furan-2-carboxamide;
5-(1,1,7-trimethyl-1-sila-1,2,3,4-tetrahydronaphthalen-6-yloxy)-*N*-(2-methylamino-4,6-dimethoxy-1,3-pyrimidin-5-yl)furan-2-carboxamide;
5-(1,1,7-trimethyl-1-sila-1,2,3,4-tetrahydronaphthalen-6-yloxy)-*N*-{2-[3-(4-methylpiperazin-1-yl)propylamino]-4,6-dimethoxy-1,3-pyrimidin-5-yl}furan-2-carboxamide;
5-(1,1,7-trimethyl-1-sila-1,2,3,4-tetrahydronaphthalen-6-yloxy)-*N*-{2-[3-(*N,N*-dimethylamino)propylamino]-4,6-dimethoxy-1,3-pyrimidin-5-yl}furan-2-carboxamide;
5-(1,1,7-trimethyl-1-sila-1,2,3,4-tetrahydronaphthalen-6-yloxy)-*N*-{2-[3-(morpholin-4-yl)propylamino]-4,6-dimethoxy-1,3-pyrimidin-5-yl}furan-2-carboxamide;
5-(1,1,2,4,4,7-hexamethyl-1,4-disila-1,2,3,4-tetrahydronaphthalen-6-yloxy)-*N*-(2,6-dimethoxyphenyl)furan-2-carboxamide;
5-(1,1,2,4,4,7-hexamethyl-1,4-disila-1,2,3,4-tetrahydronaphthalen-6-yloxy)-*N*-(2,4,6-trimethoxyphenyl)furan-2-carboxamide;
5-(1,1,2,4,4,7-hexamethyl-1,4-disila-1,2,3,4-tetrahydronaphthalen-6-yloxy)-*N*-(2,4,6-trimethoxy-1,3-pyrimidin-5-yl)furan-2-carboxamide;
5-(1,1,2,4,4,7-hexamethyl-1,4-disila-1,2,3,4-tetrahydronaphthalen-6-yloxy)-*N*-(2-methylamino-4,6-dimethoxy-1,3-pyrimidin-5-yl)furan-2-carboxamide;
5-(1,1,2,4,4,7-hexamethyl-1,4-disila-1,2,3,4-tetrahydronaphthalen-6-yloxy)-*N*-{2-[3-(4-methylpiperazin-1-yl)propylamino]-4,6-dimethoxy-1,3-pyrimidin-5-yl}furan-2-carboxamide;
5-(1,1,2,4,4,7-hexamethyl-1,4-disila-1,2,3,4-tetrahydronaphthalen-6-yloxy)-*N*-{2-[3-(*N*,*N*-dimethylamino)propylamino]-4,6-dimethoxy-1,3-pyrimidin-5-yl}furan-2-carboxamide;
5-(1,1,2,4,4,7-hexamethyl-1,4-disila-1,2,3,4-tetrahydronaphthalen-6-yloxy)-*N*-{2-[3-(morpholin-4-yl)propylamino]-4,6-dimethoxy-1,3-pyrimidin-5-yl}furan-2-carboxamide;
5-(5-methoxy-1,1,4,4,7-pentamethyl-1,4-disila-1,2,3,4-tetrahydronaphthalen-6-yloxy)-*N*-(2,6-dimethoxyphenyl)furan-2-carboxamide;
5-(5-methoxy-1,1,4,4,7-pentamethyl-1,4-disila-1,2,3,4-tetrahydronaphthalen-6-yloxy)-*N*-(2,4,6-trimethoxyphenyl)furan-2-carboxamide;
5-(5-methoxy-1,1,4,4,7-pentamethyl-1,4-disila-1,2,3,4-tetrahydronaphthalen-6-yloxy)-*N*-(2,4,6-trimethoxy-1,3-pyrimidin-5-yl)furan-2-carboxamide;
5-(5-methoxy-1,1,4,4,7-pentamethyl-1,4-disila-1,2,3,4-tetrahydronaphthalen-6-yloxy)-*N*-(2-methylamino-4,6-dimethoxy-1,3-pyrimidin-5-yl)furan-2-carboxamide;
5-(5-methoxy-1,1,4,4,7-pentamethyl-1,4-disila-1,2,3,4-tetrahydronaphthalen-6-yloxy)-*N*-{2-[3-(4-methylpiperazin-1-yl)propylamino]-4,6-trimethoxy-1,3-pyrimidin-5-yl}furan-2-carboxamide;
5-(5-methoxy-1,1,4,4,7-pentamethyl-1,4-disila-1,2,3,4-tetrahydronaphthalen-6-yloxy)-*N*-{2-[3-(*N*,*N*-dimethylamino)propylamino]-4,6-dimethoxy-1,3-pyrimidin-5-yl}furan-2-carboxamide;
5-(5-methoxy-1,1,4,4,7-pentamethyl-1,4-disila-1,2,3,4-tetrahydronaphthalen-6-yloxy)-*N*-{2-[3-(morpholin-4-yl)propylamino]-4,6-dimethoxy-1,3-pyrimidin-5-yl}furan-2-carboxamide;
5-(8-methoxy-1,1,6-trimethyl-1-sila-1,2,3,4-tetrahydronaphthalen-7-yloxy)-*N*-(2,6-dimethoxyphenyl)furan-2-carboxamide;
5-(8-methoxy-1,1,6-trimethyl-1-sila-1,2,3,4-tetrahydronaphthalen-7-yloxy)-*N*-(2,4,6-trimethoxyphenyl)furan-2-carboxamide;
5-(8-methoxy-1,1,6-trimethyl-1-sila-1,2,3,4-tetrahydronaphthalen-7-yloxy)-*N*-(2,4,6-trimethoxy-1,3-pyrimidin-5-yl)furan-2-carboxamide;
5-(8-methoxy-1,1,6-trimethyl-1-sila-1,2,3,4-tetrahydronaphthalen-7-yloxy)-*N*-(2-methylamino-4,6-dimethoxy-1,3-pyrimidin-5-yl)furan-2-carboxamide;
5-(8-methoxy-1,1,6-trimethyl-1-sila-1,2,3,4-tetrahydronaphthalen-7-yloxy)-*N*-{2-[3-(4-methylpiperazin-1-yl)propylamino]-4,6-dimethoxy-1,3-pyrimidin-5-yl}furan-2-carboxamide;
5-(8-methoxy-1,1,6-trimethyl-1-sila-1,2,3,4-tetrahydronaphthalen-7-yloxy)-*N*-{2-[3-(*N,N*-dimethylamino)propylamino]-4,6-dimethoxy-1,3-pyrimidin-5-yl}furan-2-carboxamide;
5-(8-methoxy-1,1,6-trimethyl-1-sila-1,2,3,4-tetrahydronaphthalen-7-yloxy)-*N*-{2-[3-(morpholin-4-yl)propylamino]-4,6-dimethoxy-1,3-pyrimidin-5-yl}furan-2-carboxamide;
5-(1,1,4,4,5,7-hexamethyl-1,4-disila-1,2,3,4-tetrahydronaphthalen-6-yloxy)-*N*-(2,6-dimethoxyphenyl)furan-2-carboxamide;
5-(1,1,4,4,5,7-hexamethyl-1,4-disila-1,2,3,4-tetrahydronaphthalen-6-yloxy)-*N*-(2,4,6-trimethoxyphenyl)furan-2-carboxamide;
5-(1,1,4,4,5,7-hexamethyl-1,4-disila-1,2,3,4-tetrahydronaphthalen-6-yloxy)-*N*-(2,4,6-trimethoxy-1,3-pyrimidin-5-yl)furan-2-carboxamide;
5-(1,1,4,4,5,7-hexamethyl-1,4-disila-1,2,3,4-tetrahydronaphthalen-6-yloxy)-*N*-(2-methylamino-4,6-dimethoxy-1,3-pyrimidin-5-yl)furan-2-carboxamide;
5-(1,1,4,4,5,7-hexamethyl-1,4-disila-1,2,3,4-tetrahydronaphthalen-6-yloxy)-*N*-{2-[3-(4-methylpiperazin-1-yl)propylamino]-4,6-dimethoxy-1,3-pyrimidin-5-yl}furan-2-carboxamide;
5-(1,1,4,4,5,7-hexamethyl-1,4-disila-1,2,3,4-tetrahydronaphthalen-6-yloxy)-*N*-{2-[3-(*N,N*-dimethylamino)propylamino]-4,6-dimethoxy-1,3-pyrimidin-5-yl}furan-2-carboxamide;
5-(1,1,4,4,5,7-hexamethyl-1,4-disila-1,2,3,4-tetrahydronaphthalen-6-yloxy)-*N*-{2-[3-(morpholin-4-yl)propylamino]-4,6-dimethoxy-1,3-pyrimidin-5-yl}furan-2-carboxamide;
5-(6-methoxy-1,1,4,4,8-pentamethyl-1,4-disita-1,2,3,4-tetrahydronaphthalen-7-yloxy)-*N*-(2,6-dimethoxyphenyl)furan-2-carboxamide;
5-(6-methoxy-1,1,4,4,8-pentamethyl-1,4-disila-1,2,3,4-tetrahydronaphthalen-7-yloxy)-*N*-(2,4,6-trimethoxyphenyl)furan-2-carboxamide;
5-(6-methoxy-1,1,4,4,8-pentamethyl-1,4-disila-1,2,3,4-tetrahydronaphthalen-7-yloxy)-*N*-(2,4,6-trimethoxy-1,3-pyrimidin-5-yl)furan-2-carboxamide;
5-(6-methoxy-1,1,4,4,8-pentamethyl-1,4-disila-1,2,3,4-tetrahydronaphthalen-7-yloxy)-*N*-(2-methylamino-4,6-dimethoxy-1,3-pyrimidin-5-yl)furan-2-carboxamide;
5-(6-methoxy-1,1,4,4,8-pentamethyl-1,4-disila-1,2,3,4-tetrahydronaphthalen-7-yloxy)-*N*-{2-[3-(4-methylpiperazin-1-yl)propylamino]-4,6-dimethoxy-1,3-pyrimidin-5-yl}furan-2-carboxamide;
5-(6-methoxy-1,1,4,4,8-pentamethyl-1,4-disila-1,2,3,4-tetrahydronaphthalen-7-yloxy)-*N*-{2-[3-(*N,N*-dimethylamino)propylamino]-4,6-dimethoxy-1,3-pyrimidin-5-yl}furan-2-carboxamide;
5-(6-methoxy-1,1,4,4,8-pentamethyl-1,4-disita-1,2,3,4-tetrahydronaphthalen-7-yloxy)-*N*-{2-[3-(morpholin-4-yl)propylamino]-4,6-dimethoxy-1,3-pyrimidin-5-yl}furan-2-carboxamide;
5-(6-chloro-1,1,4,4-tetramethyl-1,4-disila-1,2,3,4-tetrahydronaphthalen-7-yloxy)-*N*-(2,6-dimethoxyphenyl)furan-2-carboxamide;
5-(6-chloro-1,1,4,4-tetramethyl-1,4-disila-1,2,3,4-tetrahydronaphthalen-7-yloxy)-*N*-(2,4,6-trimethoxyphenyl)furan-2-carboxamide;
5-(6-chloro-1,1,4,4-tetramethyl-1,4-disila-1,2,3,4-tetrahydronaphthalen-7-yloxy)-*N*-(2,4,6-trimethoxy-1,3-pyrimidin-5-yl)furan-2-carboxamide;
5-(6-chloro-1,1,4,4-tetramethyl-1,4-disila-1,2,3,4-tetrahydronaphthalen-7-yloxy)-*N*-(2-methylamino-4,6-dimethoxy-1,3-pyrimidin-5-yl)furan-2-carboxamide;
5-(6-chloro-1,1,4,4-tetramethyl-1,4-disila-1,2,3,4-tetrahydronaphthalen-7-yloxy)-*N*-{2-[3-(4-methylpiperazin-1-yl)propylamino]-4,6-dimethoxy-1,3-pyrimidin-5-yl}furan-2-carboxamide;
5-(6-chloro-1,1,4,4-tetramethyl-1,4-disila-1,2,3,4-tetrahydronaphthalen-7-yloxy)-*N*-{2-[3-(*N,N*-dimethylamino)propylamino]-4,6-dimethoxy-1,3-pyrimidin-5-yl}furan-2-carboxamide;
5-(6-chloro-1,1,4,4-tetramethyl-1,4-disila-1,2,3,4-tetrahydronaphthalen-7-yloxy)-*N*-{2-[3-(morpholin-4-yl)propylamino]-4,6-dimethoxy-1,3-pyrimidin-5-yl}furan-2-carboxamide;
5-(6-chloro-1,1-dimethyl-1-sila-1,2,3,4-tetrahydronaphthalen-7-yloxy)-*N-*(2,6-dimethoxyphenyl)furan-2-carboxamide;
5-(6-chloro-1,1-dimethyl-1-sila-1,2,3,4-tetrahydronaphthalen-7-yloxy)-*N-*(2,4,6-trimethoxyphenyl)furan-2-carboxamide;
5-(6-chloro-1,1-dimethyl-1-sila-1,2,3,4-tetrahydronaphthalen-7-yloxy)-*N-*(2,4,6-trimethoxy-1,3-pyrimidin-5-yl)furan-2-carboxamide;
5-(6-chloro-1,1-dimethyl-1-sila-1,2,3,4-tetrahydronaphthalen-7-yloxy)-*N*-(2-methylamino-4,6-dimethoxy-1,3-pyrimidin-5-yl)furan-2-carboxamide;
5-(6-chloro-1,1-dimethyl-1-sila-1,2,3,4-tetrahydronaphthalen-7-yloxy)-*N*-{2-[3-(4-methylpiperazin-1-yl)propylamino]-4,6-dimethoxy-1,3-pyrimidin-5-yl}furan-2-carboxamide;
5-(6-chloro-1,1-dimethyl-1-sila-1,2,3,4-tetrahydronaphthalen-7-yloxy)-*N*-{2-[3-(*N,N*-dimethylamino)propylamino]-4,6-dimethoxy-1,3-pyrimidin-5-yl}furan-2-carboxamide;
5-(6-chloro-1,1-dimethyl-1-sila-1,2,3,4-tetrahydronaphthalen-7-yloxy)-*N*-{2-[3-(morpholin-4-yl)propylamino]-4,6-dimethoxy-1,3-pyrimidin-5-yl}furan-2-carboxamide;
5-(4,4,7-trimethyl-4-sila-chroman-6-yloxy)-*N*-(2,6-dimethoxyphenyl)furan-2-carboxamide;
5-(4,4,7-trimethyl-4-sila-chroman-6-yloxy)-*N*-(2,4,6-trimethoxyphenyl)furan-2-carboxamide;
5-(4,4,7-trimethyl-4-sila-chroman-6-yloxy)-*N*-(2,4,6-trimethoxy-1,3-pyrimidin-5-yl)furan-2-carboxamide;
5-(4,4,7-trimethyl-4-sila-chroman-6-yloxy)-*N*-(2-methylamino-4,6-dimethoxy-1,3-pyrimidin-5-yl)furan-2-carboxamide;
5-(4,4,7-trimethyl-4-sila-chroman-6-yloxy)-*N*-{2-[3-(4-methylpiperazin-1-yl)propylamino]-4,6-dimethoxy-1,3-pyrimidin-5-yl}furan-2-carboxamide;
5-(4,4,7-trimethyl-4-sila-chroman-6-yloxy)-*N*-{2-[3-(*N,N-*dimethylamino)propylamino]-4,6-dimethoxy-1,3-pyrimidin-5-yl}furan-2-carboxamide;
5-(4,4,7-trimethyl-4-sila-chroman-6-yloxy)-*N*-{2-[3-(morpholin-4-yl)propylamino]-4,6-dimethoxy-1,3-pyrimidin-5-yl}furan-2-carboxamide;
5-(1,2,3,4-tetrahydro-4,4,7-trimethyl-4-sila-quinolin-6-yloxy)-*N*-(2,6-di methoxyphenyl)furan-2-carboxamide;
5-(1,2,3,4-tetrahydro-4,4,7-trimethyl-4-sila-quinolin-6-yloxy)-*N*-(2,4,6-trimethoxyphenyl)furan-2-carboxamide;
5-(1,2,3,4-tetrahydro-4,4,7-trimethyl-4-sila-quinolin-6-yloxy)-*N*-(2,4,6-trimethoxy-1,3-pyrimidin-5-yl)furan-2-carboxamide;
5-(1,2,3,4-tetrahydro-4,4,7-trimethyl-4-sila-quinolin-6-yloxy)-*N*-(2-methylamino-4,6-dimethoxy-1,3-pyrimidin-5-yl)furan-2-carboxamide;
5-(1,2,3,4-tetrahydro-4,4,7-trimethyl-4-sila-quinolin-6-yloxy)-*N*-{2-[3-(4-methylpiperazin-1-yl)propylamino]-4,6-dimethoxy-1,3-pyrimidin-5-yl}furan-2-carboxamide;
5-(1,2,3,4-tetrahydro-4,4,7-trimethyl-4-sila-quinolin-6-yloxy)-*N*-{2-[3-(*N,N-*dimethylamino)propylamino]-4,6-dimethoxy-1,3-pyrimidin-5-yl}furan-2-carboxamide;
5-(1,2,3,4-tetrahydro-4,4,7-trimethyl-4-sila-quinolin-6-yloxy)-*N*-{2-[3-(morpholin-4-yl)propylamino]-4,6-dimethoxy-1,3-pyrimidin-5-yl}furan-2-carboxamide;
5-(1,1,3,3,6-pentamethyl-1,3-disila-2,3-dihydro-1*H*-inden-5-yloxy)-*N*-(2,6-dimethoxyphenyl)furan-2-carboxamide;
5-(1,1,3,3,6-pentamethyl-1,3-disila-2,3-dihydro-1*H*-inden-5-yloxy)-*N*-(2,4,6-trimethoxyphenyl)furan-2-carboxamide;
5-(1,1,3,3,6-pentamethyl-1,3-disila-2,3-dihydro-1*H*-inden-5-yloxy)-*N*-(2,4,6-trimethoxy-1,3-pyrimidin-5-yl)furan-2-carboxamide;
5-(1,1,3,3,6-pentamethyl-1,3-disila-2,3-dihydro-1*H*-inden-5-yloxy)-*N*-(2-methylamino-4,6-dimethoxy-1,3-pyrimidin-5-yl)furan-2-carboxamide;
5-(1,1,3,3,6-pentamethyl-1,3-disifa-2,3-dihydro-1*H*-inden-5-yloxy)-*N*-{2-[3-(4-methylpiperazin-1-yl)propylamino]-4,6-dimethoxy-1,3-pyrimidin-5-yl}furan-2-carboxamide;
5-(1,1,3,3,6-pentamethyl-1,3-disila-2,3-dihydro-1*H*-inden-5-yloxy)-*N*-{2-[3-(*N*,*N*-dimethylamino)propylamino]-4,6-dimethoxy-1,3-pyrimidin-5-yl}furan-2-carboxamide;
5-(1,1,3,3,6-pentamethyl-1,3-disila-2,3-dihydro-1*H*-inden-5-yloxy)-*N*-{2-[3-(morpholin-4-yl)propylamino]-4,6-dimethoxy-1,3-pyrimidin-5-yl}furan-2-carboxamide;
5-(1,1,5-trimethyl-1-sila-2,3-dihydro-1*H*-inden-6-yloxy)-*N*-(2,6-dimethoxyphenyl)furan-2-carboxamide;
5-(1,1,5-trimethyl-1-sila-2,3-dihydro-1*H*-inden-6-yloxy)-*N*-(2,4,6-trimethoxyphenyl)furan-2-carboxamide;
5-(1,1,5-trimethyl-1-sila-2,3-dihydro-1*H*-inden-6-yloxy)-*N*-(2,4,6-trimethoxy-1,3-pyrimidin-5-yl)furan-2-carboxamide;
5-(1,1,5-trimethyl-1-sila-2,3-dihydro-1*H*-inden-6-yloxy)-*N*-(2-methylamino-4,6-dimethoxy-1,3-pyrimidin-5-yl)furan-2-carboxamide;
5-(1,1,5-irimethyl-1-sila-2,3-dihydro-1*H*-inden-6-yloxy)-*N*-{2-[3-(4-methylpiperazin-1-yl)propylamino]-4,6-dimethoxy-1,3-pyrimidin-5-yl}furan-2-carboxamide;
5-(1,1,5-trimethyl-1-sila-2,3-dihydro-1*H*-inden-6-yloxy)-*N*-{2-[3-(*N,N-*dimethylamino)propylamino]-4,6-dimethoxy-1,3-pyrimidin-5-yl}furan-2-carboxamide;
5-(1,1,5-trimethyl-1-sila-2,3-dihydra-1*H*-inden-6-yloxy)-*N*-{2-[3-(morpholin-4-yl)propylamino]-4,6-dimethoxy-1,3-pyrimidin-5-yl}furan-2-carboxamide;
5-(1,1,5-trimethyl-1-sila-2,3-dihydro-1*H*-inden-6-yloxy)-*N*-{2-[(pyridin-2-yl)methylamino]-4,6-dimethoxy-1,3-pyrimidin-5-yl}furan-2-carboxamide;
5-(1,1,6-trimethyl-1-sila-2,3-dihydro-1*H*-inden-5-yloxy)-*N*-(2,6-dimethoxyphenyl)furan-2-carboxamide;
5-(1,1,6-trimethyl-1-sila-2,3-dihydro-1*H*-inden-5-yloxy)-*N*-(2,4,6-trimethoxyphenyl)furan-2-carboxamide;
5-(1,1,6-trimethyl-1-sila-2,3-dihydro-1*H*-inden-5-yloxy)-*N*-(2,4,6-trimethoxy-1,3-pyrimidin-5-yl)furan-2-carboxamide;
5-(1,1,6-trimethyl-1-sila-2,3-dihydro-1*H*-inden-5-yloxy)-*N*-(2-methylamino-4,6-dimethoxy-1,3-pyrimidin-5-yl)furan-2-carboxamide;
5-(1,1,6-trimethyl-1-sila-2,3-dihydro-1*H*-inden-5-yloxy)-*N*-{2-[3-(4-methylpiperazin-1-yl)propylamino]-4,6-dimethoxy-1,3-pyrimidin-5-yl}furan-2-carboxamide;
5-(1,1,6-trimethyl-1-sila-2,3-dihydro-1*H*-inden-5-yloxy)-*N*-{2-[3-(*N,N-*dimethylamino)propylamino]-4,6-dimethoxy-1,3-pyrimidin-5-yl}furan-2-carboxamide;
5-(1,1,6-trimethyl-1-sila-2,3-dihydro-1*H*-inden-5-yloxy)-*N*-{2-[3-(morpholin-4-yl)propylamino]-4,6-dimethoxy-1,3-pyrimidin-5-yl}furan-2-carboxamide;
5-(1,1,5-trimethyl-1-sila-1*H*-inden-6-yloxy)-*N*-(2,6-dimethoxyphenyl)furan-2-carboxamide;
5-(1,1,5-trimethyl-1-sila-1*H*-inden-6-yloxy)-*N*-(2,4,6-trimethoxyphenyl)furan-2-carboxamide;
5-(1,1,5-trimethyl-1-sila-1*H*-inden-6-yloxy)-*N*-(2,4,6-trimethoxy-1,3-pyrimidin-5-yl)furan-2-carboxamide;
5-(1,1,5-trimethyl-1-sila-1*H*-inden-6-yloxy)-*N*-(2-methylamino-4,6-dimethoxy-1,3-pyrimidin-5-yl)furan-2-carboxamide;
5-(1,1,5-trimethyl-1-sila-1*H*-inden-6-yloxy)-*N*-{2-[3-(4-methylpiperazin-1-yl)propylamino]-4,6-dimethoxy-1,3-pyrimidin-5-yl}furan-2-carboxamide;
5-(1,1,5-trimethyl-1-sila-1*H*-inden-6-yloxy)-*N*-{2-[3-(*N,N-*dimethylamino)propylamino]-4,6-dimethoxy-1,3-pyrimidin-5-yl}furan-2-carboxamide;
5-(1,1,5-trimethyl-1-sila-1*H*-inden-6-yloxy)-*N*-{2-[3-(morpholin-4-yl)propylamino]-4,6-dimethoxy-1,3-pyrimidin-5-yl}furan-2-carboxamide;
5-(5-methoxy-1,1-dimethyl-1-sila-1*H*-inden-6-yloxy)-*N*-(2,6-dimethoxyphenyl)furan-2-carboxamide;
5-(5-methoxy-1,-dimethyl-1-sila-1*H*-inden-6-yloxy)-*N*-(2,4,6-trimethoxyphenyl)furan-2-carboxamide;
5-(5-methoxy-1,1-dimethyl-1-sila-1*H*-inden-6-yloxy)-*N*-(2,4,6-trimethoxy-1,3-pyrimidin-5-yl)furan-2-carboxamide;
5-(5-methoxy-1,1-dimethyl-1-sila-1*H*-inden-6-yloxy)-*N*-(2-methylamino-4,6-dimethoxy-1,3-pyrimidin-5-yl)furan-2-carboxamide;
5-(5-methoxy-1,1-dimethyl-1-sila-1*H*-inden-6-yloxy)-*N*-{2-[3-(4-methylpiperazin-1-yl)propylamino]-4,6-dimethoxy-1,3-pyrimidin-5-yl}furan-2-carboxamide;
5-(5-methoxy-1,1-dimethyl-1-sila-1 *H*-inden-6-yloxy)-*N*-{2-[3-(*N*,*N-*dimethylamino)propylamino]-4,6-dimethoxy-1,3-pyrimidin-5-yl}furan-2-carboxamide;
5-(5-methoxy-1,1-dimethyl-1-sila-1*H*-inden-6-yloxy)-*N*-{2-[3-(morpholin-4-yl)propylamino]-4,6-dimethoxy-1,3-pyrimidin-5-yl}furan-2-carboxamide;
5-[1,1,6-trimethyl-3-(2-propyl)-1-sila-2,3-dihydro-1*H*-inden-5-yloxy]-*N*-(2,6-dimethoxyphenyl)furan-2-carboxamide;
5-[1,1,6-trimethyl-3-(2-propyl)-1-sila-2,3-dihydro-1*H*-inden-5-yloxy]-*N-*(2,4,6-trimethoxyphenyl)furan-2-carboxamide;
5-[1,1,6-trimethyl-3-(2-propyl)-1-sila-2,3-dihydro-1*H*-inden-5-yloxy]-*N-*(2,4,6-trimethoxy-1,3-pyrimidin-5-yl)furan-2-carboxamide;
5-[1,1,6-trimethyl-3-(2-propyl)-1-sila-2,3-dihydro-1*H*-inden-5-yloxy]-*N*-(2-methylamino-4,6-dimethoxy-1,3-pyrimidin-5-yl)furan-2-carboxamide;
5-[1,1,6-trimethyl-3-(2-propyl)-1-sila-2,3-dihydro-1*H*-inden-5-y)oxy]-*N*-{2-[3-(4-methylpiperazin-1-yl)propylamino]-4,6-dimethoxy-1,3-pyrimidin-5-yl}furan-2-carboxamide;
5-[1,1,6-trimethyl-3-(2-propyl)-1-sila-2,3-dihydro-1*H*-inden-5-yloxy]-*N*-{2-[3-(*N,N*-dimethylamino)propylamino]-4,6-dimethoxy-1,3-pyrimidin-5-yl}furan-2-carboxamide;
5-[1,1,6-trimethyl-3-(2-propyl)-1-sila-2,3-dihydro-1*H*-inden-5-yloxy]-*N*-{2-[3-(morpholin-4-yl)propylamino]-4,6-dimethoxy-1,3-pyrimidin-5-yl}furan-2-carboxamide;
5-(5-chloro-1,1-dimethyl-1-sila-2,3-dihydro-1*H*-inden-6-yloxy)-*N*-(2,6-dimethoxyphenyl)furan-2-carboxamide;
5-(5-chloro-1,1-dimethyl-1-sila-2,3-dihydro-1*H*-inden-6-yloxy)-*N*-(2,4,6-trimethoxyphenyl)furan-2-carboxamide;
5-(5-chloro-1,1-dimethyl-1-sila-2,3-dihydro-1*H*-inden-6-yloxy)-*N*-(2,4,6-trimethoxy-1,3-pyrimidin-5-yl)furan-2-carboxamide;
5-(5-chloro-1,1-dimethyl-1-sila-2,3-dihydro-1*H*-inden-6-yloxy)-*N*-(2-methylamino-4,6-dimethoxy-1,3-pyrimidin-5-yl)furan-2-carboxamide;
5-(5-chloro-1,1-dimethyl-1-sila-2,3-dihydro-1*H*-inden-6-yloxy)-*N*-{2-[3-(4-methylpiperazin-1-yl)propylamino]-4,6-dimethoxy-1,3-pyrimidin-5-yl}furan-2-carboxamide;
5-(5-chloro-1,1-dimethyl-1-sila-2,3-dihydro-1*H*-inden-6-yloxy)-*N*-{2-[3-(*N,N-*dimethylamino)propylamino]-4,6-dimethoxy-1,3-pyrimidin-5-yl}furan-2-carboxamide;
5-(5-chloro-1,1-dimethyl-1-sila-2,3-dihydro-1*H*-inden-6-yloxy)-*N*-{2-[3-(morpholin-4-yl)propylamino]-4,6-dimethoxy-1,3-pyrimidin-5-yl}furan-2-carboxamide;
5-(5-chloro-1,1,7-trimethyl-1-sila-2,3-dihydro-1*H*-inden-4-yloxy)-*N*-(2,6-dimethoxyphenyl)furan-2-carboxamide;
5-(5-chloro-1,1,7-trimethyl-1-sila-2,3-dihydro-1*H*-inden-4-yloxy)-*N*-(2,4,6-trimethoxyphenyl)furan-2-carboxamide;
5-(5-chloro-1,1,7-trimethyl-1-sila-2,3-dihydro-1*H*-inden-4-yloxy)-*N*-(2,4,6-trimethoxy-1,3-pyrimidin-5-yl)furan-2-carboxamide;
5-(5-chloro-1,1,7-trimethyl-1-sila-2,3-dihydro-1*H*-inden-4-yloxy)-*N*-(2-methylamino-4,6-dimethoxy-1,3-pyrimidin-5-yl)furan-2-carboxamide;
5-(5-chloro-1,1,7-trimethyl-1-sila-2,3-dihydro-1*H*-inden-4-yloxy)-*N*-{2-[3-(4-methylpiperazin-1-yl)propylamino]-4,6-dimethoxy-1,3-pyrimidin-5-yl}furan-2-carboxamide;
5-(5-chloro-1,1,7-trimethyl-1-sila-2,3-dihydro-1*H*-inden-4-yloxy)-*N*-{2-[3-(*N, N*-dimethylamino)propylamino]-4,6-dimethoxy-1,3-pyrimidin-5-yl}furan-2-carboxamide;
5-(5-chloro-1,1,7-trimethyl-1-sila-2,3-dihydro-1*H*-inden-4-yloxy)-*N*-{2-[3-(morpholin-4-yl)propylamino]-4,6-dimethoxy-1,3-pyrimidin-5-yl}furan-2-carboxamide;
5-(5-methoxy-1,1-dimethyl-1-sila-2,3-dihydro-1*H*-inden-6-yloxy)-*N*-(2,6-dimethoxyphenyl)furan-2-carboxamide;
5-(5-methoxy-1,1-dimethyl-1-sila-2,3-dihydro-1*H*-inden-6-yloxy)-*N*-(2,4,6-trimethoxyphenyl)furan-2-carboxamide;
5-(5-methoxy-1,1-dimethyl-1-sila-2,3-dihydro-1*H*-inden-6-yloxy)-*N*-(2,4,6-trimethoxy-1,3-pyrimidin-5-yl)furan-2-carboxamide;
5-(5-methoxy-1,1-dimethyl-1-sila-2,3-dihydro-1*H*-inden-6-yloxy)-*N*-(2-methylamino-4,6-dimethoxy-1,3-pyrimidin-5-yl)furan-2-carboxamide;
5-(5-methoxy-1,1-dimethyl-1-sila-2,3-dihydro-1*H*-inden-6-yloxy)-*N*-{2-[3-(4-methylpiperazin-1-yl)propylamino]-4,6-dimethoxy-1,3-pyrimidin-5-yl}furan-2-carboxamide;
5-(5-methoxy-1,1-dimethyl-1-sila-2,3-dihydro-1*H*-inden-6-yloxy)-*N*-{2-[3-(*N,N*-dimethylamino)propylamino]-4,6-dimethoxy-1,3-pyrimidin-5-yl}furan-2-carboxamide;
5-(5-methoxy-1,1-dimethyl-1-sila-2,3-dihydro-1*H*-inden-6-yloxy)-*N*-{2-[3-(morpholin-4-yl)propylamino]-4,6-dimethoxy-1,3-pyrimidin-5-yl}furan-2-carboxamide;
5-(5-methoxy-1,1-dimethyl-1-sila-2,3-dihydro-1*H*-inden-6-yloxy)-*N*-{2-[(pyridin-2-yl)methylamino]-4,6-dimethoxy-1,3-pyrimidin-5-yl}furan-2-carboxamide;
5-[(1,1,4,4,7-pentamethyl-1,4-disila-1,2,3,4-tetrahydronaphthalen-6-yl)methyl]-*N*-{2-[3-(4-methylpiperazin-1-yl)propylamino]-4,6-dimethoxy-1,3-pyrimidin-5-yl}furan-2-carboxamide;
5-[(1,1,4,4,7-pentamethyl-1,4-disila-1,2,3,4-tetrahydronaphthalen-6-yl)methyl]-*N*-{2-[3-(*N,N*-dimethylamino)propylamino]-4,6-dimethoxy-1,3-pyrimidin-5-yl}furan-2-carboxamide;
5-[(1,1,4,4,7-pentamethyl-1,4-disila-1,2,3,4-tetrahydronaphthalen-6-yl)methyl]-*N*-{2-[3-(morpholin-4-yl)propylamino]-4,6-dimethoxy-1,3-pyrimidin-5-yl}furan-2-carboxamide;
5-[(1,1,3,3,6-pentamethyl-1,3-disila-2,3-dihydro-1*H*-inden-5-yl)methyl]-*N-*(2,6-dimethoxyphenyl)furan-2-carboxamide;
5-[(1,1,3,3,6-pentamethyl-1,3-disila-2,3-dihydro-1*H*-inden-5-yl)methyl]-*N-*(2,4,6-trimethoxyphenyl)furan-2-carboxamide;
5-[(1,1,3,3,6-pentamethyl-1,3-disila-2,3-dihydro-1*H*-inden-5-yl)methyl]-*N-*(2,4,6-trimethoxy-1,3-pyrimidin-5-yl)furan-2-carboxamide;
5-[(1,1,3,3,6-pentamethyl-1,3-disila-2,3-dihydro-1*H*-inden-5-yl)methyl]-*N-*(2-methylamino-4,6-dimethoxy-1,3-pyrimidin-5-yl)furan-2-carboxamide;
5-[(1,1,3,3,6-pentamethyl-1,3-disiia-2,3-dihydro-1*H*-inden-5-yl)methyl]-*N-*{2-[3-(4-methylpiperazin-1-yl)propylamino]-4,6-dimethoxy-1,3-pyrimidin-5-yl}furan-2-carboxamide;
5-[(1,1,3,3,6-pentamethyl-1,3-disila-2,3-dihydro-1*H*-inden-5-yl)methyl]-*N-*{2-[3-(*N,N*-dimethylamino)propylamino]-4,6-dimethoxy-1,3-pyrimidin-5-yl}furan-2-carboxamide;
5-[(1,1,3,3,6-pentamethyl-1,3-disila-2,3-dihydro-1*H*-inden-5-yl)methyl]-*N-*{2-[3-(morpholin-4-yl)propylamino]-4,6-dimethoxy-1,3-pyrimidin-5-yl}furan-2-carboxamide;
5-[(1,1,5-trimethyl-1-sila-2,3-dihydro-1*H*-inden-6-yl)methyl]-*N*-(2,6-dimethoxyphenyl)furan-2-carboxamide;
5-[(1,1,5-trimethyl-1-sila-2,3-dihydro-1*H*-inden-6-yl)methyl]-*N*-(2,4,6-trimethoxyphenyl)furan-2-carboxamide;
5-[(1,1,5-trimethyl-1-sita-2,3-dihydro-1*H*-inden-6-yl)methyl]-*N*-(2,4,6-trimethoxy-1,3-pyrimidin-5-yl)furan-2-carboxamide;
5-[(1,1,5-trimethyl-1-sila-2,3-dihydro-1*H*-inden-6-yl)methyl]-*N*-(2-methylamino-4,6-dimethoxy-1,3-pyrimidin-5-yl)furan-2-carboxamide;
5-[(1,1,5-trimethyl-1-sila-2,3-dihydro-1*H*-inden-6-yl)methyl]-*N*-{2-[3-(4-methylpiperazin-1-yl)propylamino]-4,6-dimethoxy-1,3-pyrimidin-5-yl}furan-2-carboxamide;
5-[(1,1,5-trimethyl-1-sila-2,3-dihydro-1*H*-inden-6-yl)methyl]-*N* -{2-[3-(*N,N-*dimethylamino)propylamino]-4,6-dimethoxy-1,3-pyrimidin-5-yl}furan-2-carboxamide;
5-[(1,1,5-trimethyl-1-sila-2,3-dihydro-1*H*-inden-6-yl)methyl]-*N*-{2-[3-(morpholin-4-yl)propylamino]-4,6-dimethoxy-1,3-pyrimidin-5-yl}furan-2-carboxamide;
5-[(1,1,5-trimethyl-1-sila-2,3-dihydro-1*H*-inden-6-yl)methyl]-*N*-{2-[(pyridin-2-yl)methylamino]-4,6-dimethoxy-1,3-pyrimidin-5-yl}furan-2-carboxamide;
5-[(1,1,5-trimethyl-1-sila-2,3-dihydroinden-6-yl)methyl]-*N*-(4-chloro-2,6-dimethoxyphenyl)furan-2-carboxamide;
5-(1,1,3,4,4,6-hexamethyl-1-sila-1,2,3,4-tetrahydronaphthalen-7-yloxy)-*N-*{2-[2-(morpholin-4-yl)-ethylamino]-4,6-dimethoxy-1,3-pyrimidin-5-yl}furan-2-carboxamide;
5-(1,1,4,4,6-pentamethyl-1,4-disila-1,2,3,4-tetrahydronaphthalen-5-oxy)-*N-*(2,4,6-trimethoxyphenyl)furan-2-carboxamide;
5-(7'-hydroxy-1,1,1',1',6,6'-hexamethyl-3,3'-spiro-1,1'-disila-4,4'-dioxo-1,1',2,2',3,3',4,4'-octahydrodinaphthalen-7-yloxy)-*N*-(2,6-dimethoxyphenyl)furan-2-carboxamide;
5-[1,1,2,6-tetramethyl-3-(2-propy)-1-sila-2,3-dihydroinden-5-yloxy]-*N-*(2,4,6-trimethoxyphenyl)furan-2-carboxamide;
5-(1,1,3,4,4-pentamethyl-1-sila-1,2,3,4-tetrahydronaphthalen-7-yloxy)-*N-*(2,4,6-trimethoxy-1,3-pyrimidin-5-yl)furan-2-carboxamide.
5-(1,1,5-trimethyl-1-sila-2,3-dihydroinden-6-yloxy)-*N*-(4-chloro-2,6-dimethoxyphenyl)furan-2-carboxamide;
5-(1,1,3,3,5-pentamethyl-1-sila-2,3-dihydroinden-6-yloxy)-*N*-(2,6-dimethoxyphenyl)furan-2-carboxamide;
5-(1,1,3,3,6-pentamethyl-1-sila-2,3-dihydroinden-5-yloxy)-*N*-(2,6-dimethoxyphenyl)furan-2-carboxamide;
5-(8-methoxy-1,1,4,4,6-pentamethyl-1-sila-1,2,3,4-tetrahydronaphthalen-7-yloxy)-*N*-{2-[3-(morpholin-4-yl)-*N*-propylamino]-4,6-dimethoxy-1,3-pyrimidin-5-yl}furan-2-carboxamide;
5-(1,1,6-trimethyl-1-sila-1,2,3,4-tetrahydronaphthalen-7-yloxy)-*N-*(2,4-dimethoxypyridin-3-yl)furan-2-carboxamide.
5-(1,1,3,4,4,6-hexamethyl-1-sila-1,2,3,4-tetrahydronaphthalen-7-yloxy)-*N*-(2,4,6-trimethoxy-1,3-pyrimidin-5-yl)furan-2-carboxamide and
5-(1,1,3,4,4,6-hexamethyl-1-sila-1,2,3,4-tetrahydronaphthalen-7-yloxy)-*N*-[2-(2-*N,N*-dimethylaminoethyl)-4,6-trimethoxy-1,3-pyrimidin-5-yl]furan-2-carboxamide;
the corresponding structures of which are shown below, respectively (ordered left to right):

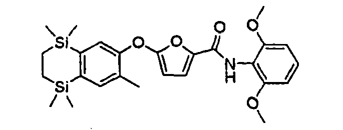

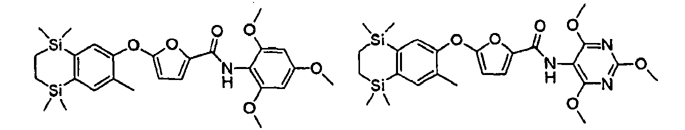

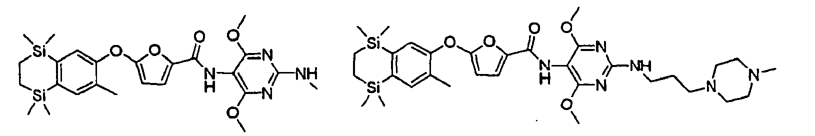

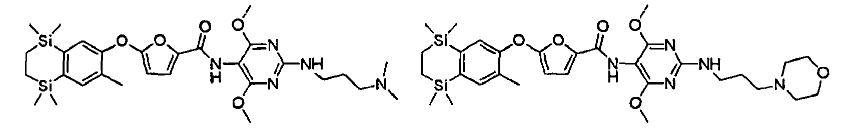

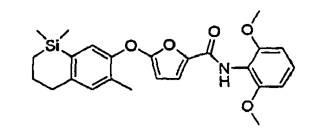

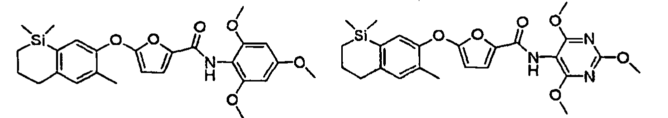

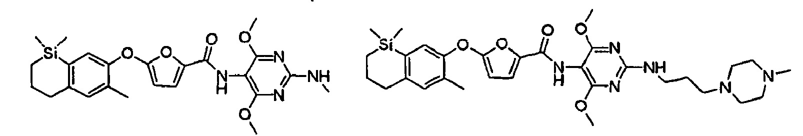

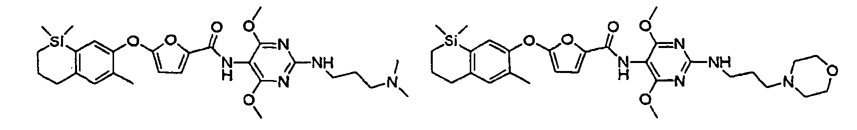

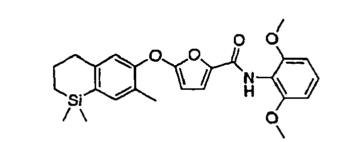

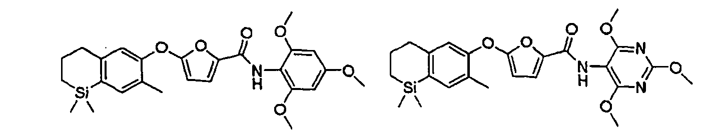

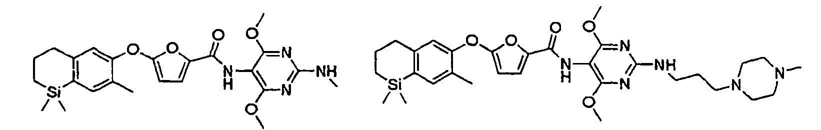

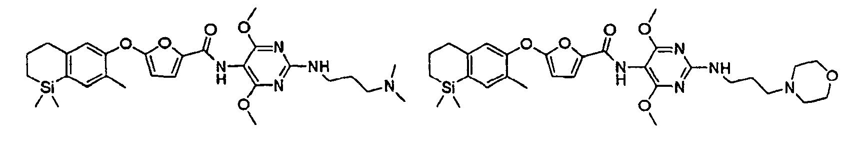

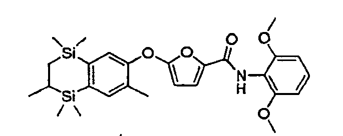

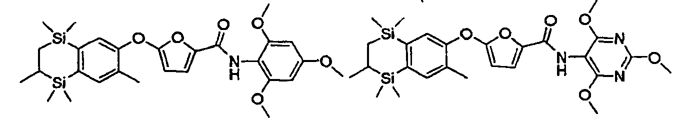

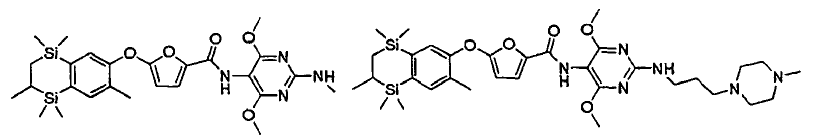

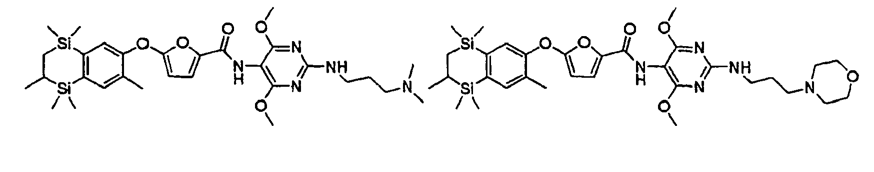

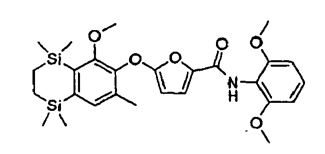

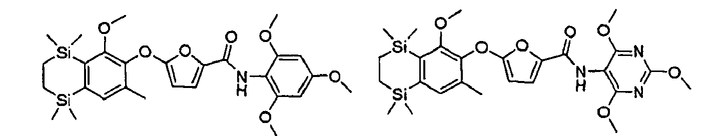

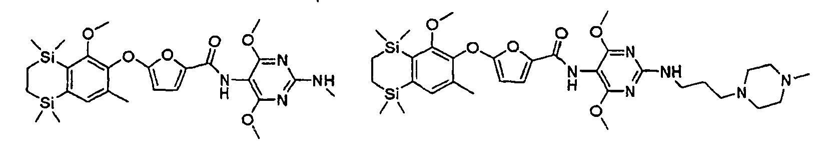

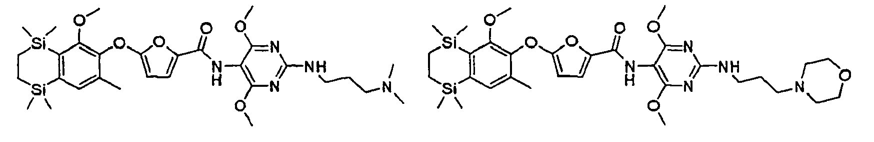

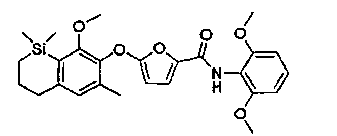

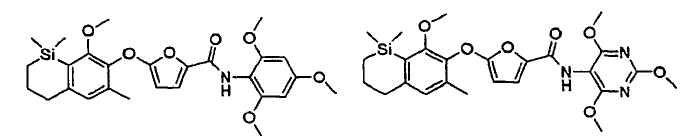

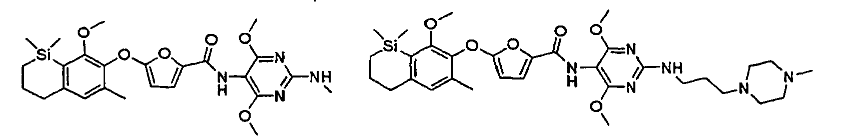

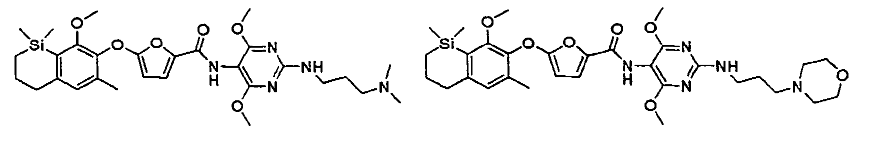

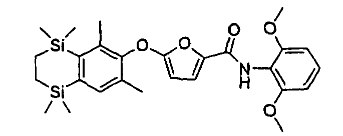

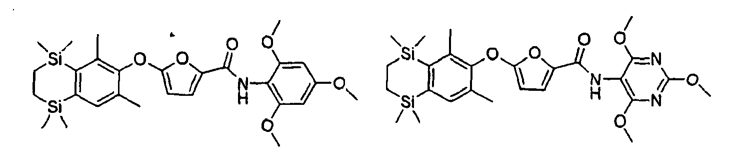

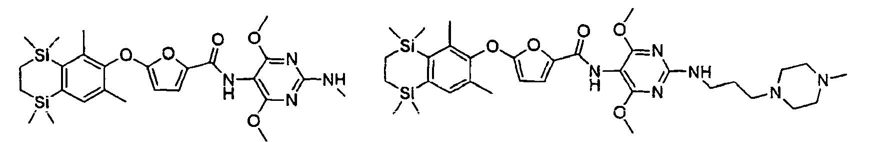

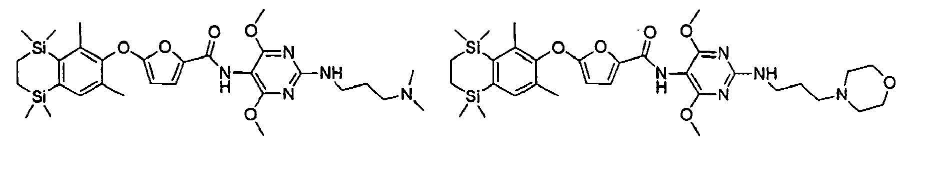

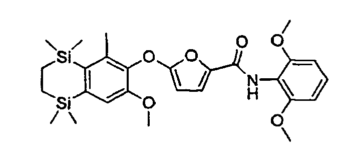

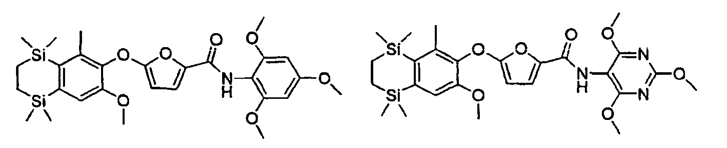

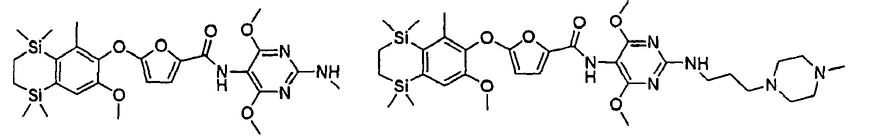

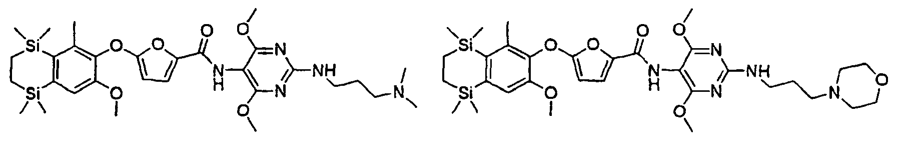

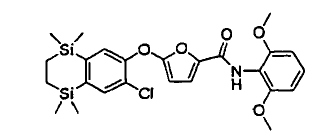

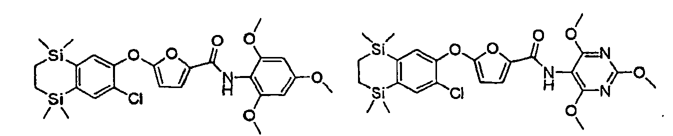

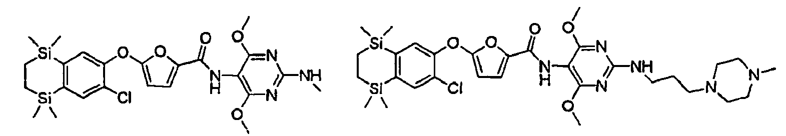

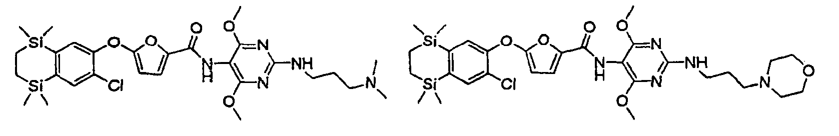

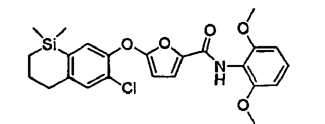

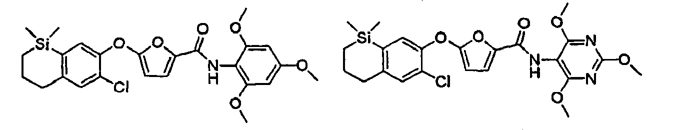

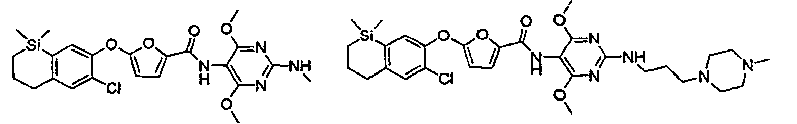

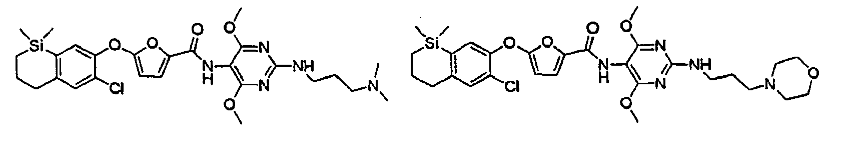

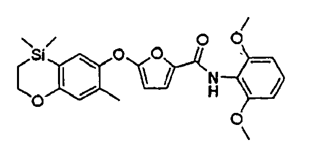

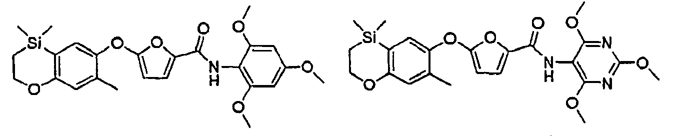

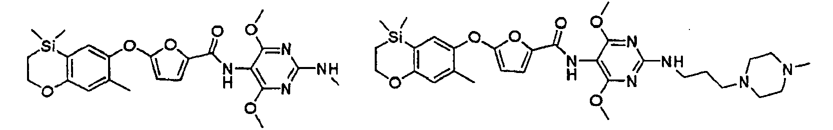

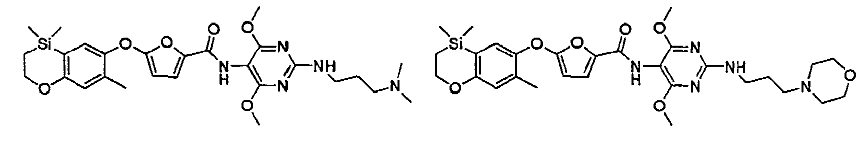

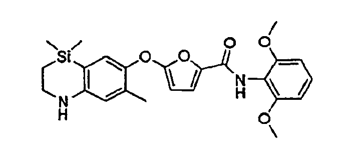

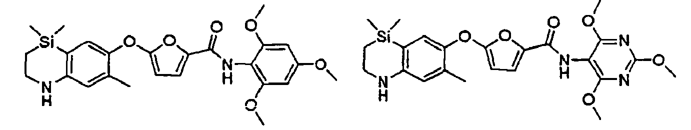

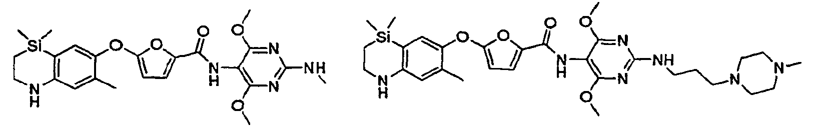

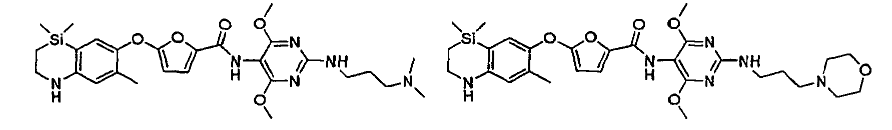

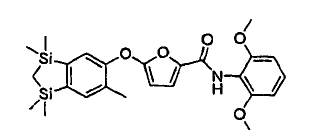

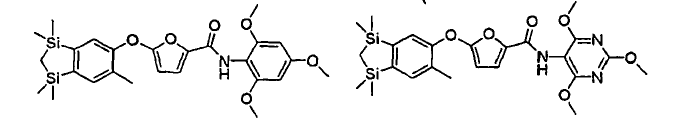

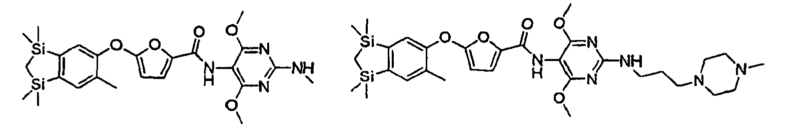

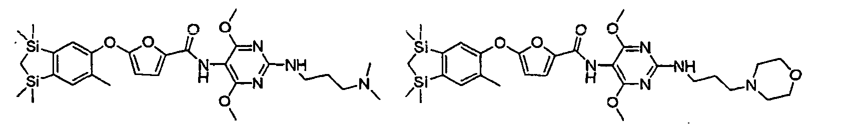

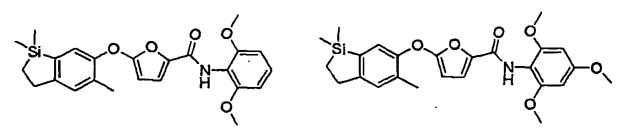

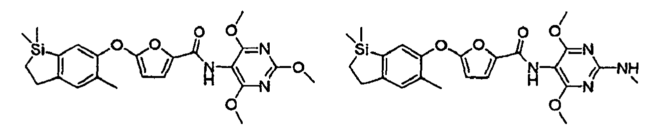

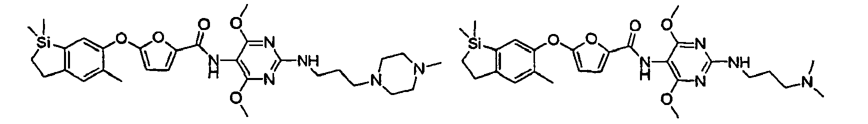

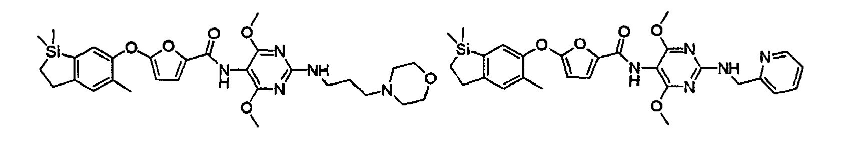

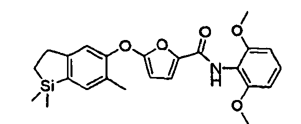

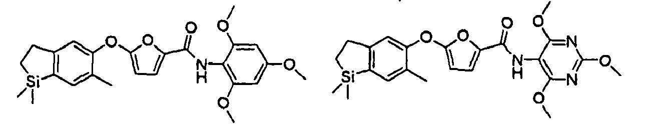

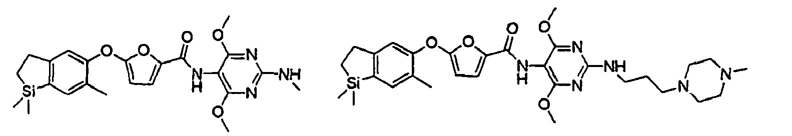

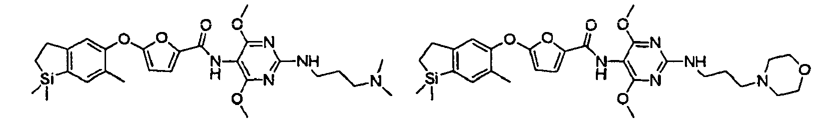

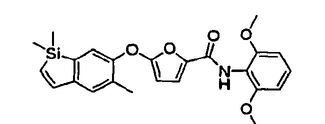

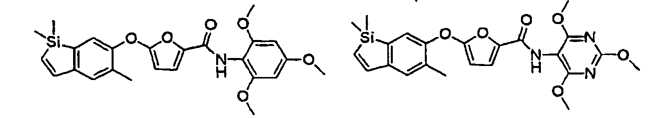

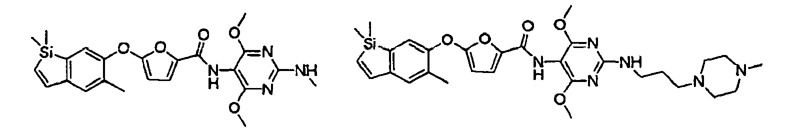

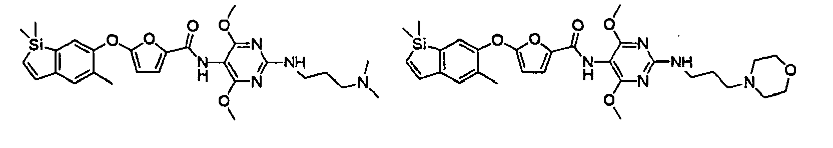

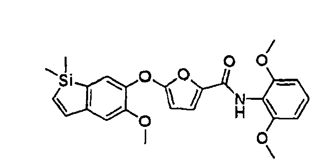

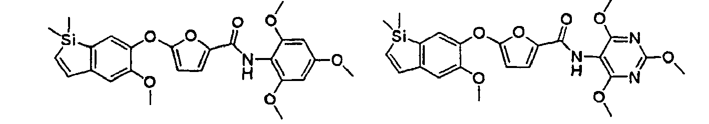

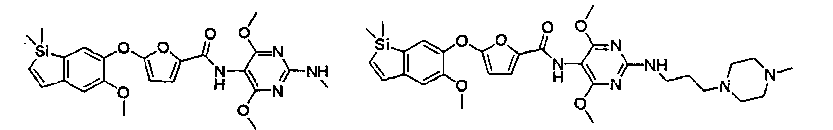

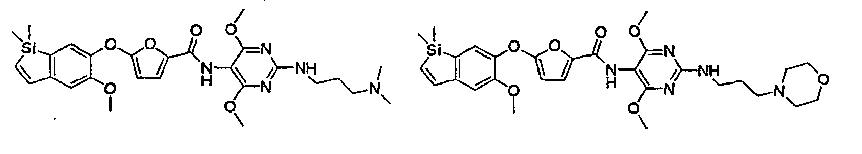

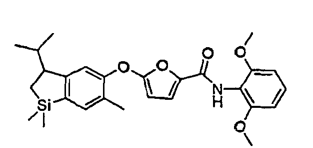

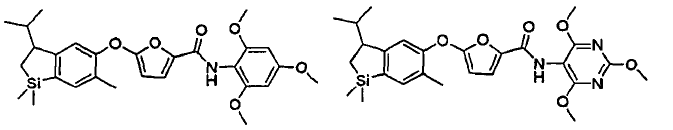

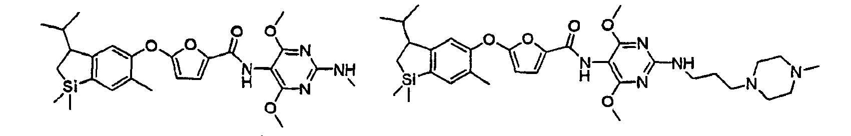

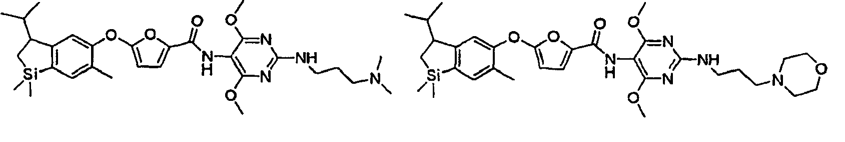

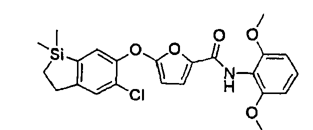

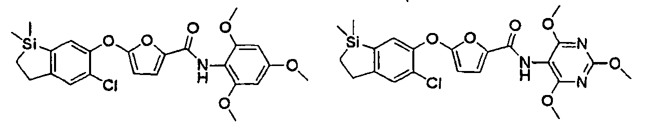

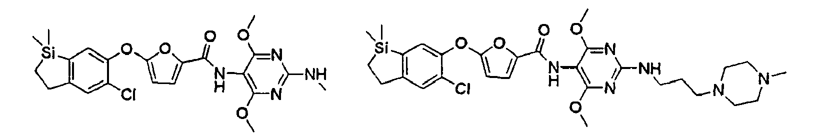

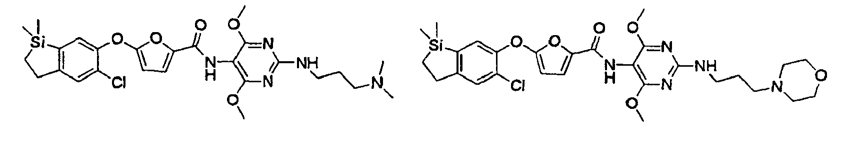

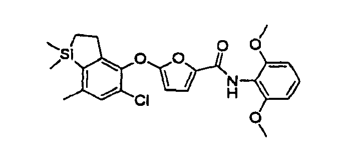

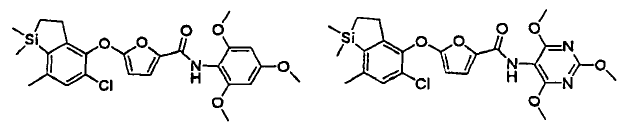

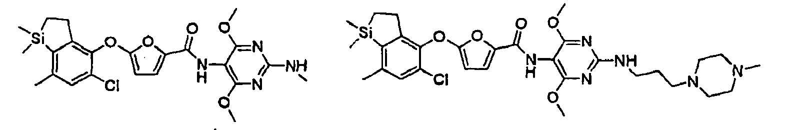

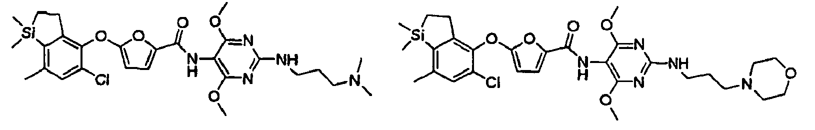

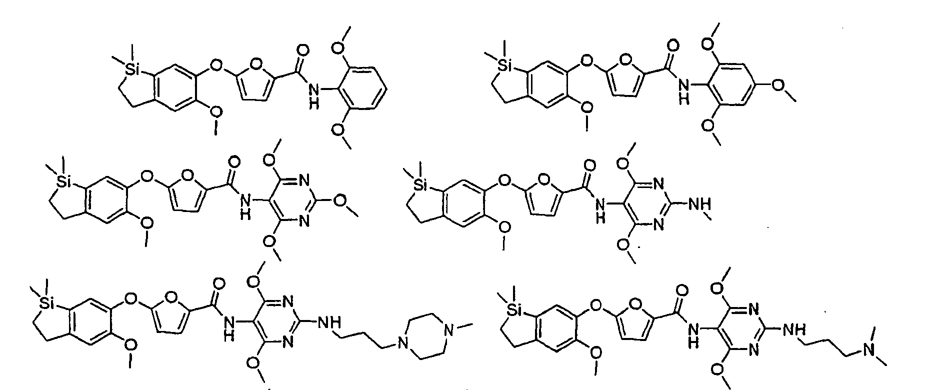

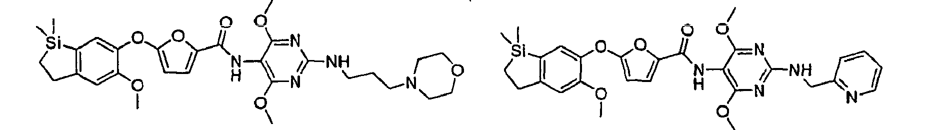

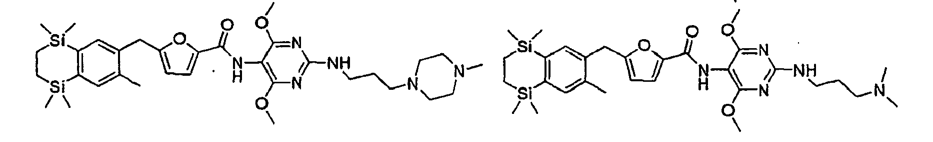

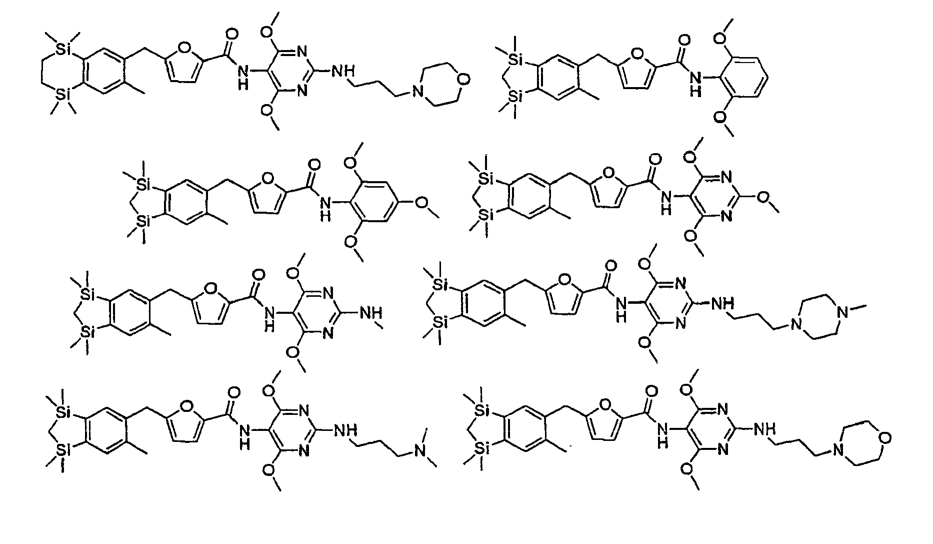

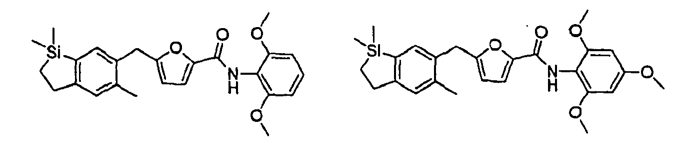

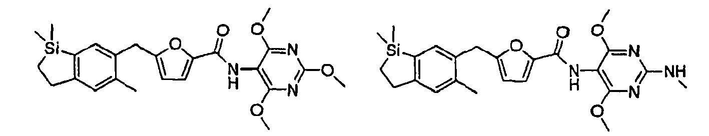

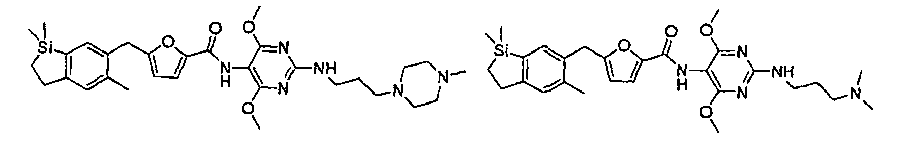

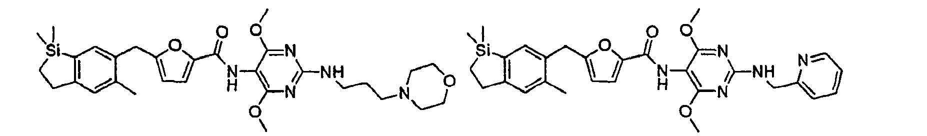

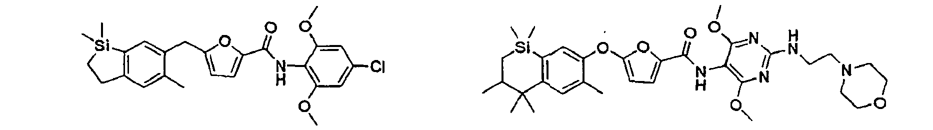

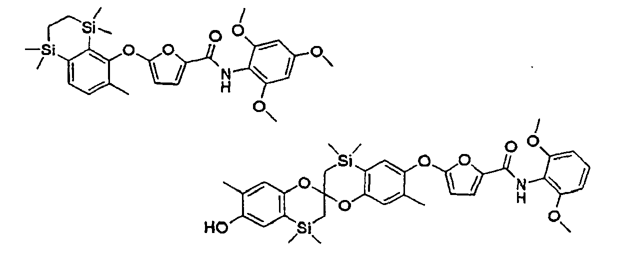

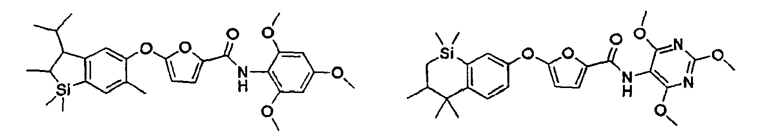

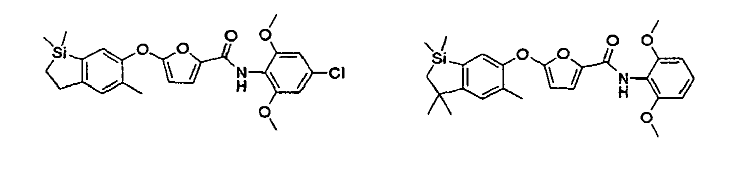

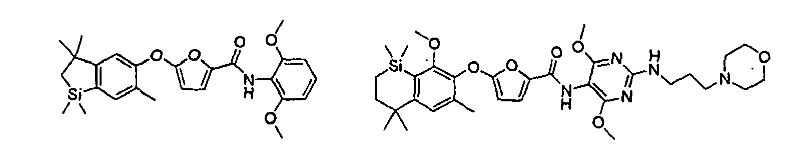

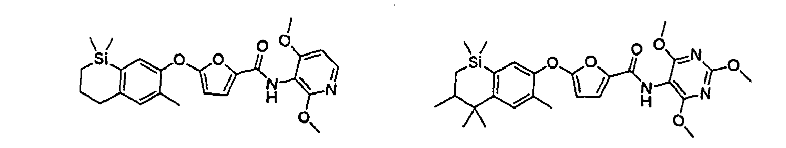

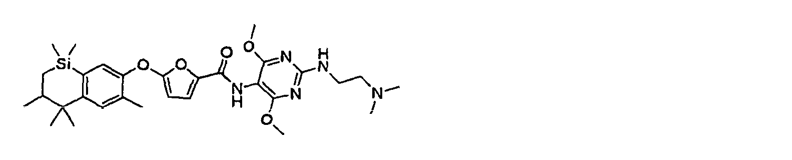

Compounds of the invention may be chiral. They may be in the form of a single enantiomer or diastereomer, or a racemate.

The compounds of the invention may be prepared in racemic form, or prepared in individual enantiomeric form by specific synthesis or resolution as will be appreciated in the art. The compounds may, for example, be resolved into their enantiomers by standard techniques, such as the formation of diastereomeric pairs by salt formation with an optically active acid followed by fractional crystallisation and regeneration of the free base. Alternatively, the enantiomers of the novel compounds may be separated by HPLC using a chiral column.

Some compounds of the formula may exist in different tautomeric forms, which also fall within the scope of the invention.

A compound of the invention may be in a protected amino, or protected hydroxy or protected carboxy form. The terms "protected amino", "protected hydroxy" and "protected carboxy" as used herein refer to amino, hydroxy and carboxy groups which are protected in a manner familiar to those skilled in the art. For example, an amino group can be protected by a benzyloxycarbonyl, tert-butoxycarbonyl, acetyl or like group, or in the form of a phthalimido or like group. A carboxyl group can be protected in the form of a readily cleavable ester such as the methyl, ethyl, benzyl or tert-butyl ester.

Some compounds of the formula may exist in the form of solvates, for example hydrates, which also fall within the scope of the present invention.

Compounds of the invention may be in the form of pharmaceutically acceptable salts, for example, addition salts of inorganic or organic acids. Such inorganic acid addition salts include, for example, salts of hydrobromic acid, hydrochloric acid, nitric acid, phosphoric acid and sulphuric acid. Organic acid addition salts include, for example, salts of acetic acid, benzenesulphonic acid, benzoic acid, camphorsulphonic acid, citric acid, 2-(4-chlorophenoxy)-2-methylpropionic acid, 1,2-ethanedisulphonic acid, ethanesulphonic acid, ethylenediaminetetraacetic acid (EDTA), fumaric acid, glucoheptonic acid, gluconic acid, glutamic acid, N-glycolylarsanilic acid, 4-hexylresorcinol, hippuric acid, 2-(4-hydroxybenzoyl)benzoic acid, 1-hydroxy-2-naphthoic acid, 3-hydroxy-2-naphthoic acid, 2-hydroxyethanesulphonic acid, lactobionic acid, n-dodecyl sulphuric acid, maleic acid, malic acid, mandelic acid, methanesulphonic acid, methyl sulphuric acid, mucic acid, 2-naphthalenesulphonic acid, pamoic acid, pantothenic acid, phosphanilic acid ((4-aminophenyl)phosphonic acid), picric acid, salicylic acid, stearic acid, succinic acid, tannic acid, tartaric acid, terephthalic acid, p-toluenesulphonic acid, 10-undecenoic acid and the like.

It will be appreciated that such salts, provided that they are pharmaceutically acceptable, may be used in therapy. Such salts may be prepared by reacting the compound with a suitable acid in a conventional manner.

A compound of the invention may be prepared by any suitable method known in the art and/or by the following processes:

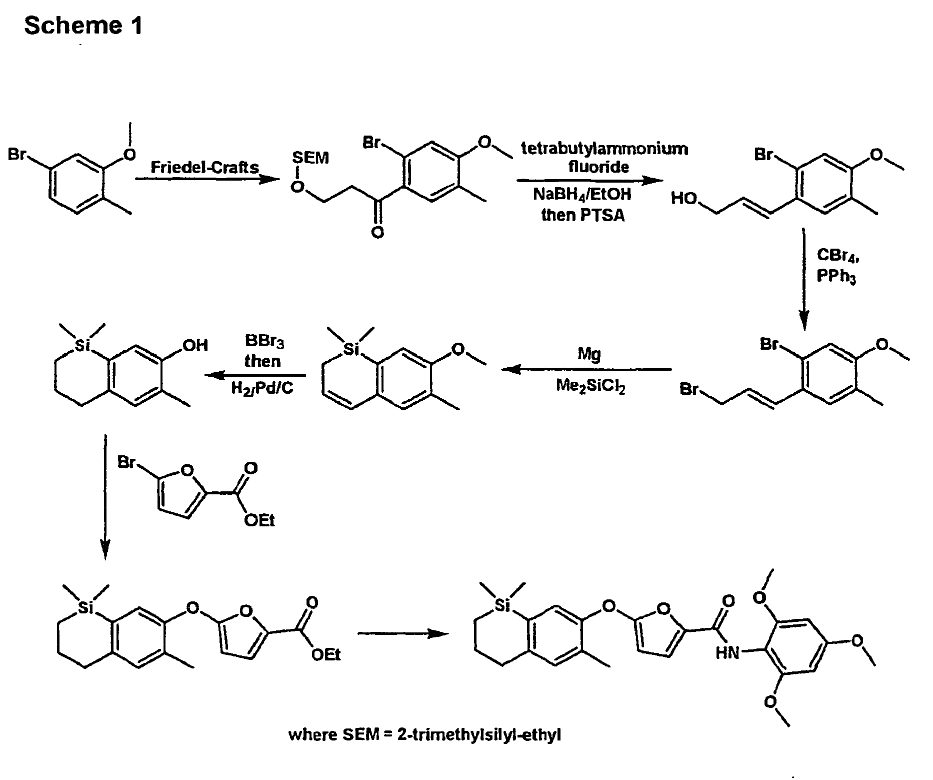

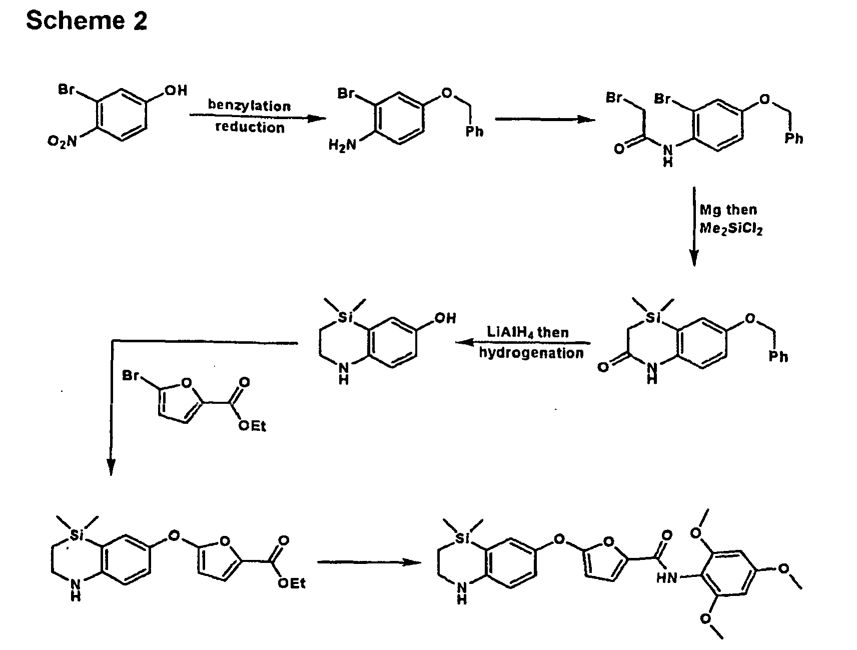

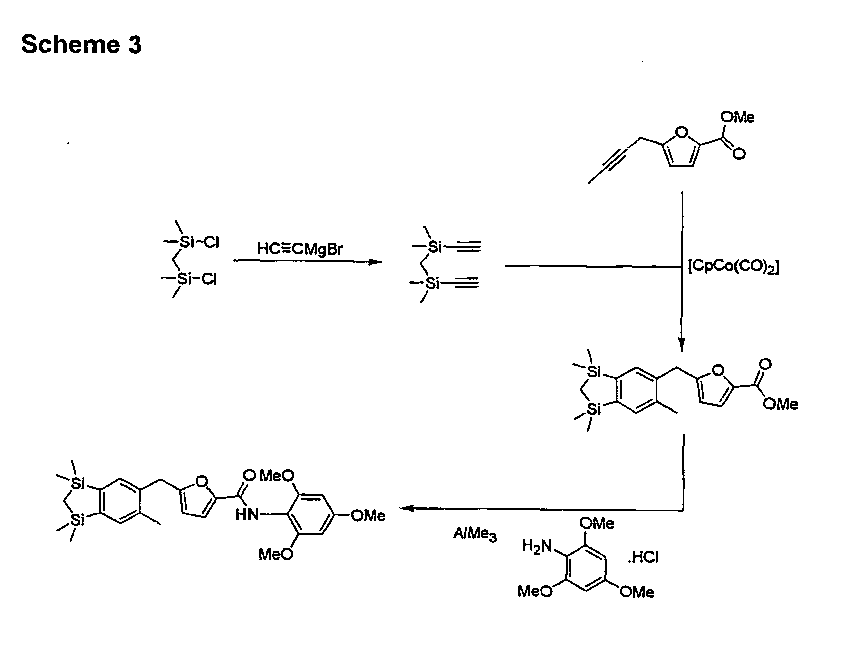

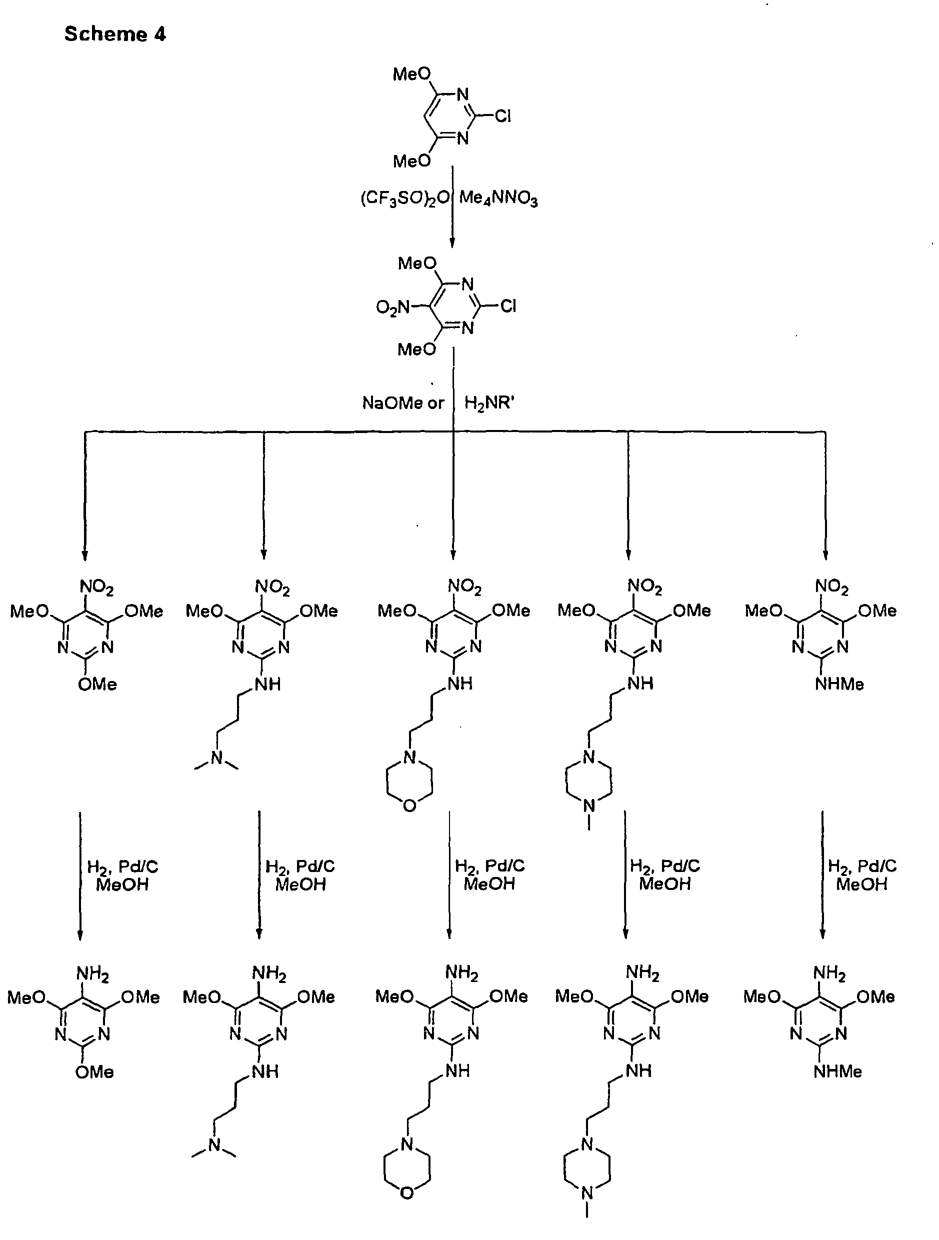

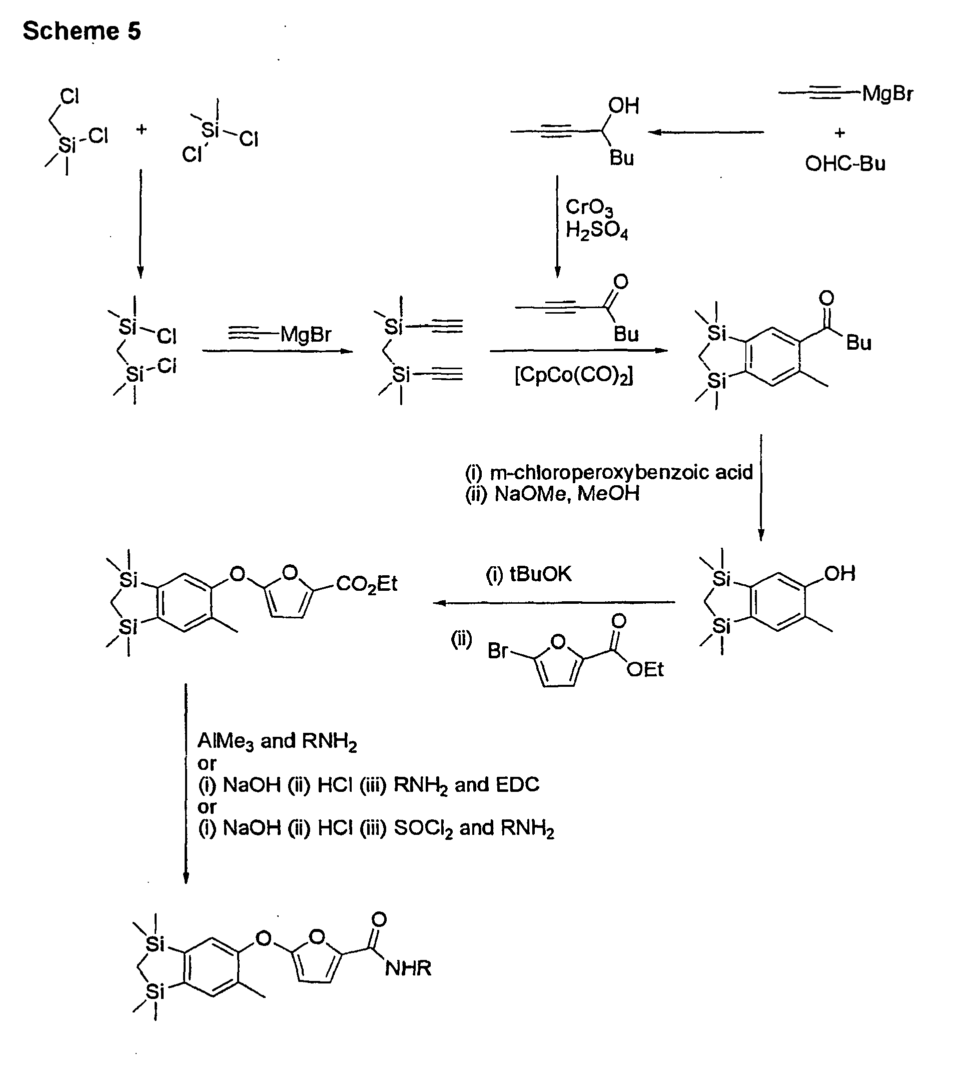

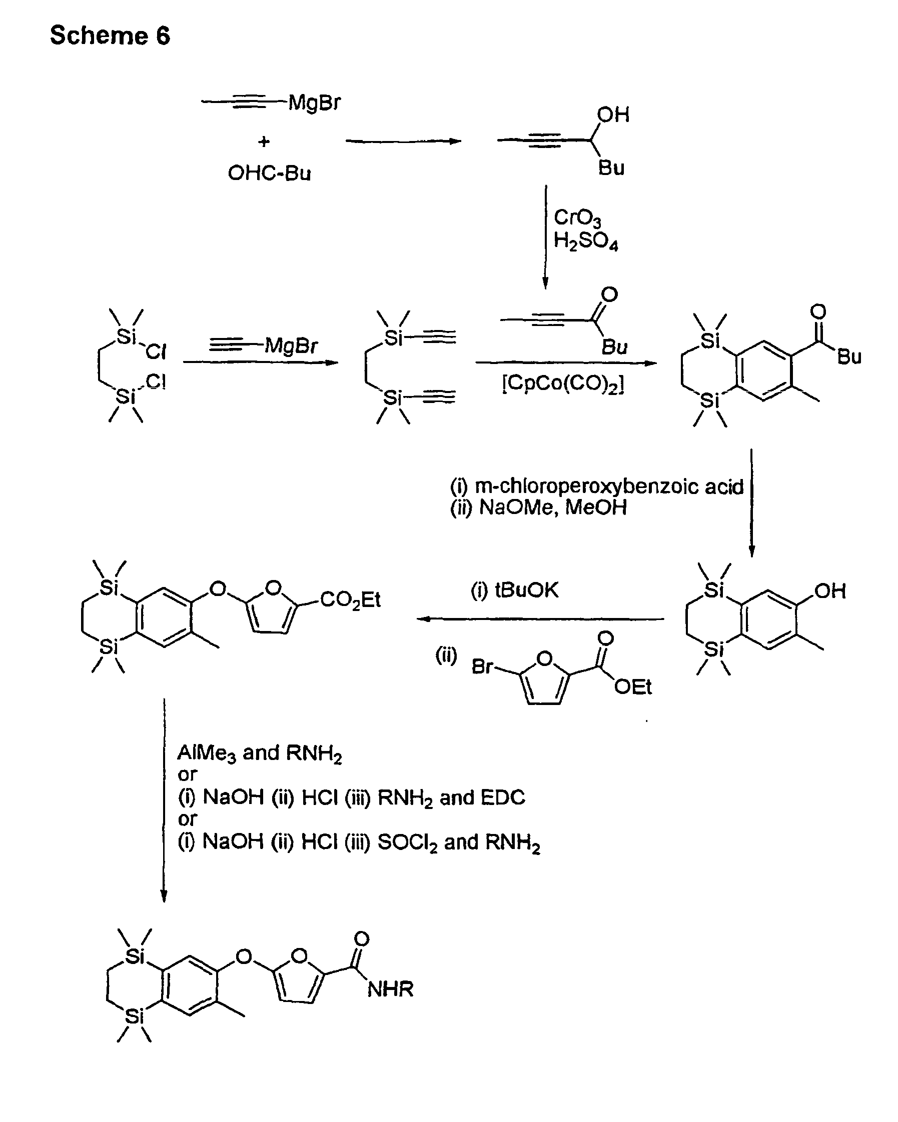

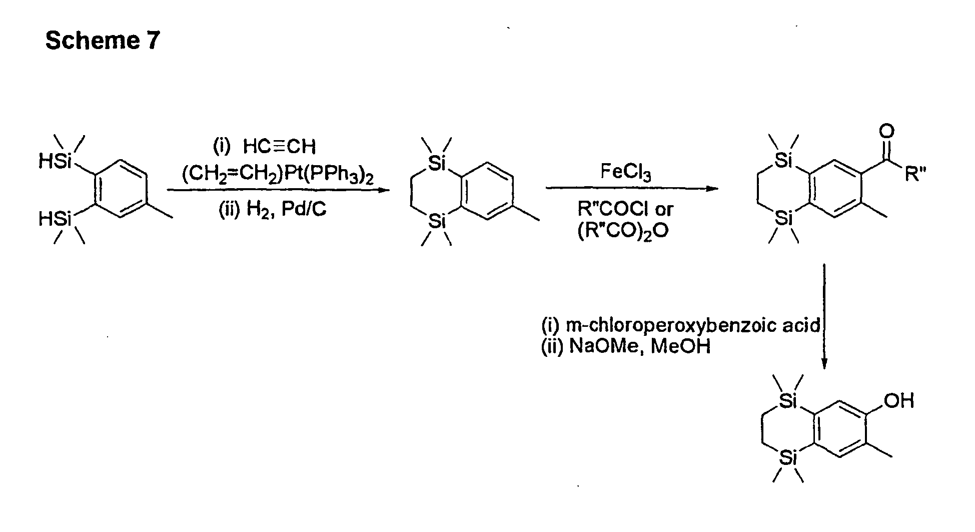

It will be understood that the processes detailed above are solely for the purpose of illustrating the invention and should not be construed as limiting. A process utilising similar or analogous reagents and/or conditions known to one skilled in the art may also be used to obtain a compound of the invention.

Any mixtures of final products or intermediates obtained can be separated on the basis of the physico-chemical differences of the constituents, in a known manner, into the pure final products or intermediates, for example by chromatography, distillation, fractional crystallisation, or by the formation of a salt if appropriate or possible under the circumstances.

The activity and selectivity of the compounds may be determined by any suitable assay known in the art.

The compounds of the invention may be used in the treatment of numerous ailments, conditions and diseases including, but not limited thereto, cancer, endometriosis, uterine myoma, an ovarian disease, a mammary cystic disease, prostatic hypertrophy, amenorrhea, precocious puberty, premenstrual syndrome, a sex-steroid-dependent pathophysiology, benign prostatic hyperplasia, Alzheimer's disease, HIV infection, AIDS and diseases caused by thyroid malfunction, or to arrest spermatogenesis.

The term "cancer" as used herein refers to any disease or condition characterised by uncontrolled, abnormal growth of cells and includes all known types of cancer, for example cancer of the bladder, breast, colon, brain, bone, head, blood, eye, neck, skin, lungs, ovaries, prostate and rectum; digestive, gastrointestinal, endometrial, hematological, AIDS-related, muscoskeletal, neurological and gynecological cancers; lympomas, melanomas and leukaemia.

In therapeutic use, the active compound may be administered orally, rectally, intra-vaginally, parenterally, by inhalation (pulmonary delivery), topically, ocularly, nasally, or to the buccal cavity. Oral administration is preferred. Thus, the therapeutic compositions of the present invention may take the form of any of the known pharmaceutical compositions for such methods of administration. The compositions may be formulated in a manner known to those skilled in the art so as to give a controlled release, for example rapid release or sustained release, of the compounds of the present invention. Pharmaceutically acceptable carriers suitable for use in such compositions are well known in the art. The compositions of the invention may contain 0.1-99% by weight of active compound. The compositions of the invention are generally prepared in unit dosage form. Preferably, a unit dose comprises the active ingredient in an amount of 1-500 mg. The excipients used in the preparation of these compositions are the excipients known in the art.

Appropriate dosage levels may be determined by any suitable method known to one skilled in the art. It will be understood, however, that the specific dose level for any particular patient will depend upon a variety of factors including the activity of the specific compound employed, the age, body weight, general health, sex, diet, time of administration, route of administration, rate of excretion, drug combination and the severity of the disease undergoing treatment.

Compositions for oral administration are preferred compositions of the invention and there are known pharmaceutical forms for such administration, for example tablets, capsules, granules, syrups and aqueous or oily suspensions. The pharmaceutical composition containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions, and such compositions may contain one or more agents selected from the group consisting of sweetening agents, flavouring agents, colouring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example corn starch or alginic acid; binding agents, for example starch gelatin, acacia, microcrystalline cellulose or polyvinyl pyrrolidone; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredient is mixed with an inert solid diluent, for example calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinyl pyrrolidone, gum tragacanth and gum acacia; dispersing or wetting agents may be a naturally occurring phosphatide, for example lecithin, or condensation products of an alkylene oxide with fatty acids, for example polyoxyethylene stearate, or condensation products of ethylene oxide with long-chain aliphatic alcohols, for example heptadecaethyleneoxycetanol, or condensation products of ethylene oxide with partial esters derived from fatty acids, for example polyoxyethylene sorbitan monooleate. The aqueous suspensions may also contain one or more preservatives, for example ethyl or n-propyl p-hydroxybenzoate, one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents, such as those set forth above, and flavouring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable sweetening, flavouring and colouring agents may also be present.

The pharmaceutical compositions of the invention may also be in the form of oil-in-water emulsions. The oily phase may be a vegetable oil, for example olive oil or arachis oil, or a mineral oil, for example liquid paraffin, or mixtures of these. Suitable emulsifying agents may be naturally occurring gums, for example gum acacia or gum tragacanth, naturally occurring phosphatides, for example soya bean, lecithin, and esters or partial esters derived from fatty acids and hexitol anhydrides, for example sorbitan monooleate and condensation products of the said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, a preservative and flavouring and colouring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be in a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid, find use in the preparation of injectables.

The compounds of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

Compositions for topical administration are also suitable for use in the invention. The pharmaceutically active compound may be dispersed in a pharmaceutically acceptable cream, ointment or gel. A suitable cream may be prepared by incorporating the active compound in a topical vehicle such as light liquid paraffin, dispersed in an aqueous medium using surfactants. An ointment may be prepared by mixing the active compound with a topical vehicle such as a mineral oil or wax. A gel may be prepared by mixing the active compound with a topical vehicle comprising a gelling agent. Topically administrable compositions may also comprise a matrix in which the pharmaceutically active compounds of the present invention are dispersed so that the compounds are held in contact with the skin in order to administer the compounds transdermally.

The following Examples illustrate the invention.

In the Examples, all syntheses were carried out under dry nitrogen. Tetrahydrofuran (THF), diethyl ether, dichloromethane, toluene and *m*-xylene were dried and purified according to standard procedures and stored under nitrogen. Light petroleum refers to the fraction with b.p. 40-60 °C. Thin layer chromatography was performed on silica (SiO₂) plates. ¹H NMR spectra were generated at 400 MHz in CDCl₃ unless otherwise stated.

### Intermediate 1: Bis(chlorodimethylsilyl)methane

This compound was synthesised according to Greber et. al, Makromol. Chem., 1962, 52, 174-183.

### Intermediate 2: Bis(ethynyldimethylsilyl)methane

This compound was synthesised according to Kusumoto *et. al, Chem. Lett.*, **1988,** 1149-1152.

### Intermediate 3: Methyl 5-(but-2-ynyl)-2-furoate

This compound was synthesised according to the method disclosed in WO-A-04/045625.

### Intermediate 4: Methyl 5-[(1,1,3,3,6-pentamethyl-1,3-disila-2,3-dihydro-1H-inden-5-yl)methyl]-2-furoate

A solution of cyclopentadienylcobaltdicarbonyl [CpCo(CO)₂; 328 mg, 1.82 mmol] in *m*-xylene (90 mL) was added dropwise within 14 hours to a stirred boiling mixture of bis(ethynyldimethylsilyl)methane (**Intermediate 2**, 2.02 g, 11.2 mmol) and methyl 5-(but-2-ynyl)-2-furoate (**Intermediate 3**, 2.00 g, 11.2 mmol) in *m*-xylene (20 mL). (To avoid heating of the CpCo(CO)₂ solution before its addition, the dropping funnel containing this catalyst was separated from the refluxing reaction mixture by a glass tube (length, 20 cm), through which the CpCo(CO)₂ solution was allowed to drop freely into the refluxing mixture.) The solvent was removed under reduced pressure, and the residue was purified by column chromatography on silica gel [column dimensions, 60 x 3.5 cm; silica gel, 300 g (32-63 µm, ICN 02826); eluent, *n*-hexane/ethyl acetate (95:5)]. The relevant fractions (GC control) were combined, the solvent was removed under reduced pressure, and the residue was dried *in vacuo* (15 mbar, 20 °C, 30 minutes) to give 2.62 g of a yellowish oil, which was crystallised from boiling *n*-hexane (17 mL, crystallisation at -20 °C over a period of 3 days). The product was recrystallised under the same conditions, and the resulting solid was isolated by decantation, washed with cold (20 °C) *n*-hexane (8 mL), and dried *in vacuo* (0.001 mbar, 20°C, 2 hours) to give the title compound as a colourless crystalline solid (1.78 g, 44 %); m.p. 111 °C. Anal. Calcd for C₁₉H₂₆O₃Si₂: C, 63.64; H, 7.31. Found: C, 63.7; H, 7.2.

### Intermediate 5: 2,4,6-Trimethoxyaniline hydrochloride

This compound was synthesised according to the method disclosed in WO-A-04/045625.

### Intermediate 6: oct-2-yn-4-ol

To a solution of 1-propynylmagnesium bromide (0.5 M in THF, 100 mL) was added valeraldehyde (5.84 mL, 1.1 mole equiv.) in dry THF (100 mL) at room temperature with stirring over a period of 30 minutes. The reaction was allowed to stir for a further 3 hours at room temperature before being quenched with a mixture of ice and saturated aqueous ammonium chloride (1:1, 120 mL). The organic phase was separated and the aqueous portion was extracted with light petroleum (3 x 100 mL) before the combined organic portions were dried (magnesium sulphate) and concentrated *in vacuo.* The resulting pale yellow oil (7.28 g) was essentially homogeneous and used in the next step without further purification. R_{f} 0.19 (10 % diethyl ether-light petroleum). ¹H NMR δ_{H} 0.92 (3 H, t, *J* = 7.1 Hz), 1.31-1.49 (4 H, m), 1.65-1.79 (2 H, m), 1.86 (3 H, s) and 4.34 (1 H, br, s).

### Intermediate 7: oct-2-yn-4-one

To a solution of oct-2-yn-4-ol (**Intermediate 6,** 2.41 g) in acetone (30 mL) was added mixture of chromium trioxide (1.91 g), concentrated sulphuric acid (1.62 mL) and water (20 mL) dropwise over a period of 3 hours. After a further 30 minutes stirring at room temperature the reaction was diluted further with water (50 mL) and the product was isolated by extraction into ether (3 x 30 mL), drying (magnesium sulphate) and concentration. The desired material was purified by vacuum distillation (b.p. 61-63 °C at 8 mbar, 1.54 g). ¹H NMR (CDCl₃) δ_{H} 0.96 (3 H, t, *J* = 7.3 Hz), 1.27-1.41 (2 H, m), 1.61-1.69 (2 H, m), 2.05 (3 H, s) and 2.56 (2 H, t, *J* = 7.3 *Hz*).

### Intermediate 8: 1-(1,3,6-trimethyl-1,3-disila-2,3-dihydro-1H-inden-5-yl)-pentanone

To a mixture of bis(ethynyldimethylsilyl)methane (**Intermediate 2**, 1.40 g, 7.78 mmol), oct-2-yn-4-one (**Intermediate 7**, 0.96 g, 7.78 mmol) and dry xylene (100 mL) at reflux was added a solution of CpCo(CO)₂ (0.25 mole equiv., 0.241 mL) in dry xylene (40 mL), dropwise over a period of approximately 3 hours. The mixture was allowed to heat to reflux overnight before a further solution of CpCo(CO)₂(0.25 mole equiv., 0.241 mL) in dry xylene (40 mL) was added as before. Again the mixture was heated at reflux overnight. Upon cooling the volatiles were removed in vacuo and the residue was purified by column chromatography (SiO₂, 10% dichloromethane in light petroleum) to afford the desired compound (748 mgs, 32 %). LC-MS: m/z = 305 (M+H⁺). ¹H NMR δ_{H} 0.03 (2 H, s), 0.32 (6 H, s), 0.33 (6 H, s), 0.97 (3 H, t, J = 7.3), 1.38-1.47 (2 H, m), 1.68-1.75 (2 H, m) 2.48 (3 H, s), 2.93 (2 H, t, J = 7.32), 7.42 (1 H, S) and 7.72 (1 H, s).

### Intermediate 9: 1,1,3,3,6-pentamethyl-1,3-disila-2,3-dihydro-1H-inden-5-ol

To a solution of 1-(1,3,6-trimethyl-1,3-disila-2,3-dihydro-1*H*-inden-5-yl)-pentanone (**Intermediate 8,** 250 mg, 0.82 mmol) in dichloromethane (5 mL) was added 3-chloroperoxybenzoic acid (mCPBA, 190 mg; 0.82 mmol approximately 71 % mCPBA). The following day a further portion of mCPBA (190 mg; 0.82 mmol) was added to the reaction mixture, which was then heated to reflux overnight. The following day a colourless precipitate was removed by filtration before the mother liquors were reduced *in vacuo,* dissolved in methyl alcohol (10 mL) and treated with sodium methoxide (25 % by weight in methyl alcohol, 0.822 moles). After stirring at room temperature for 2.5 hours the mixture was concentrated, taken up into water (10 mL) and acidified with hydrochloric acid (1 M). The resulting mixture was extracted into ethyl acetate (3 x 25 mL) and the combined extracts were dried (magnesium sulphate) and concentrated. The desired phenol was obtained in a homogeneous state by column chromatography (SiO₂, 10 % to 25 % diethyl ether in light petroleum) as an off-white solid (10 mg). R_{f} = 0.43 (25 % diethyl ether in light petroleum). ¹H NMR (CDCl₃) δ -0.05 (2 H, s), 0.29 (12 H, s), 2.29 (3 H, s), 4.75 (1 H, s, br), 6.96 (1 H, S) and 7.35 (1 H, s).

### Intermediate 10: 1,1,4,4,7-pentamethyl-1,4-disila-1,2,3,4-tetrahydronauhthalen-6-ol

This compound was prepared according to the procedures described for **Intermediate 9**. R_{f} 0.36 (10 % diethyl ether in light petroleum). ¹H NMR δ_{H} 0.200 (6 H, s), 0.203 (6 H, s), 0.97 (4 H, s), 2.25 (3 H, s), 4.70 (1 H, s), 6.89 (1 H, s) and 7.25 (1 H, s).

### Example 1: 5-[(1,1,3,3,6-pentamethyl-1,3-disila-2,3-dihydro-1H-inden-5-yl)methyl]-N-(2,4,6-trimethoxyphenyl)furan-2-carboxamide

A solution of trimethylaluminium (115 mg, 1.60 mmol) in toluene (5 mL) was added dropwise at -30 °C within 10 minutes to a stirred suspension of 2,4,6-trimethoxyaniline hydrochloride (351 mg, 1.60 mmol) in toluene (5 mL) (dissolution of 2,4,6-trimethoxyaniline hydrochloride, followed by the formation of a precipitate). The stirred mixture was warmed to 0 °C within 1 hour and then to 20 °C within a further 1 hour (dissolution of the precipitate), and the resulting solution was then added dropwise at 0 °C within 10 minutes to a stirred solution of methyl 5-[(1,1,3,3,6-pentamethyl-1,3-disila-2,3-dihydro-1*H-*inden-5-yl)methyl]-2-furoate (**Intermediate 4,** 359 mg, 1.00 mmol) in dichloromethane (10 mL). The resulting mixture was stirred at 0 °C for 30 minutes and then at 20 °C for 3 days [almost quantitative conversion (HPLC control), change of colour from colourless to black], followed by the addition of a half-saturated aqueous ammonium acetate solution (20 mL) (formation of a precipitate). The precipitate was separated by filtration and washed with ethyl acetate (10 mL), the filtrate and wash solutions were combined, and the organic layer was separated and washed with water (20 mL). The resulting aqueous wash solutions (A and B) were extracted with ethyl acetate (2 x 20 mL) as follows: The first aqueous wash solution (A) was extracted with ethyl acetate (20 mL), the resulting extract was then used to extract the second aqueous wash solution (B), and the organic extract was separated, followed by a second extraction of the wash solutions A and B with a fresh portion of ethyl acetate (20 mL), using the same protocol as described for the first extraction sequence. All organic extracts were combined and dried over anhydrous sodium sulphate, the solvent was removed under reduced pressure and the residue was purified by column chromatography on silica gel [column dimensions, 60 x 3.5 cm; silica gel, 300 g (32-63 µm, ICN 02826); eluent, ethyl acetate/*n*-hexane (55:45)]. The relevant fractions (TLC control) were combined, and the solvent was removed under reduced pressure. The residue was crystallised at 20 °C by vapour diffusion of *n*-pentane (200 mL) into a solution of the crude material in diethyl ether (10 mL) over a period of 7 days. The product was recrystallised under the same conditions and the resulting solid was isolated by decantation, washed with *n*-pentane (5 mL), and dried *in vacuo* (0.001 mbar, 20°C, 2 hours) to give the title compound in 70% yield as a colourless crystalline solid (357 mg, 0.700 mmol); m.p. 105°C. Anal. Calcd for C₂₇H₃₅NO₅Si₂: C, 63.62; H, 6.92; N, 2.75. Found: C, 63.6; H, 6.9; N, 2.8.

### Example 2: 5-(1,1,3,3,6-pentamethyl-1,3-disila-2,3-dihydro-1H-inden-5-yloxy)-N-{2-[3-(morpholin-4-yl)propylamino]-4,6-dimethoxy-1,3-pyrimidin-5-yl}furan-2-carboxamide

This compound can be obtained from 4,6-dimethoxy-*N*²-(3-morpholinopropyl)pyrimidine-2,5-diamine (which can be synthesised according to the procedures described in WO-A-02/098363) and **Intermediate 9**, using the procedures illustrated by **Scheme 5** herein.

### Example 3: 5-(1,1,4,4,7-pentamethyl-1,4-disila-1,2,3,4-tetrahydronaphthalen-6-yloxy)-N-{2-[3-(4-methylpiperazin-1-yl)propylamino]-4,6-dimethoxy-1,3-pyrimidin-5-yl}furan-2-carboxamide

This compound can be obtained from 4,6-dimethoxy-*N*²-[3-(4-methylpiperazin-1-yl)propyl]pyrimidine-2,5-diamine (which can be synthesised according to the procedures described in WO-A-02/098363) and **Intermediate 10**, according to the procedures illustrated by **Scheme 6** herein.

## Claims

1. A compound of formula (I) or formula (II) 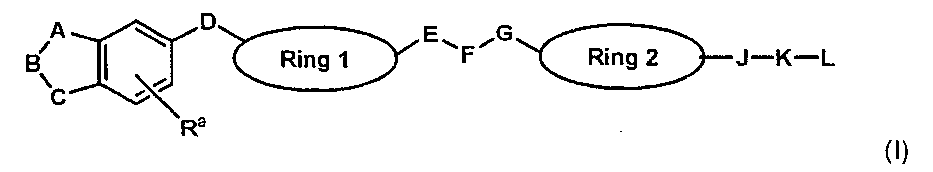 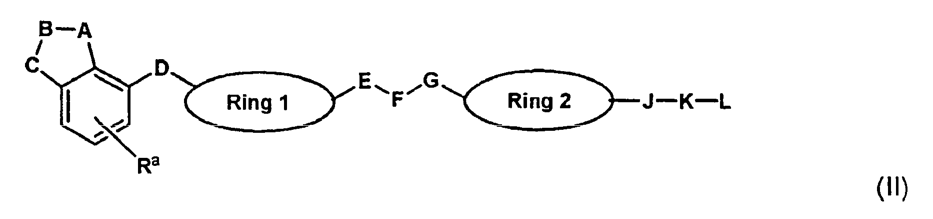 wherein
A and C are the same or different and are each a bond, -(CH₂)ₙ-, -C(R^{b})₂-, -Si(R^{c})₂-, -O-, -S(O)ₘ-, -N=, -N(R^{b})-, -N(R^{e})C(=X)- or -C(=X)-;
B is -(CH₂)ₙ-, -O-, -C(R^{b})₂-, -Si(R^{c})₂-, -C(R^{b})=C(R^{b})-, -C(R^{b})=, -(CH₂)ₙC(R^{g})₂-, -C(R^{g})₂(CH₂)ₙ- or -CH(R^{b})CH(R^{b})-;
wherein any of A, B and C is optionally substituted with -Si(R^{c})₃;
D is -(CH₂)ₙ-, -C(=X)-, -O-, -S(O)ₘ-, -C(=X)N(R^{e})-, -C(R^{b})₂-, -C(R^{b})=C(R^{b})-, -CH(R^{b})CH(R^{b})-;
E is optionally present and is -(CH₂)ₙ-, -N(R^{d})-, -(CH₂)ₙN(R^{d})- or -N(R^{d})(CH₂)ₙ-;
F is -C(=_{X})- or -N(R^{d})-;
G is -(CH₂)ₙ-, -N(R^{d})-, -(CH₂)ₙN(R^{d})- or -N(R^{d})(CH₂)ₙ;
J is optionally present and is -O-, -N(R^{c})C(=X)-, -C(=X)N(R^{c})-, -S(O)ₘ-, -N(R^{c})S(O)ₘ-, -S(O)ₘN(R^{c})- or -N(R^{e})-;
K is optionally present and is alkylene optionally substituted with R^{b}; or K is cycloalkylene, cycloalkenylene, arylene, heterocycloalkylene, heterocycloalkylene or heteroarylene, any of which is optionally substituted with R^{a};
L is hydrogen, halogen, -N(R^{f})₂, cycloalkyl, cycloalkenyl, aryl, heterocycloalkyl, heterocycloalkenyl or heteroaryl, any of which is optionally substituted with R^{a}, -C(=X)OR^{d}, -OH, -OR^{c}, -C(=X)N(R^{b})(R^{c}), -S(O)ₘN(R^{b})(R^{c}) or -CN;
each R^{a} is the same or different and is hydrogen, halogen, alkyl, aryl, hydroxy, alkoxy, -alkoxy-(CH₂)ₙC(O)₂R^{b}, -O-aryl, -C(=X)R^{c}, -NO₂, -CN, -N(R^{c})C(=X)R^{c}, -C(=X)N(R^{c})₂, -S(O)₂N(R^{c})₂ or -N(R^{e})₂;
each R^{b} is the same or different and is hydrogen or alkyl;
each R^{c} is the same or different and is alkyl, cycloalkyl, -alkyl-aryl, -alkyl-cycloalkyl or aryl optionally substituted with R^{a};
each R^{d} is the same or different and is hydrogen, alkyl or aryl optionally substituted with R^{a};
each R^{e} is the same or different and is hydrogen or alkyl; or R^{e} is aryl or heteroaryl, either of which is optionally substituted with R^{a};
each R^{f} is the same or different and is hydrogen or alkyl; or R^{f}-N-R^{f} taken together form heterocycloalkyl, heterocycloalkenyl or heteroaryl;
R⁹-C-R⁹ taken together form heterocyclyl;
each X is the same or different and is oxygen or sulphur;
Rings 1 and 2 are the same or different and are each arylene or heteroarylene, either of which is optionally substituted with R^{a};
each m is the same or different and is 0, 1 or 2; and
each n is the same or different and is 0, 1, 2, or 3;
with the provisos that at least one of A, B and C comprises a silicon atom; A and C are not each a bond; and the compound does not comprise a Si-Si bond, a N-N single bond, a Si-O bond, a Si-N bond or a N-O-N linkage;
with the further proviso that the compound is not of the formula 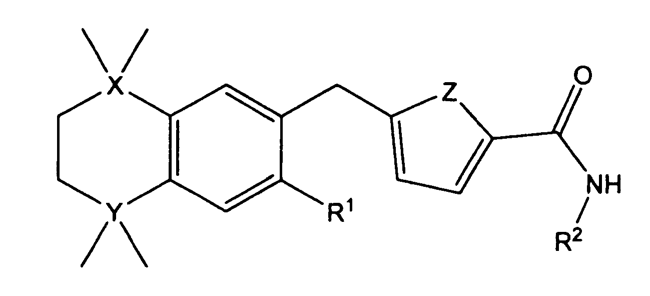 wherein
one of X and Y is silicon, and the other is carbon or silicon;
Z is oxygen, sulphur or -N(R)-, wherein R is hydrogen or alkyl;
R¹ is hydrogen, halogen, alkyl, alkenyl, alkynyl, alkoxy or cycloalkyl; and
R² is alkyl, alkenyl, alkynyl, cycloalkyl, heterocycloalkyl, aryl, heteroaryl, -alkyl-cycloalkyl, -alkyl-heterocycloalkyl, -alkyl-aryl or -alkyl-heteroaryl;
or a pharmaceutically acceptable salt thereof.

2. A compound according to claim 1, which is of formula (I).

3. A compound according to claim 1 or claim 2, wherein A and/or C is -Si(R^{c})₂-.

4. A compound according to claim 3, wherein each R^{c} is the same or different and is alkyl..

5. A compound according to claim 1 or claim 2, wherein A and/or C is -C(R^{b})₂-, -(CH₂)ₙ-, -N(R^{b})- or -O-.

6. A compound according to claim 5, wherein each R^{b} is the same or different and is hydrogen or alkyl.

7. A compound according to any preceding claim, wherein B is -(CH₂)ₙ-, -C(R^{b})₂-, -CH(R^{b})CH(R^{b})-, -C(R^{b})=C(R^{b})-, or -CH₂-C(R^{g})₂-.

8. A compound according to claim 7, wherein each R^{b} is the same or different and is hydrogen or alkyl.

9. A compound according to any preceding claim, wherein D is -O-, -S- or -CH₂-.

10. A compound according to any preceding claim, wherein E is absent.

11. A compound according to any preceding claim, wherein F is -C(O)-.

12. A compound according to any preceding claim, wherein G is -N(R^{d})-.

13. A compound according to claim 12, wherein R^{d} is hydrogen.

14. A compound according to any preceding claim, wherein J and K are absent, and L is hydrogen or -N(R^{f})₂.

15. A compound according to any of claims 1 to 13, wherein J is -NH-, K is alkylene and L is heterocycloalkyl.

16. A compound according to any preceding claim, wherein Ring 1 is heteroarylene.

17. A compound according to claim 16, wherein Ring 1 is furanylene.

18. A compound according to any preceding claim, wherein Ring 2 is phenyl, pyrimidinyl or pyridinyl, any of which is optionally substituted.

19. A compound according to claim 18, wherein Ring 2 is substituted 1, 2 or 3 times, the substituents being the same or different in each occurrence and selected from alkoxy and halogen.

20. A compound according to claim 1, selected from:
5-(1,1,4,4,7-pentamethyl-1,4-disila-1,2,3,4-tetrahydronaphthalen-6-yloxy)-*N*-(2,6-dimethoxyphenyl)furan-2-carboxamide;
5-(1,1,4,4,7-pentamethyl-1,4-disila-1,2,3,4-tetrahydronaphthalen-6-yloxy)-*N*-(2,4,6-trimethoxyphenyl)furan-2-carboxamide;
5-(1,1,4,4,7-pentamethyl-1,4-disila-1,2,3,4-tetrahydronaphthalen-6-yloxy)-*N*-(2,4,6-trimethoxy-1,3-pyrimidin-5-yl)furan-2-carboxamide;
5-(1,1,4,4,7-pentamethyl-1,4-disila-1,2,3,4-tetrahydronaphthalen-6-yloxy)-*N*-(2-methylamino-4,6-dimethoxy-1,3-pyrimidin-5-yl)furan-2-carboxamide;
5-(1,1,4,4,7-pentamethyl-1,4-disila-1,2,3,4-tetrahydronaphthalen-6-yloxy)-*N*-{2-[3-(4-methylpiperazin-1-yl)propylamino]-4,6-dimethoxy-1,3-pyrimidin-5-yl}furan-2-carboxamide;
5-(1,1,4,4,7-pentamethyl-1,4-disila-1,2,3,4-tetrahydronaphthalen-6-yloxy)-*N*-{2-[3-(*N,N*-dimethylamino)propylamino]-4,6-dirnethoxy-1,3-pyrimidin-5-yl}furan-2-carboxamide;
5-(1,1,4,4,7-pentamethyl-1,4-disila-1,2,3,4-tetrahydronaphthalen-6-yloxy)-*N*-{2-[3-(morpholin-4-yl)propylamino]-4,6-dimethoxy-1,3-pyrimidin-5-yl}furan-2-carboxamide;
5-(1,1,6-trimethyl-1-sila-1,2,3,4-tetrahydronaphthaten-7-yloxy)-*N*-(2,6-dimethoxyphenyl)furan-2-carboxamide;
5-(1,1,6-trimethyl-1-sila-1,2,3,4-tetrahydronaphthalen-7-yloxy)-*N*-(2,4,6-trimethoxyphenyl)furan-2-carboxamide;
5-(1,1,6-trimethyl-1-sila-1,2,3,4-tetrahydronaphthalen-7-yloxy)-*N*-(2,4,6-trimethoxy-1,3-pyrimidin-5-yl)furan-2-carboxamide;
-5-(1,1,6-trimethyl-1-sila-1,2,3,4-tetrahydronaphthalen-7-yloxy)-*N*-(2-methylamino-4,6-dimethoxy-1,3-pyrimidin-5-yl)furan-2-carboxamide;
5-(1,1,6-trimethyl-1-sila-1,2,3,4-tetrahydronaphthalen-7-yloxy)-*N*-{2-[3-(4-methylpiperazin-1-yl)propylamino]-4,6-dimethoxy-1,3-pyrimidin-5-yl}furan-2-carboxamide;
5-(1,1,6-trimethyl-1-sila-1,2,3,4-tetrahydronaphthalen-7-yloxy)-*N-*{2-[3-(*N,N*-dimethylamino)propylamino]-4,6-dimethoxy-1,3-pyrimidin-5-yl}furan-2-carboxamide;
5-(1,1,6-trimethyl-1-sila-1,2,3,4-tetrahydronaphthalen-7-yloxy)-*N*-{2-[3-(morpholin-4-yl)propylamino]-4,6-dimethoxy-1,3-pyrimidin-5-yl}furan-2-carboxamide;
5-(1,1,7-trimethyl-1-sila-1,2,3,4-tetrahydronaphthalen-6-yloxy)-*N*-(2,6-dimethoxyphenyl)furan-2-carboxamide;
5-(1,1,7-trimethyl-1-sila-1,2,3,4-tetrahydronaphthalen-6-yloxy)-*N*-(2,4,6-trimethoxyphenyl)furan-2-carboxamide;
5-(1,1,7-trimethyl-1-sila-1,2,3,4-tetrahydronaphthalen-6-yloxy)-*N*-(2,4,6-trimethoxy-1,3-pyrimidin-5-yl)furan-2-carboxamide;
5-(1,1,7-trimethyl-1-sila-1,2,3,4-tetrahydronaphthalen-6-yloxy)-*N*-(2-methylamino-4,6-dimethoxy-1,3-pyrimidin-5-yl)furan-2-carboxamide;
5-(1,1,7-trimethyl-1-sila-1,2,3,4-tetrahydronaphthalen-6-yloxy)-*N*-{2-[3-(4-methylpiperazin-1-yl)propylamino]-4,6-dimethoxy-1,3-pyrimidin-5-yl}furan-2-carboxamide;
5-(1,1,7-trimethyl-1-sila-1,2,3,4-tetrahydronaphthalen-6-yloxy)-*N*-{2-[3-(*N,N*-dimethylamino)propylamino]-4,6-dimethoxy-1,3-pyrimidin-5-yl}furan-2-carboxamide;
5-(1,1,7-trimethyl-1-sila-1,2,3,4-tetrahydronaphthalen-6-yloxy)-*N*-{2-[3-(morpholin-4-yl)propylamino]-4,6-dimethoxy-1,3-pyrimidin-5-yl}furan-2-carboxamide;
5-(1,1,2,4,4,7-hexamethyl-1,4-disila-1,2,3,4-tetrahydronaphthalen-6-yloxy)-*N*-(2,6-dimethoxyphenyl)furan-2-carboxamide;
5-(1,1,2,4,4,7-hexamethyl-1,4-disila-1,2,3,4-tetrahydronaphthalen-6-yloxy)-*N*-(2,4,6-trimethoxyphenyl)furan-2-carboxamide;
5-(1,1,2,4,4,7-hexamethyl-1,4-disila-1,2,3,4-tetrahydronaphthalen-6-yloxy)-*N*-(2,4,6-trimethoxy-1,3-pyrimidin-5-yl)furan-2-carboxamide;
5-(1,1,2,4,4,7-hexamethyl-1,4-disila-1,2,3,4-tetrahydronaphthalen-6-yloxy)-*N*-(2-methylamino-4,6-dimethoxy-1,3-pyrimidin-5-yl)furan-2-carboxamide;
5-(1,1,2,4,4,7-hexamethyl-1,4-disila-1,2,3,4-tetrahydronaphthalen-6-yloxy)-*N*-{2-[3-(4-methylpiperazin-1-yl)propylamino]-4,6-dimethoxy-1,3-pyrimidin-5-yl}furan-2-carboxamide;
5-(1,1,2,4,4,7-hexamethyl-1,4-disila-1,2,3,4-tetrahydronaphthalen-6-yloxy)-*N*-{2-[3-(*N,N*-dimethylamino)propylamino]-4,6-dimethoxy-1,3-pyrimidin-5-yl}furan-2-carboxamide;
5-(1,1,2,4,4,7-hexamethyl-1,4-disila-1,2,3,4-tetrahydronaphthalen-6-yloxy)-*N*-{2-[3-(morpholin-4-yl)propylamino]-4,6-dimethoxy-1,3-pyrimidin-5-yl}furan-2-carboxamide;
5-(5-methoxy-1,1,4,4,7-pentamethyl-1,4-disita-1,2,3,4-tetrahydronaphthalen-6-yloxy)-*N*-(2,6-dimethoxyphenyl)furan-2-carboxamide;
5-(5-methoxy-1,1,4,4,7-pentamethyl-1,4-disila-1,2,3,4-tetrahydronaphthalen-6-yloxy)-*N*-(2,4,6-trimethoxyphenyl)furan-2-carboxamide;
5-(5-methoxy-1,1,4,4,7-pentamethyl-1,4-disila-1,2,3,4-tetrahydronaphthalen-6-yloxy)-*N*-(2,4,6-trimethoxy-1,3-pyrimidin-5-yl)furan-2-carboxamide;
5-(5-methoxy-1,1,4,4,7-pentamethyl-1,4-disila-1,2,3,4-tetrahydronaphthalen-6-yloxy)-*N*-(2-methylamino-4,6-dimethoxy-1,3-pyrimidin-5-yl)furan-2-carboxamide;
5-(5-methoxy-1,1,4,4,7-pentamethyl-1,4-disila-1,2,3,4-tetrahydronaphthalen-6-yloxy)-*N*-{2-[3-(4-methylpiperazin-1-yl)propylamino]-4,6-trimethoxy-1,3-pyrimidin-5-yl}furan-2-carboxamide;
5-(5-methoxy-1,1,4,4,7-pentamethyl-1,4-disila-1,2,3,4-tetrahydronaphthalen-6-yloxy)-*N*-{2-[3-(*N,N*-dimethylamino)propylamino]-4,6-dimethoxy-1,3-pyrimidin-5-yl}furan-2-carboxamide;
5-(5-methoxy-1,1,4,4,7-pentamethyl-1,4-disila-1,2,3,4-tetrahydronaphthalen-6-yloxy)-*N*-{2-[3-(morpholin-4-yl)propylamino]-4,6-dimethoxy-1,3-pyrimidin-5-yl}furan-2-carboxamide;
5-(8-methoxy-1,1,6-trimethyl-1-sila-1,2,3,4-tetrahydronaphthalen-7-yloxy)-*N*-(2,6-dimethoxyphenyl)furan-2-carboxamide;
5-(8-methoxy-1,1,6-trimethyl-1-sila-1,2,3,4-tetrahydronaphthalen-7-yloxy)-*N*-(2,4,6-trimethoxyphenyl)furan-2-carboxamide;
5-(8-methoxy-1,1,6-trimethyl-1-sila-1,2,3,4-tetrahydronaphthalen-7-yloxy)-*N*-(2,4,6-trimethoxy-1,3-pyrimidin-5-yl)furan-2-carboxamide;
5-(8-methoxy-1,1,6-trimethyl-1-sila-1,2,3,4-tetrahydronaphthalen-7-yloxy)-*N*-(2-methylamino-4,6-dimethoxy-1,3-pyrimidin-5-yl)furan-2-carboxamide;
5-(8-methoxy-1,1,6-trimethyl-1-sila-1,2,3,4-tetrahydronaphthalen-7-yloxy)-*N*-{2-[3-(4-methylpiperazin-1-yl)propylamino]-4,6-dimethoxy-1,3-pyrimidin-5-yl}furan-2-carboxamide;
5-(8-methoxy-1,1,6-trimethyl-1-sila-1,2,3,4-tetrahydronaphthalen-7-yloxy)-*N*-{2-[3-(*N,N*-dimethylamino)propylamino]-4,6-dimethoxy-1,3-pyrimidin-5-yl}furan-2-carboxamide;
5-(8-methoxy-1,1,6-trimethyl-1-sila-1,2,3,4-tetrahydronaphthalen-7-yloxy)-*N*-{2-[3-(morpholin-4-yl)propylamino]-4,6-dimethoxy-1,3-pyrimidin-5-yl}furan-2-carboxamide;
5-(1,1,4,4,5,7-hexamethyl-1,4-disila-1,2,3,4-tetrahydronaphthalen-6-yloxy)-*N*-(2,6-dimethoxyphenyl)furan-2-carboxamide;
5-(1,1,4,4,5,7-hexamethyl-1,4-disila-1,2,3,4-tetrahydronaphthalen-6-yloxy)-*N*-(2,4,6-trimethoxyphenyl)furan-2-carboxamide;
5-(1,1,4,4,5,7-hexamethyl-1,4-disila-1,2,3,4-tetrahydronaphthalen-6-yloxy)-*N*-(2,4,6-trimethoxy-1,3-pyrimidin-5-yl)furan-2-carboxamide;
5-(1,1,4,4,5,7-hexamethyl-1,4-disila-1,2,3,4-tetrahydronaphthalen-6-yloxy)-*N*-(2-methylamino-4,6-dimethoxy-1,3-pyrimidin-5-yl)furan-2-carboxamide;
5-(1,1,4,4,5,7-hexamethyl-1,4-disila-1,2,3,4-tetrahydronaphthalen-6-yloxy)-*N*-{2-[3-(4-methylpiperazin-1-yl)propylamino]-4,6-dimethoxy-1,3-pyrimidin-5-yl}furan-2-carboxamide;
5-(1,1,4,4,5,7-hexamethyl-1,4-disila-1,2,3,4-tetrahydronaphthalen-6-yloxy)-*N*-{2-[3-(*N,N*-dimethylamino)propylamino]-4,6-dimethoxy-1,3-pydmidin-5-yl}furan-2-carboxamide;
5-(1,1,4,4,5,7-hexamethyl-1,4-disila-1,2,3,4-tetrahydronaphthalen-6-yloxy)-*N-*2-{3-(morpholin-4-yl)propylamino]-4,6-dimethoxy-1,3-pyrimidin-5-yl}furan-2-carboxamide;
5-(6-methoxy-1,1,4,4,8-pentamethyl-1,4-disila-1,2,3,4-tetrahydronaphthalen-7-yloxy)-*N*-(2,6-dimethoxyphenyl)furan-2-carboxamide;
5-(6-methoxy-1,1,4,4,8-pentamethyl-1,4-disila-1,2,3,4-tetrahydronaphthalen-7-yloxy)-*N*-(2,4,6-trimethoxyphenyl)furan-2-carboxamide;
5-(6-methoxy-1,1,4,4,8-pentamethyl-1,4-disila-1,2,3,4-tetrahydronaphthalen-7-yloxy)-*N*-(2,4,6-trimethoxy-1,3-pyrimidin-5-yl)furan-2-carboxamide;
5-(6-methoxy-1,1,4,4,8-pentamethyl-1,4-disila-1,2,3,4-tetrahydronaphthalen-7-yloxy)-*N*-(2-methylamino-4,6-dimethoxy-1,3-pyrimidin-5-yl)furan-2-carboxamide;
5-(6-methoxy-1,1,4,4,8-pentamethyl-1,4-disila-1,2, 3,4-tetrahydronaphthalen-7-yloxy)-*N*-{2-[3-(4-methylpiperazin-1-yl)propylamino]-4,6-dimethoxy-1,3-pyrimidin-5-yl}furan-2-carboxamide;
5-(6-methoxy-1,1,4,4,8-pentamethyl-1,4-disila-1,2,3,4-tetrahydronaphthalen-7-yloxy)-*N*-{2-[3-(*N,N*-dimethylamino)propylamino]-4,6-dimethoxy-1,3-pyrimidin-5-yl}furan-2-carboxamide;
5-(6-methoxy-1,1,4,4,8-pentamethyl-1,4-disila-1,2,3,4-tetrahydronaphthalen-7-yloxy)-*N*-{2-[3-(morpholin-4-yl)propylamino]-4,6-dimethoxy-1,3-pyrimidin-5-yl}furan-2-carboxamide;
5-(6-chloro-1,1,4,4-tetramethyl-1,4-disila-1,2,3,4-tetrahydronaphthalen-7-yloxy)-*N*-(2,6-dimethoxyphenyl)furan-2-carboxamide;
5-(6-chloro-1,1,4,4-tetramethyl-1,4-disila-1,2,3,4-tetrahydronaphthalen-7-yloxy)-*N*-(2,4,6-trimethoxyphenyl)furan-2-carboxamide;
5-(6-chloro-1,1,4,4-tetramethyl-1,4-disila-1,2,3,4-tetrahydronaphthalen-7-yloxy)-*N*-(2,4,6-trimethoxy-1,3-pyrimidin-5-yl)furan-2-carboxamide;
5-(6-chloro-1,1,4,4-tetramethyl-1,4-disila-1,2,3,4-tetrahydronaphthalen-7-yloxy)-*N*-(2-methylamino-4,6-dimethoxy-1,3-pyrimidin-5-yl)furan-2-carboxamide;
5-(6-chloro-1,1,4,4-tetramethyl-1,4-disila-1,2,3,4-tetrahydronaphthalen-7-yloxy)-*N*-{2-[3-(4-methylpiperazin-1-yl)propylamino]-4,6-dimethoxy-1,3-pyrimidin-5-yl}furan-2-carboxamide;
5-(6-chloro-1,1,4,4-tetramethyl-1,4-disila-1,2,3,4-tetrahydronaphthalen-7-yloxy)-*N*-{2-[3-(*N*,*N*-dimethylamino)propylamino]-4,6-dimethoxy-1,3-pyrimidin-5-yl}furan-2-carboxamide;
5-(6-chloro-1,1,4,4-tetramethyl-1,4-disila-1,2,3,4-tetrahydronaphthalen-7-yloxy)-*N*-{2-(3-morpholin-4-yl)propylamino]-4,6-dimethoxy-1,3-pyrimidin-5-yl}furan-2-carboxamide;
5-(6-chloro-1,1-dimethyl-1-sila-1,2,3,4-tetrahydronaphthalen-7-yloxy)-*N-*(2,6-dimethoxyphenyl)furan-2-carboxamide;
5-(6-chloro-1,1-dimethyl-1-sila-1,2,3,4-tetrahydronaphthalen-7-yloxy)-*N-*(2,4,6-trimethoxyphenyl)furan-2-carboxamide;
5-(6-chloro-1,1-dimethyl-1-sila-1,2,3,4-tetrahydronaphthalen-7-yloxy)-*N-*(2,4,6-trimethoxy-1,3-pyrimidin-5-yl)furan-2-carboxamide;
5-(6-chloro-1,1-dimethyl-1-sila-1,2,3,4-tetrahydronaphthalen-7-yloxy)-*N*-(2-methylamino-4,6-dimethoxy-1,3-pyrimidin-5-yl)furan-2-carboxamide;
5-(6-chloro-1,1-dimethyl-1-sila-1,2,3,4-tetrahydronaphthalen-7-yloxy)-*N*-{2-[3-(4-methylpiperazin-1-yl)propylamino]-4,6-dimethoxy-1,3-pyrimidin-5-yl}furan-2-carboxamide;
5-(6-chloro-1,-dimethyl-1-sila-1,2,3,4-tetrahydronaphthalen-7-yloxy)-*N*-{2-[3-(*N,N*-dimethylamino)propylamino]-4,6-dimethoxy-1,3-pyrimidin-5-yl}furan-2-carboxamide;
5-(6-chloro-1,1-dimethyl-1-sila-1,2,3,4-tetrahydronaphthalen-7-yloxy)-*N*-{2-[3-(morpholin-4-yl)propylamino]-4,6-dimethoxy-1,3-pyrimidin-5-yl}furan-2-carboxamide;
5-(4,4,7-trimethyl-4-sila-chroman-6-yloxy)-*N*-(2,6-dimethoxyphenyl)furan-2-carboxamide;
5-(4,4,7-trimethyl-4-sila-chroman-6-yloxy)-*N*-(2,4,6-trimethoxyphenyl)furan-2-carboxamide;
5-(4,4,7-trimethyl-4-sila-chroman-6-yloxy)-*N*-(2,4,6-trimethoxy-1,3-pyrimidin-5-yl)furan-2-carboxamide;
5-(4,4,7-trimethyl-4-sila-chroman-6-yloxy)-*N*-(2-methylamino-4,6-dimethoxy-1,3-pyrimidin-5-yl)furan-2-carboxamide;
5-(4,4,7-trimethyl-4-sila-chroman-6-yloxy)-*N*-{2-[3-(4-methylpiperazin-1-yl)propylamino]-4,6-dimethoxy-1,3-pyrimidin-5-yl}furan-2-carboxamide;
5-(4,4,7-trimethyl-4-sila-chroman-6-yloxy)-*N*-{2-[3-(*N,N-*dimethylamino)propylamino]-4,6-dimethoxy-1,3-pyrimidin-5-yl}furan-2-carboxamide;
5-(4,4,7-trimethyl-4-sila-chroman-6-yloxy)-*N*-{2-[3-(morpholin-4-yl)propylamino]-4,6-dimethoxy-1,3-pyrimidin-5-yl}furan-2-carboxamide;
5-(1,2,3,4-tetrahydro-4,4,7-trimethyl-4-sila-quinolin-6-yloxy)-*N*-(2,6-dimethoxyphenyl)furan-2-carboxamide;
5-(1,2,3,4-tetrahydro-4,4,7-trimethyl-4-sila-quinolin-6-yloxy)-*N*-(2,4,6-trimethoxyphenyl)furan-2-carboxamide;
5-(1,2,3,4-tetrahydro-4,4,7-trimethyl-4-sila-quinolin-6-yloxy)-*N*-(2,4,6-trimethoxy-1,3-pyrimidin-5-yl)furan-2-carboxamide;
5-(1,2,3,4-tetrahydro-4,4,7-trimethyl-4-sila-quinolin-6-yloxy)-*N*-(2-methylamino-4,6-dimethoxy-1,3-pyrimidin-5-yl)furan-2-carboxamide;
5-(1,2,3,4-tetrahydro-4,4,7-trimethyl-4-sila-quinolin-6-yloxy)-*N*-{2-[3-(4-methylpiperazin-1-yl)propylamino]-4,6-dimethoxy-1,3-pyrimidin-5-yl}furan-2-carboxamide;
5-(1,2,3,4-tetrahydro-4,4,7-trimethyl-4-sila-quinolin-6-yloxy)-*N*-{2-[3-(*N,N-*dimethylamino)propylamino]-4,6-dimethoxy-1,3-pyrimidin-5-yl}furan-2-carboxamide;
5-(1,2,3,4-tetrahydro-4,4,7-trimethyl-4-sila-quinolin-6-yloxy)-*N*-{2-[3-(morpholin-4-yl)propylamino]-4,6-dimethoxy-1,3-pyrimidin-5-yl}furan-2-carboxamide;
5-(1,1,3,3,6-pentamethyl-1,3-disila-2,3-dihydro-1*H*-inden-5-yloxy)-*N*-(2,6-dimethoxyphenyl)furan-2-carboxamide;
5-(1,1,3,3,6-pentamethyl-1,3-disila-2,3-dihydro-1*H*-inden-5-yloxy)-*N*-(2,4,6-trimethoxyphenyl)furan-2-carboxamide;
5-(1,1,3,3,6-pentamethyl-1,3-disila-2,3-dihydro-1*H*-inden-5-yloxy)-*N*-(2,4,6-trimethoxy-1,3-pyrimidin-5-yl)furan-2-carboxamide;
5-(1,1,3,3,6-pentamethyl-1,3-disila-2,3-dihydro-1*H*-inden-5-yloxy)-*N*-(2-methylamino-4,6-dimethoxy-1,3-pyrimidin-5-yl)furan-2-carboxamide;
5-(1,1,3,3,6-pentamethyl-1,3-disila-2,3-dihydro-1*H*-inden-5-yloxy)-*N*-{2-[3-(4-methylpiperazin-1-yl)propylamino]-4,6-dimethoxy-1,3-pyrimidin-5-yl}furan-2-carboxamide;
5-(1,1,3,3,6-pentamethyl-1,3-disila-2,3-dihydro-1*H*-inden-5-yloxy)-*N*-{2-[3-(*N,N*-dimethylamino)propylamino]-4,6-dimethoxy-1,3-pyrimidin-5-yl}furan-2-carboxamide;
5-(1,1,3,3,6-pentamethyl-1,3-disila-2,3-dihydro-1*H*-inden-5-yloxy)-*N*-{2-[3-(morpholin-4-yl)propylamino]-4,6-dimethoxy-1,3-pyrimidin-5-yl}furan-2-carboxamide;
5-(1,1,5-trimethyl-1-sila-2,3-dihydro-1*H*-inden-6-yloxy)-*N*-(2,6-dimethoxyphenyl)furan-2-carboxamide;
5-(1,1,5-trimethyl-1-sila-2,3-dihydro-1*H*-inden-6-yloxy)-*N*-(2,4,6-trimethoxyphenyl)furan-2-carboxamide;
5-(1,1,5-trimethyl-1-sila-2,3-dihydro-1*H*-inden-6-yloxy)-*N*-(2,4,6-trimethoxy-1,3-pyrimidin-5-yl)furan-2-carboxamide;
5-(1,1,5-trimethyl-1-sila-2,3-dihydro-1*H*-inden-6-yloxy)-*N*-(2-methylamino-4,6-dimethoxy-1,3-pyrimidin-5-yl)furan-2-carboxamide;
5-(1,1,5-trimethyl-1-sila-2,3-dihydro-1*H*-inden-6-yloxy)-*N*-{2-[3-(4-methylpiperazin-1-yl)propylamino]-4,6-dimethoxy-1,3-pyrimidin-5-yl}furan-2-carboxamide;
5-(1,1,5-trimethyl-1-sila-2,3-dihydro-1*H*-inden-6-yloxy)-*N*-{2-[3-(*N,N-*dimethylamino)propylamino]-4,6-dimethoxy-1,3-pyrimidin-5-yl}furan-2-carboxamide;
5-(1,1,5-trimethyl-1-sila-2,3-dihydro-1*H*-inden-6-yloxy)-*N*-{2-[3-(morpholin-4-yl)propylamino]-4,6-dimethoxy-1,3-pyrimidin-5-yl}furan-2-carboxamide;
5-(1,1,5-trimethyl-1-sila-2,3-dihydro-1*H*-inden-6-yloxy)-*N*-{2-[(pyridin-2-yl)methylamino]-4,6-dimethoxy-1,3-pyrimidin-5-yl}furan-2-carboxamide;
5-(1,1,6-trimethyl-1-sila-2,3-dihydro-1*H*-inden-5-yloxy)-*N*-(2,6-dimethoxyphenyl)furan-2-carboxamide;
5-(1,1,6-trimethyl-1-sila-2,3-dihydro-1*H*-inden-5-yloxy)-*N*-(2,4,6-trimethoxyphenyl)furan-2-carboxamide;
5-(1,1,6-trimethyl-1-sila2,3-dihydro-1*H*-inden-5-yloxy)-*N*-(2,4,6-trimethoxy-1,3-pyrimidin-5-yl)furan-2-carboxamide;
5-(1,1,6-trimethyl-1-sila-2,3-dihydro-1*H*-inden-5-yloxy)-*N*-(2-methylamino-4,6-dimethoxy-1,3-pyrimidin-5-yl)furan-2-carboxamide;
5-(1,1,6-trimethyl-1-sila-2,3-dihydro-1*H*-inden-5-yloxy)-*N*-{2-[3-(4-methylpiperazin-1-yl)propylamino]-4,6-dimethoxy-1,3-pyrimidin-5-yl}furan-2-carboxamide;
5-(1,1,6-trimethyl-1-sila-2,3-dihydro-1*H*-inden-5-yloxy)-*N*-{2-[3-(*N,N-*dimethylamino)propylamino]-4,6-dimethoxy-1,3-pyrimidin-5-yl}furan-2-carboxamide;
5-(1,1,6-trimethyl-1-sila-2,3-dihydro-1*H*-inden-5-yloxy)-*N*-{2-[3-(morpholin-4-yl)propylamino]-4,6-dimethoxy-1,3-pyrimidin-5-yl}furan-2-carboxamide;
5-(1,1,5-trimethyl-1-sila-1*H*-inden-6-yloxy)-*N*-(2,6-dimethoxyphenyl)furan-2-carboxamide;
5-(1,1,5-trimethyl-1-sila-1*H*-inden-6-yloxy)-*N*-(2,4,6-trimethoxyphenyl)furan-2-carboxamide;
5-(1,1,5-trimethyl-1-sila-1*H*-inden-6-yloxy)-*N*-(2,4,6-trimethoxy-1,3-pyrimidin-5-yl)furan-2-carboxamide;
5-(1,1,5-trimethyl-1-sila-1*H*-inden-6-yloxy)-*N*-(2-methylamino-4,6-dimethoxy-1,3-pyrimidin-5-yl)furan-2-carboxamide;
5-(1,1,5-trimethyl-1-sila-1*H*-inden-6-yloxy)-*N*-{2-[3-(4-methylpiperazin-1-yl)propylamino]-4,6-dimethoxy-1,3-pyrimidin-5-yl}furan-2-carboxamide;
5-(1,1,5-trimethyl-1-sila-1*H*-inden-6-yloxy)-*N*-{2-[3-(*N,N-*dimethylamino)propylamino]-4,6-dimethoxy-1,3-pyrimidin-5-yl}furan-2-carboxamide;
5-(1,1,5-trimethyl-1-sila-1*H*-inden-6-yloxy)-*N*-{2-[3-(morpholin-4-yl)propylamino]-4,6-dimethoxy-1,3-pyrimidin-5-yl}furan-2-carboxamide;
5-(5-methoxy-1,1-dimethyl-1-sila-1*H*-inden-6-yloxy)-*N*-(2,6-dimethoxyphenyl)furan-2-carboxamide;
5-(5-methoxy-1,1-dimethyl-1-sila-1*H*-inden-6-yloxy)-*N*-(2,4,6-trimethoxyphenyl)furan-2-carboxamide;
5-(5-methoxy-1,1-dimethyl-1-sila-1*H*-inden-6-yloxy)-*N*-(2,4,6-trimethoxy-1,3-pyrimidin-5-yl)furan-2-carboxamide;
5-(5-methoxy-1,1-dimethyl-1-sila-1*H*-inden-6-yloxy)-*N*-(2-methylamino-4,6-dimethoxy-1,3-pyrimidin-5-yl)furan-2-carboxamide;
5-(5-methoxy-1,1-dimethyl-1-sila-1*H*-inden-6-yloxy)-*N*-{2-[3-(4-methylpiperazin-1-yl)propylamino]-4,6-dimethoxy-1,3-pyrimidin-5-yl}furan-2-carboxamide;
5-(5-methoxy-1,1-dimethyl-1-sila-1*H*-inden-6-yloxy)-*N*-{2-[3-(*N,N-*dimethylamino)propylamino]-4,6-dimethoxy-1,3-pyrimidin-5-yl}furan-2-carboxamide;
5-(5-methoxy-1,1-dimethyl-1-sila-1*H*-inden-6-yloxy)-*N*-{2-[3-(morpholin-4-yl)propylamino]-4,6-dimethoxy-1,3-pyrimidin-5-yl}furan-2-carboxamide;
5-[1,1,6-trimethyl-3-(2-propyl)-1-sila-2,3-dihydro-1*H*-inden-5-yloxy]-*N*-(2,6-dimethoxyphenyl)furan-2-carboxamide;
5-[1,1,6-trimethyl-3-(2-propyl)-1-sila-2,3-dihydro-1*H*-inden-5-yloxy]-*N-*(2,4,6-trimethoxyphenyl)furan-2-carboxamide;
5-[1,1,6-trimethyl-3-(2-propyl)-1-sila-2,3-dihydro-1*H*-inden-5-yloxy]-*N-*(2,4,6-trimethoxy-1,3-pyrimidin-5-yl)furan-2-carboxamide;
5-[1,1,6-trimethyl-3-(2-propyl)-1-sila-2,3-dihydro-1*H*-inden-5-yloxy]-*N*-(2-methylamino-4,6-dimethoxy-1,3-pyrimidin-5-yl)furan-2-carboxamide;
5-[1,1,6-trimethyl-3-(2-propyl)-1-sila-2,3-dihydro-1*H*-inden-5-yloxy]-*N*-{2-[3-(4-methylpiperazin-1-yl)propylamino]-4,6-dimethoxy-1,3-pyrimidin-5-yl}furan-2-carboxamide;
5-[1,1,6-trimethyl-3-(2-propyl)-1-sila-2,3-dihydro-1*H*-inden-5-yloxy]-*N*-{2-[3-(*N,N*-dimethylamino)propylamino]-4,6-dimethoxy-1,3-pyrimidin-5-yl}furan-2-carboxamide;
5-[1,1,6-trimethyl-3-(2-propyl)-1-sila-2,3-dihydro-1*H*-inden-5-yloxy]-*N*-{2-[3-(morpholin-4-yl)propylamino]-4,6-dimethoxy-1,3-pyrimidin-5-yl}furan-2-carboxamide;
5-(5-chloro-1,1-dimethyl-1-sila-2,3-dihydro-1*H*-inden-6-yloxy)-*N*-(2,6-dimethoxyphenyl)furan-2-carboxamide;
5-(5-chloro-1,1-dimethyl-1-sila-2,3-dihydro-1*H*-inden-6-yloxy)-*N*-(2,4,6-trimethoxyphenyl)furan-2-carboxamide;
5-(5-chloro-1,1-dimethyl-1-sila-2,3-dihydro-1*H*-inden-6-yloxy)-*N*-(2,4,6-trimethoxy-1,3-pyrimidin-5-yl)furan-2-carboxamide;
5-(5-chloro-1,1-dimethyl-1-sila-2,3-dihydro-1*H*-inden-6-yloxy)-*N*-(2-methylamino-4,6-dimethoxy-1,3-pyrimidin-5-yl)furan-2-carboxamide;
5-(5-chloro-1,1-dimethyl-1-sila-2,3-dihydro-1*H*-inden-6-yloxy)-*N*-{2-[3-(4-methylpiperazin-1-yl)propylamino]-4,6-dimethoxy-1,3-pyrimidin-5-yl}furan-2-carboxamide;
5-(5-chloro-1,1-dimethyl-1-sila-2,3-dihydro-1*H*-inden-6-yloxy)-*N*-{2-[3-(*N,N-*dimethylamino)propylamino]-4,6-dimethoxy-1,3-pyrimidin-5-yl}furan-2-carboxamide;
5-(5-chloro-1,1-dimethyl-1-sila-2,3-dihydro-1*H*-inden-6-yloxy)-*N*-{2-[3-(morpholin-4-yl)propylamino]-4,6-dimethoxy-1,3-pyrimidin-5-yl}furan-2-carboxamide;
5-(5-chloro-1,1,7-trimethyl-1-sila-2,3-dihydro-1*H*-inden-4-yloxy)-*N*-(2,6-dimethoxyphenyl)furan-2-carboxamide;
5-(5-chloro-1,1,7-trimethyl-1-sila-2,3-dihydro-1*H*-inden-4-yloxy)-*N*-(2,4,6-trimethoxyphenyl)furan-2-carboxamide;
5-(5-chtoro-1,1,7-trimethyl-1-sila-2,3-dihydro-1*H*-inden-4-yloxy)-*N*-(2,4,6-trimethoxy-1,3-pyrimidin-5-yl)furan-2-carboxamide;
5-(5-chloro-1,1,7-trimethyl-1-sila-2,3-dihydro-1*H*-inden-4-yloxy)-*N*-(2-methylamino-4,6-dimethoxy-1,3-pyrimidin-5-yl)furan-2-carboxamide;
5-(5-chloro-1,1,7-trimethyl-1-sila-2,3-dihydro-1*H*-inden-4-yloxy)-*N*-{2-[3-(4-methylpiperazin-1-yl)propylamino]-4,6-dimethoxy-1,3-pyrimidin-5-yl}furan-2-carboxamide;
5-(5-chloro-1,1,7-trimethyl-1-sila-2,3-dihydro-1*H*-inden-4-yloxy)-*N*-{2-[3-(*N,N*-dimethylamino)propylamino]-4,6-dimethoxy-1,3-pyrimidin-5-yl}furan-2-carboxamide;
5-(5-chloro-1,1,7-trimethyl-1-sila-2,3-dihydro-1*H*-inden-4-yloxy)-*N*-{2-[3-(morpholin-4-yl)propylamino]-4,6-dimethoxy-1,3-pyrimidin-5-yl}furan-2-carboxamide;
5-(5-methoxy-1,1-dimethyl-1-sila-2,3-dihydro-1*H*-inden-6-yloxy)-*N*-(2,6-dimethoxyphenyl)furan-2-carboxamide;
5-(5-methoxy-1,1-dimethyl-1-sila-2,3-dihydro-1*H*-inden-6-yloxy)-*N*-(2,4,6-trimethoxyphenyl)furan-2-carboxamide;
5-(5-methoxy-1,1-dimethyl-1-sila-2,3-dihydro-1*H*-inden-6-yloxy)-*N*-(2,4,6-trimethoxy-1,3-pyrimidin-5-yl)furan-2-carboxamide;
5-(5-methoxy-1,1-dimethyl-1-sila-2,3-dihydro-1*H*-inden-6-yloxy)-*N*-(2-methylamino-4,6-dimethoxy-1,3-pyrimidin-5-yl)furan-2-carboxamide;
5-(5-methoxy-1,1-dimethyl-1-sila-2,3-dihydro-1*H*-inden-6-yloxy)-*N*-{2-[3-(4-methylpiperazin-1-yl)propylamino]-4,6-dimethoxy-1,3-pyrimidin-5-yl}furan-2-carboxamide;
5-(5-methoxy-1,1-dimethyl-1-sila-2,3-dihydro-1*H*-inden-6-yloxy)-*N*-{2-[3-(*N,N*-dimethylamino)propylamino]-4,6-dimethoxy-1,3-pyrimidin-5-yl}furan-2-carboxamide;
5-(5-methoxy-1,1-dimethyl-1-sila-2,3-dihydro-1*H*-inden-6-yloxy)-*N*-{2-[3-(morpholin-4-yl)propylamino]-4,6-dimethoxy-1,3-pyrimidin-5-yl}furan-2-carboxamide;
5-(5-methoxy-1,1-dimethyl-1-sila-2,3-dihydro-1*H*-inden-6-yloxy)-*N*-{2-[(pyridin-2-yl)methylamino]-4,6-dimethoxy-1,3-pyrimidin-5-yl}furan-2-carboxamide;
5-[(1,1,4,4,7-pentamethyl-1,4-disila-1,2,3,4-tetrahydronaphthalen-6-yl)methyl]-*N*-{2-[3-(4-methylpiperazin-1-yl)propylamino]-4,6-dimethoxy-1,3-pyrimidin-5-yl}furan-2-carboxamide;
5-[(1,1,4,4,7-pentamethyl-1,4-disila-1,2,3,4-tetrahydronaphthalen-6-yl)methyl]-*N*-{2-[3-(*N,N*-dimethylamino)propylamino]-4,6-dimethoxy-1,3-pyrimidin-5-yl}furan-2-carboxamide;
5-[(1,1,4,4,7-pentamethyl-1,4-disila-1,2,3,4-tetrahydronaphthalen-6-yl)methyl]-*N*-{2-[3-(morpholin-4-yl)propylamino]-4,6-dimethoxy-1,3-pyrimidin-5-yl}furan-2-carboxamide;
5-[(1,1,3,3,6-pentamethyl-1,3-disila-2,3-dihydro-1*H*-inden-5-yl)methyl]-*N-*(2,6-dimethoxyphenyl)furan-2-carboxamide;
5-[(1,1,3,3,6-pentamethyl-1,3-disila-2,3-dihydro-1*H*-inden-5-yl)methyl]-*N-*(2,4,6-trimethoxyphenyl)furan-2-carboxamide;
5-[(1,1,3,3,6-pentamethyl-1,3-disila-2,3-dihydro-1*H*-inden-5-yl)methyl]-*N-*(2,4,6-trimethoxy-1,3-pyrimidin-5-yl)furan-2-carboxamide;
5-[(1,1,3,3,6-pentamethyl-1,3-disila-2,3-dihydro-1*H*-inden-5-yl)methyl]-*N-*(2-methylamino-4,6-dimethoxy-1,3-pyrimidin-5-yl)furan-2-carboxamide;
5-[(1,1,3,3,6-pentamethyl-1,3-disila-2,3-dihydro-1*H*-inden-5-yl)methyl]-*N-*{2-[3-(4-methylpiperazin-1-yl)propylamina]-4,6-dimethoxy-1,3-pyrimidin-5-yl}furan-2-carboxamide;
5-[(1,1,3,3,6-pentamethyl-1,3-disila-2,3-dihydro-1*H*-inden-5-yl)methyl]-*N-*{2-[3-(*N,N*-dimethylamino)propylamino]-4,6-dimethoxy-1,3-pyrimidin-5-yl}furan-2-carboxamide;
5-[(1,1,3,3,6-pentamethyl-1,3-disila-2,3-dihydro-1*H*-inden-5-yl)methyl]-*N-*{2-[3-(morpholin-4-yl)propylamino]-4,6-dimethoxy-1,3-pyrimidin-5-yl}furan-2-carboxamide;
5-[(1,1,5-trimethyl-1-sila-2,3-dihydro-1*H*-inden-6-yl)methyl]-*N*-(2,6-dimethoxyphenyl)furan-2-carboxamide;
5-[(1,1,5-trimethyl-1-sila-2,3-dihydro-1*H*-inden-6-yl)methyl]-*N*-(2,4,6-trimethoxyphenyl)furan-2-carboxamide;
5-[(1,1,5-trimethyl-1-sila-2,3-dihydro-1*H*-inden-6-yl)methyl]-*N*-(2,4,6-trimethoxy-1,3-pyrimidin-5-yl)furan-2-carboxamide;
5-[(1,1,5-trimethyl-1-sila-2,3-dihydro-1*H*-inden-6-yl)methyl]-*N*-(2-methylamino-4,6-dimethoxy-1,3-pyrimidin-5-yl)furan-2-carboxamide;
5-[(1,1,5-trimethyl-1-sila-2,3-dihydro-1*H*-inden-6-yl)methyl]-*N*-{2-[3-(4-methylpiperazin-1-yl)propylamino]-4,6-dimethoxy-1,3-pyrimidin-5-yl}furan-2-carboxamide;
5-[(1,1,5-trimethyl-1-sila-2,3-dihydro-1*H*-inden-6-yl)methyl]-*N*-{2-[3-(*N,N-*dimethylamino)propylamino]-4,6-dimethoxy-1,3-pyrimidin-5-yl}furan-2-carboxamide;
5-[(1,1,5-trimethyl-1-sila-2,3-dihydro-1*H*-inden-6-yl)methyl]-*N*-[2-(3-morpholinopropylamino)-4,6-dimethoxy-1,3-pyrimidin-5-yl]furan-2-carboxamide;
5-[(1,1,5-trimethyl-1-sila-2,3-dihydro-1*H*-inden-6-yl)methyl]-*N*-{2-[(pyridin-2-yl)methylamino]-4,6-dimethoxy-1,3-pyrimidin-5-yl}furan-2-carboxamide;
5-[(1,1,5-trimethyl-1-sila-2,3-dihydroinden-6-yl)methyl]-*N*-(4-chloro-2,6-dimethoxyphenyl)furan-2-carboxamide;
5-(1,1,3,4,4,6-hexamethyl-1-sila-1,2,3,4-tetrahydronaphthalen-7-yloxy)-*N-*{[2-(2-(morpholin-4-yl)-ethylamino)-4,6-dimethoxy-1,3-pyrimidin-5-yl]furan-2-carboxamide;
5-(1,1,4,4,6-pentamethyl-1,4-disila-1,2,3,4-tetrahydronaphthalen-5-oxy)-*N-*(2,4,6-trimethoxyphenyl)furan-2-carboxamide;
5-(7'-hydroxy-1,1,1',1',6,6'-hexamethyl-3,3'-spiro-1,1'-disila-4,4'-dioxo-1,1',2,2',3,3',4,4'-octahydrodinaphthalen-7-yloxy)-*N*-(2,6-dimethoxyphenyl)furan-2-carboxamide;
5-[1,1,2,6-tetramethyl-3-(2-propy)-1-sila-2,3-dihydroinden-5-yloxy]-*N-*(2,4,6-trimethoxyphenyl)furan-2-carboxamide;
5-(1,1,3,4,4-pentamethyt-1-sita-1,2,3,4-tetrahydronaphthalen-7-yloxy)-*N-*(2,4,6-trimethoxy-1,3-pyrimidin-5-yl)furan-2-carboxamide.
5-(1,1,5-trimethyl-1-sila-2,3-dihydroinden-6-yloxy)-*N*-(4-chloro-2,6-dimethoxyphenyl)furan-2-carboxamide;
5-(1,1,3,3,5-pentamethyl-1-sila-2,3-dihydroinden-6-yloxy)-*N*-(2,6-dimethoxyphenyl)furan-2-carboxamide;
5-(1,1,3,3,6-pentamethyl-1-sila-2,3-dihydroinden-5-yloxy)-*N*-(2,6-dimethoxyphenyl)furan-2-carboxamide;
5-(8-methoxy-1,1,4,4,6-pentamethyl-1-sila-1,2,3,4-tetrahydronaphthalen-7-yloxy)-*N*-{2-[3-(morpholin-4-yl)-*N*-propylamino]-4,6-dimethoxy-1,3-pyrimidin-5-yl}furan-2-carboxamide;
5-(1,1,6-trimethyl-1-sila-1,2,3,4-tetrahydronaphthalen-7-yloxy)-*N*-(2,4-dimethoxypyridin-3-yl)furan-2-carboxamide.
5-(1,1,3,4,4,6-hexamethyl-1-sila-1,2,3,4-tetrahydronaphthalen-7-yloxy)-*N*-(2,4,6-trimethoxy-1,3-pyrimidin-5-yl)furan-2-carboxamide and
5-(1,1,3,4,4,6-hexamethyl-1-sila-1,2,3,4-tetrahydronaphthalen-7-yloxy)-*N*-[2-(2-*N,N*-dimethylaminoethyl)-4,6-trimethoxy-1,3-pyrimidin-5-yl]furan-2-carboxamide;

21. A compound according to any preceding claim, which is in the form of a single enantiomer or diastereomer or tautomer.

22. A compound according to any preceding claim, for therapeutic use.

23. A pharmaceutical composition comprising a compound of any of claims 1 to 21 and a pharmaceutically acceptable diluent or carrier.

24. Use of a compound of any of claims 1 to 21, for the manufacture of a medicament for cancer therapy.

25. Use of a compound of any of claims 1 to 21, for the manufacture of a medicament for the treatment or prevention of endometriosis, uterine myoma, an ovarian disease, a mammary cystic disease, prostatic hypertrophy, amenorrhea, precocious puberty, premenstrual syndrome, a sex-steroid-dependent pathophysiology or benign prostatic hyperplasia, or to arrest spermatogenesis.

26. Use according to claim 25, for the treatment or prevention of endometriosis with pain, polycystic ovarian disease or secondary amenorrhea.

27. Use of a compound of any of claims 1 to 21, for the manufacture of a medicament for the treatment or prevention of Alzheimer's disease.

28. Use of a compound of any of claims 1 to 21, for the manufacture of a medicament for the treatment or prevention of HIV infection or AIDS.

29. Use of a compound of any of claims 1 to 21, for the manufacture of a medicament for the treatment or prevention of a disease caused by thymic malfunction.

30. Use according to claim 29, for the treatment or prevention of multiple sclerosis, rheumatoid arthritis or type 1 diabetes.

## Patentansprüche

1. Verbindung der Formel (I) oder der Formel (II) 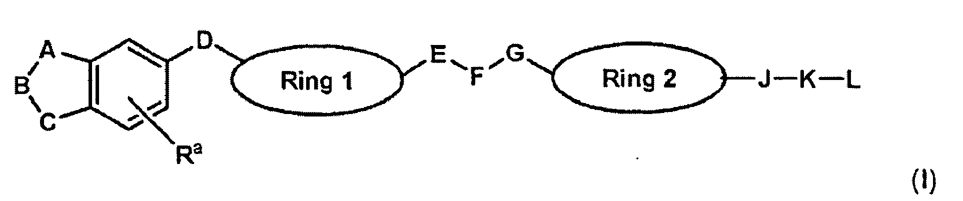 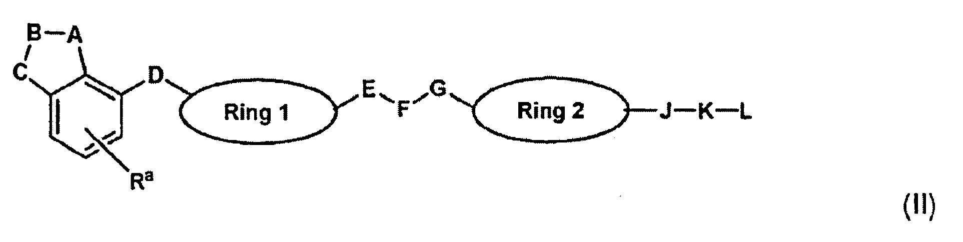 wobei
A und C gleich oder verschieden sind und jeweils eine Bindung, -(CH₂)ₙ-, -C(R^{b})₂-, -Si(R^{c})₂-, -O-, -S(O)ₘ-, -N=, -N(R^{b})-, -N(R^{e})C(=X)- oder -C(=X)- sind;
B -(CH₂)ₙ-, -O-, -C(R^{b})₂-, -Si(R^{c})₂-, -C(R^{b})=C(R^{b})-, -C(R^{b})=, -(CH₂)ₙC(R^{g})₂-, -C(R^{g})₂(CH₂)ₙ- oder -CH(R^{b})CH(R^{b})- ist;
wobei irgendeines von A, B und C wahlweise mit -Si(R^{c})₃ substituiert ist;
D -(CH₂)ₙ-, -C(=X)-, -O-, -S(O)ₘ-, -C(=X)N(R^{e})-, -C(R^{b})₂-, -C(R^{b})=C(R^{b})-, -CH(R^{b})CH(R^{b})- ist;
E wahlweise anwesend ist und -(CH₂)ₙ-, -N(R^{d})-, -(CH₂)ₙN(R^{d})- oder -N(R^{d})(CH₂)ₙ- ist;
F -C(=X)- oder -N(R^{d})- ist;
G -(CH₂)ₙ-, -N(R^{d})-, -(CH₂)ₙN(R^{d})- oder -N(R^{d})(CH₂) ist;
J wahlweise anwesend ist und -O-, -N(R^{c})C(=X)-, -C(=X)N(R^{c})-, -S(O)ₘ-, -N(R^{c})S(O)ₘ-, -S(O)ₘN(R^{c})- oder -N(R^{e})- ist;
K wahlweise anwesend ist und Alkylen ist, das wahlweise mit R^{b} substituiert ist; oder K Cycloalkylen, Cycloalkenylen, Arylen, Heterocycloalkylen, Heterocycloalkenylen oder Heteroarylene ist, von denen irgendeines wahlweise mit R^{a} substituiert ist;
L Wasserstoff, Halogen, -N(R^{f})₂, Cycloalkyl, Cycloalkenyl, Aryl, Heterocycloalkyl, Heterocycloalkenyl oder Heteroaryl ist, von denen irgendeines wahlweise mit R^{a}, -C(=X)OR^{d}, -OH, -OR^{c}, -C(=X)N(R^{b})(R^{c}), -S(O)ₘN(R^{b})(R^{c}) oder -CN substituiert ist;
jedes R^{a} gleich oder verschieden ist und Wasserstoff, Halogen, Alkyl, Aryl, Hydroxy, Alkoxy, -Alkoxy-(CH₂)ₙC(O)₂R^{b}, -O-Aryl, -C(=X)R^{c}, -NO₂, -CN, -N(R^{c})C(=X)R^{c}, -C(=X)N(R^{c})₂, -S(O)₂N(R^{c})₂ oder -N(R^{e})₂ ist;
jedes R^{b} gleich oder verschieden ist und Wasserstoff oder Alkyl ist;
jedes R^{c} gleich oder verschieden ist und Alkyl, Cycloalkyl, -Alkyl-aryl, -Alkyl-cycloalkyl oder Aryl ist, wahlweise substituiert mit R^{a};
jedes R^{d} gleich oder verschieden ist und Wasserstoff, Alkyl oder Aryl ist, wahlweise substituiert mit R^{a};
jedes R^{e} gleich oder verschieden ist und Wasserstoff oder Alkyl ist; oder R^{e} Aryl oder Heteroaryl ist, von denen eines wahlweise mit R^{a} substituiert ist;
jedes R^{f} gleich oder verschieden ist und Wasserstoff oder Alkyl ist; oder R^{f}-N-R^{f} zusammengenommen Heterocycloalkyl, Heterocycloalkenyl oder Heteroaryl bilden;
R^{g}-C-R^{g} zusammengenommen Heterocyclyl bilden;
Jedes X gleich oder verschieden ist und Sauerstoff oder Schwefel ist;
die Ringe 1 und 2 gleich oder verschieden sind und jeweils Arylen oder Heteroarylen sind, von denen eines wahlweise mit R^{a} substituiert ist;
jedes m gleich oder verschieden ist und 0, 1 oder 2 ist;
jedes n gleich oder verschieden ist und 0,1,2 oder 3 ist;
mit der Maßgabe, dass mindestens eines von A, B und C ein Siliziumatom aufweist; A und C nicht beide eine Bindung sind; und die Verbindung keine Si-Si Bindung, keine N-N Einfachbindung, keine Si-O Bindung, keine Si-N Bindung oder keine N-O-N Verknüpfung aufweist;
mit der weiteren Maßgabe, dass die Verbindung nicht die Formel 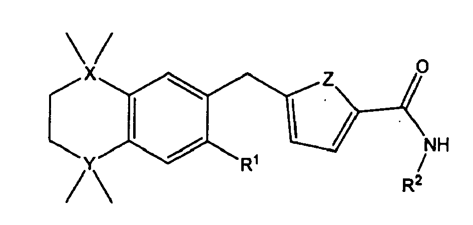 aufweist, wobei
eines von X oder Y Silizium ist und das andere Kohlenstoff oder Silizium ist;
Z Sauerstoff, Schwefel oder -N(R)- ist, wobei R Wasserstoff oder Alkyl ist;
R¹ Wasserstoff, Halogen, Alkyl, Alkenyl, Alkinyl, Alkoxy oder Cycloalkyl ist; und
R² Alkyl, Alkenyl, Alkinyl, Cycloalkyl, Heterocycloalkyl, Aryl, Heteroaryl, -Alkyl-cycloalkyl, -Alkyl-heterocycloalkyl, -Alkyl-aryl oder -Alkyl-heteroaryl ist;
oder ein pharmazeutisch verträgliche Salz davon.

2. Verbindung gemäß Anspruch 1, welche die Formel (I) aufweist.

3. Verbindung gemäß Anspruch 1 oder Anspruch 2, wobei A und/oder C -Si(R^{c})₂- ist/sind.

4. Verbindung gemäß Anspruch 3, wobei jedes R^{c} gleich oder verschieden ist und Alkyl ist.

5. Verbindung gemäß Anspruch 1 oder Anspruch 2, wobei A und/oder C -C(R^{b})₂-, -(CH₂)ₙ-, -N(R^{b})- oder -O- ist.

6. Verbindung gemäß Anspruch 5, wobei jedes R^{b} gleich oder verschieden ist und Wasserstoff oder Alkyl ist.

7. Verbindung gemäß einem der vorhergehenden Ansprüche, wobei B -(CH₂)ₙ-, -C(R^{b})₂-, -CH(R^{b})CH(R^{b})-, -C(R^{b})=C(R^{b})- oder -CH₂-C(R^{g})₂- ist.

8. Verbindung gemäß Anspruch 7, wobei jeder R^{b} gleich oder verschieden ist und Wasserstoff oder Alkyl ist.

9. Verbindung gemäß einem der vorhergehenden Ansprüche, wobei D -O-, -S- oder -CH₂- ist.

10. Verbindung gemäß einem der vorhergehenden Ansprüche, wobei E abwesend ist.

11. Verbindung gemäß einem der vorhergehenden Ansprüche, wobei F -C(O)- ist.

12. Verbindung gemäß einem der vorhergehenden Ansprüche, wobei G -N(R^{d})- ist.

13. Verbindung gemäß Anspruch 12, wobei R^{d} Wasserstoff ist.

14. Verbindung gemäß einem der vorhergehenden Ansprüche, wobei J und K abwesend sind und L Wasserstoff oder -N(R^{f})₂ ist.

15. Verbindung gemäß einem der Ansprüche 1 bis 13, wobei J -NH- ist, K Alkylene ist und L Heterocycloalkyl ist.

16. Verbindung gemäß einem der vorhergehenden Ansprüche, wobei der Ring 1 Heteroarylen ist.

17. Verbindung gemäß Anspruch 16, wobei der Ring 1 Furanylen ist.

18. Verbindung gemäß einem der vorhergehenden Ansprüche, wobei der Ring 2 Phenyl, Pyrimidinyl oder Pyridinyl ist, von denen irgendeiner wahlweise substituiert ist.

19. Verbindung gemäß Anspruch 18, wobei der Ring 2 1-, 2- oder 3-fach substituiert ist, die Substituenten bei jedem Auftreten gleich oder verschieden sind und aus Alkoxy und Halogen ausgewählt sind.

20. Verbindung gemäß Anspruch 1, ausgewählt aus:
5-(1,1,4,4,7-Pentamethyl-1,4-disila-1,2,3,4-tetrahydronaphthalen-6-yloxy)-*N-*(2,6-dimethoxyphenyl)furan-2-carboxamid;
5-(1,1,4,4,7-Pentamethyl-1,4-disila-1,2,3,4-tetrahydronaphthalen-6-yloxy)-*N-*(2,4,6-trimethoxyphenyl)furan-2-carboxamid;
5-(1,1,4,4,7-Pentamethyl-1,4-disila-1,2,3,4-tetrahydronaphthalen-6-yloxy)-*N-*(2,4,6-trimethoxy-1,3-pyrimidin-5-yl)furan-2-carboxamid;
5-(1,1,4,4,7-Pentamethyl-1,4-disila-1,2,3,4-tetrahydronaphthalen-6-yloxy)-*N-*(2-methylamino-4,6-dimethoxy-1,3-pyrimidin-5-yl)furan-2-carboxamid;
5-(1,1,4,4,7-Pentamethyl-1,4-disila-1,2,3,4-tetrahydronaphthalen-6-yloxy)-*N-*{2-[3-(4-methylpiperazin-1-yl)propylamino]-4,6-dimethoxy-1,3-pyrimidin-5-yl}furan-2-carboxamid;
5-(1,1,4,4,7-Pentamethyl-1,4-disila-1,2,3,4-tetrahydronaphthalen-6-yloxy)-*N-*{2-[3-(*N,N*-dimethylamino)propylamino]-4,6-dimethoxy-1,3-pyrimidin-5-yl}furan-2-carboxamid;
5-(1,1,4,4,7-Pentamethyl-1,4-disila-1,2,3,4-tetrahydronaphthalen-6-yloxy)-*N-*{2-[3-(morpholin-4-yl)propylamino]-4,6-dimethoxy-1,3-pyrimidin-5-yl}furan-2-carboxamid;
5-(1,1,6-Trimethyl-1-sila-1,2,3,4-tetrahydronaphthalen-7-yloxy)-*N*-(2,6-dimethoxyphenyl)furan-2-carboxamid;
5-(1,1,6-Trimethyl-1-sila-1,2,3,4-tetrahydronaphthalen-7-yloxy)-*N*-(2,4,6-trimethoxyphenyl)furan-2-carboxamid;
5-(1,1,6-Trimethyl-1-sila-1,2,3,4-tetrahydronaphthalen-7-yloxy)-*N*-(2,4,6-trimethoxy-1,3-pyrimidin-5-yl)furan-2-carboxamid;
5-(1,1,6-Trimethyl-1-sila-1,2,3,4-tetrahydronaphthalen-7-yloxy)-*N*-(2-methylamino-4,6-dimethoxy-1,3-pyrimidin-5-yl)furan-2-carboxamid;
5-(1,1,6-Trimethyl-1-sila-1,2,3,4-tetrahyd ronaphthalen-7-yloxy)-*N*-{2-[3-(4-methylpiperazin-1-yl)propylamino]-4,6-dimethoxy-1,3-pyrimidin-5-yl}furan-2-carboxamid;
5-(1,1,6-Trimethyl-1-sila-1,2,3,4-tetrahydronaphthalen-7-yloxy)-*N*-{2-[3-(*N,N-*dimethylamino)propylamino]-4,6-dimethoxy-1,3-pyrimidin-5-yl}furan-2-carboxamid;
5-(1,1,6-Trimethyl-1-sila-1,2,3,4-tetrahydronaphthalen-7-yloxy)-*N*-{2-[3-(morpholin-4-yl)propylamino]-4,6-dimethoxy-1,3-pyrimidin-5-yl}furan-2-carboxamid;
5-(1,1,7-Trimethyl-1-sila-1,2,3,4-tetrahydronaphthalen-6-yloxy)-*N*-(2,6-dimethoxyphenyl)furan-2-carboxamid;
5-(1,1,7-Trimethyl-1-sila-1,2,3,4-tetrahydronaphthalen-6-yloxy)-*N*-(2,4,6-trimethoxyphenyl)furan-2-carboxamid;
5-(1,1,7-Trimethyl-1-sita-1,2,3,4-tetrahydronaphthalen-6-yloxy)-*N*-(2,4,6-trimethoxy-1,3-pyrimidin-5-yl)furan-2-carboxamid;
5-(1,1,7-Trimethyl-1-sila-1,2,3,4-tetrahydronaphthalen-6-yloxy)-*N*-(2-methylamino-4,6-dimethoxy-1,3-pyrimidin-5-yl)furan-2-carboxamid;
5-(1,1,7-Trimethyl-1-sila-1,2,3,4-tetrahydronaphthalen-6-yloxy)-*N*-{2-[3-(4-methylpiperazin-1-yl)propylamino]-4,6-dimethoxy-1,3-pyrimidin-5-yl}furan-2-carboxamid;
5-(1,1,7-Trimethyl-1-sila-1,2,3,4-tetrahydronaphthalen-6-yloxy)-*N*-{2-[3-(*N,N-*dimethylamino)propylamino]-4,6-dimethoxy-1,3-pyrimidin-5-yl}furan-2-carboxamid;
5-(1,1,7-Trimethyl-1-sila-1,2,3,4-tetrahydronaphthalen-6-yloxy)-*N*-{2-[3-(morpholin-4-yl)propylamino]-4,6-dimethoxy-1,3-pyrimidin-5-yl}furan-2-carboxamid;
5-(1,1,2,4,4,7-Hexamethyl-1,4-disila-1,2,3,4-tetrahydronaphthalen-6-yloxy)-*N-*(2,6-dimethoxyphenyl)furan-2-carboxamid;
5-(1,1,2,4,4,7-Hexamethyl-1,4-disila-1,2,3,4-tetrahydronaphthalen-6-yloxy)-*N-*(2,4,6-trimethoxyphenyl)furan-2-carboxamid;
5-(1,1,2,4,4,7-Hexamethyl-1,4-disila-1,2,3,4-tetrahydronaphthalen-6-yloxy)-*N-*(2,4,6-trimethoxy-1,3-pyrimidin-5-yl)furan-2-carboxamid;
5-(1,1,2,4,4,7-Hexamethyl-1,4-disila-1,2,3,4-tetrahydronaphthalen-6-yloxy)-*N-*(2-methylamino-4,6-dimethoxy-1,3-pyrimidin-5-yl)furan-2-carboxamid;
5-(1,1,2,4,4,7-Hexamethyl-1,4-disila-1,2,3,4-tetrahydronaphthalen-6-yloxy)-*N-*{2-[3-(4-methylpiperazin-1-yl)propylamino]-4,6-dimethoxy-1,3-pyrimidin-5-yl}furan-2-carboxamid;
5-(1,1,2,4,4,7-Hexamethyl-1,4-disila-1,2,3,4-tetrahydronaphthalen-6-yloxy)-*N-*{2-[3-(*N,N*-dimethylamino)propylamino]-4,6-dimethoxy-1,3-pyrimidin-5-yl}furan-2-carboxamid;
5-(1,1,2,4,4,7-Hexamethyl-1,4-disila-1,2,3,4-tetrahydronaphthalen-6-yloxy)-*N-*{2-[3-(morpholin-4-yl)propylamino]-4,6-dimethoxy-1,3-pyrimidin-5-yl}furan-2-carboxamid;
5-(5-Methoxy-1,1,4,4,7-pentamethyl-1,4-d isila-1,2, 3,4-tetrahydronaphthalen-6-yloxy)-*N*-(2,6-dimethoxyphenyl)furan-2-carboxamid;
5-(5-Methoxy-1,1,4,4,7-pentamethyl-1,4-disila-1,2,3,4-tetrahydronaphthalen-6-yloxy)-*N*-(2,4,6-trimethoxyphenyl)furan-2-carboxamid;
5-(5-Methoxy-1,1,4,4,7-pentamethyl-1,4-disila-1,2,3,4-tetrahydronaphthalen-6-yloxy)-*N*-(2,4,6-trimethoxy-1,3-pyrimidin-5-yl)furan-2-carboxamid;
5-(5-Methoxy-1,1,4,4,7-pentamethyl-1,4-disila-1,2,3,4-tetrahydronaphthalen-6-yloxy)-*N*-(2-methylamino-4,6-dimethoxy-1,3-pyrimidin-5-yl)furan-2-carboxamid;
5-(5-Methoxy-1,1,4,4,7-pentamethyl-1,4-disila-1,2,3,4-tetrahydronaphthalen-6-yloxy)-*N*-{2-[3-(4-methylpiperazin-1-yl)propylamino]-4,6-trimethoxy-1,3-pyrimidin-5-yl}furan-2-carboxamid;
5-(5-Methoxy-1,1,4,4,7-pentamethyl-1,4-disila-1,2,3,4-tetrahydronaphthalen-6-yloxy)-*N*-{2-[3-(*N,N*-dimethylamino)propylamino]-4,6-dimethoxy-1,3-pyrimidin-5-yl}furan-2-carboxamid;
5-(5-Methoxy-1,1,4,4,7-pentamethyl-1,4-disila-1,2,3,4-tetrahydronaphthalen-6-yloxy)-*N*-{2-[3-(morpholin-4-yl)propylamino]-4,6-dimethoxy-1,3-pyrimidin-5-yl}furan-2-carboxamid;
5-(8-Methoxy-1,1,6-trimethyl-1-sila-1,2,3,4-tetrahydronaphthalen-7-yloxy)-*N*-(2,6-dimethoxyphenyl)furan-2-carboxamid;
5-(8-Methoxy-1,1,6-trimethyl-1-sila-1,2,3,4-tetrahydronaphthalen-7-yloxy)-*N*-(2,4,6-trimethoxyphenyl)furan-2-carboxamid;
5-(8-Methoxy-1,1,6-trimethyl-1-sila-1,2,3,4-tetrahydronaphthalen-7-yloxy)-*N*-(2,4,6-trimethoxy-1,3-pyrimidin-5-yl)furan-2-carboxamid;
5-(8-Methoxy-1,1,6-trimethyl-1-sila-1,2,3,4-tetrahydronaphthalen-7-yloxy)-*N*-(2-methylamino-4,6-dimethoxy-1,3-pyrimidin-5-yl)furan-2-carboxamid;
5-(8-Methoxy-1,1,6-trimethyl-1-sila-1,2,3,4-tetrahydronaphthalen-7-yloxy)-*N*-{2-[3-(4-methylpiperazin-1-yl)propylamino]-4,6-dimethoxy-1,3-pyrimidin-5-yl}furan-2-carboxamid;
5-(8-Methoxy-1,1,6-trimethyl-1-sila-1,2,3,4-tetrahydronaphthalen-7-yloxy)-*N*-{2-[3-(*N,N*-dimethylamino)propylamino]-4,6-dimethoxy-1,3-pyrimidin-5-yl}furan-2-carboxamid;
5-(8-Methoxy-1,1,6-trimethyl-1-sila-1,2,3,4-tetrahydronaphthalen-7-yloxy)-*N*-{2-[3-(morpholin-4-yl)propylamino]-4,6-dimethoxy-1,3-pyrimidin-5-yl}furan-2-carboxamid;
5-(1,1,4,4,5,7-Hexamethyl-1,4-disila-1,2,3,4-tetrahydronaphthalen-6-yloxy)-*N-*(2,6-dimethoxyphenyl)furan-2-carboxamid;
5-(1,1,4,4,5,7-Hexamethyl-1,4-disila-1,2,3,4-tetrahydronaphthalen-6-yloxy)-*N-*(2,4,6-trimethoxyphenyl)furan-2-carboxamid;
5-(1,1,4,4,5,7-Hexamethyl-1,4-disila-1,2,3,4-tetrahydronaphthalen-6-yloxy)-*N-*(2,4,6-trimethoxy-1,3-pyrimidin-5-yl)furan-2-carboxamid;
5-(1,1,4,4,5,7-Hexamethyl-1,4-disila-1,2,3,4-tetrahydronaphthalen-6-yloxy)-*N-*(2-methylamino-4,6-dimethoxy-1,3-pyrimidin-5-yl)furan-2-carboxamid;
5-(1,1,4,4,5,7-Hexamethyl-1,4-disila-1,2,3,4-tetrahydronaphthalen-6-yloxy)-*N-*{2-[3-(4-methylpiperazin-1-yl)propylamino]-4,6-dimethoxy-1,3-pyrimidin-5-yl}furan-2-carboxamid;
5-(1,1,4,4,5,7-Hexamethyl-1,4-disila-1,2, 3,4-tetrahydronaphthalen-6-yloxy)-*N-*{2-[3-(*N,N*-dimethylamino)propylamino]-4,6-dimethoxy-1,3-pyrimidin-5-yl}furan-2-carboxamid;
5-(1,1,4,4,5,7-Hexamethyl-1,4-disila-1,2,3,4-tetrahydronaphthalen-6-yloxy)-*N-*2-{3-(morpholin-4-yl)propylamino]-4,6-dimethoxy-1,3-pyrimidin-5-yl}furan-2-carboxamid;
5-(6-Methoxy-1,1,4,4,8-pentamethyl-1,4-disila-1,2,3,4-tetrahydronaphthalen-7-yloxy)-*N*-(2,6-d imethoxyphenyl)furan-2-carboxamid;
5-(6-Methoxy-1,1,4,4,8-pentamethyl-1,4-disila-1,2,3,4-tetrahydronaphthalen-7-yloxy)-*N*-(2,4,6-trimethoxyphenyl)furan-2-carboxamid;
5-(6-Methoxy-1,1,4,4,8-pentamethyl-1,4-disila-1,2,3,4-tetrahydronaphthalen-7-yloxy)-*N*-(2,4,6-trimethoxy-1,3-pyrimidin-5-yl)furan-2-carboxamid;
5-(6-Methoxy-1,1,4,4,8-pentamethyl-1,4-disila-1,2,3,4-tetrahydronaphthalen-7-yloxy)-*N*-(2-methylamino-4,6-dimethoxy-1,3-pyrimidin-5-yl)furan-2-carboxamid;
5-(6-Methoxy-1,1,4,4,8-pentamethyl-1,4-disila-1,2,3,4-tetrahydronaphthalen-7-yloxy)-*N*-{2-[3-(4-methylpiperazin-1-yl)propylamino]-4,6-dimethoxy-1,3-pyrimidin-5-yl}furan-2-carboxamid;
5-(6-Methoxy-1,1,4,4,8-pentamethyl-1,4-disila-1,2,3,4-tetrahydronaphthalen-7-yloxy)-*N*-{2-[3-(*N,N*-dimethylamino)propylamino]-4,6-dimethoxy-1,3-pyrimidin-5-yl}furan-2-carboxamid;
5-(6-Methoxy-1,1,4,4,8-pentamethyl-1,4-disila-1,2,3,4-tetrahydronaphthalen-7-yloxy)-*N*-{2-[3-(morpholin-4-yl)propylamino]-4,6-dimethoxy-1,3-pyrimidin-5-yl}furan-2-carboxamid;
5-(6-Chlor-1,1,4,4-tetramethyl-1,4-disila-1,2,3,4-tetrahyd ronaphthalen-7-yloxy)-*N*-(2,6-dimethoxyphenyl)furan-2-carboxamid;
5-(6-Chlor-1,1,4,4-tetramethyl-1,4-disila-1,2,3,4-tetrahydronaphthalen-7-yloxy)-*N*-(2,4,6-trimethoxyphenyl)furan-2-carboxamid;
5-(6-Chlor-1,1,4,4-tetramethyl-1,4-disila-1,2,3,4-tetrahydronaphthalen-7-yloxy)-*N*-(2,4,6-trimethoxy-1,3-pyrimidin-5-yl)furan-2-carboxamid;
5-(6-Chlor-1,1,4,4-tetramethyl-1,4-disila-1,2,3,4-tetrahydronaphthalen-7-yloxy)-*N*-(2-methylamino-4,6-dimethoxy-1,3-pyrimidin-5-yl)furan-2-carboxamid;
5-(6-Chlor-1,1,4,4-tetramethyl-1,4-disila-1,2,3,4-tetrahydronaphthalen-7-yloxy)-*N*-{2-[3-(4-methylpiperazin-1-yl)propylamino]-4,6-dimethoxy-1,3-pyrimidin-5-yl}furan-2-carboxamid;
5-(6-Chlor-1,1,4,4-tetramethyl-1,4-disila-1,2,3,4-tetrahydronaphthalen-7-yloxy)-*N*-{2-[3-(*N,N*-dimethylamino)propylamino]-4,6-dimethoxy-1,3-pyrimidin-5-yl}furan-2-carboxamid;
5-(6-Chlor-1,1,4,4-tetramethyl-1,4-disila-1,2,3,4-tetrahydronaphthaten-7-yloxy)-*N*-{2-(3-morpholin-4-yl)propylamino]-4,6-dimethoxy-1,3-pyrimidin-5-yl}furan-2-carboxamid;
5-(6-Chlor-1,1-dimethyl-1-sila-1,2, 3,4-tetrahydronaphthalen-7-yloxy)-*N*-(2,6-dimethoxyphenyl)furan-2-carboxamid;
5-(6-Chlor-1,1-dimethyl-1-sila-1,2,3,4-tetrahydronaphthalen-7-yloxy)-*N*-(2,4,6-trimethoxyphenyl)furan-2-carboxamid;
5-(6-Chlor-1,1-dimethyl-1-sila-1,2,3,4-tetrahydronaphthalen-7-yloxy)-*N*-(2,4,6-trimethoxy-1,3-pyrimidin-5-yl)furan-2-carboxamid;
5-(6-Chlor-1,1-d imethyl-1-sila-1,2,3,4-tetrahydronaphthalen-7-yloxy)-*N*-(2-methylamino-4,6-dimethoxy-1,3-pyrimidin-5-yl)furan-2-carboxamid;
5-(6-Chlor-1,1-dimethyl-1-sila-1,2,3,4-tetrahydronaphthalen-7-yloxy)-*N*-{2-[3-(4-methylpiperazin-1-yl)propylamino]-4,6-dimethoxy-1,3-pyrimidin-5-yl}furan-2-carboxamid;
5-(6-Chlor-1,-dimethyl-1-sila-1,2,3,4-tetrahydronaphthalen-7-yloxy)-*N*-{2-[3-(*N,N*-dimethylamino)propylamino]-4,6-dimethoxy-1,3-pyrimidin-5-yl}furan-2-carboxamid;
5-(6-Chlor-1,1-dimethyl-1-sila-1,2,3,4-tetrahydronaphthalen-7-yloxy)-*N*-{2-[3-(morpholin-4-yl)propylamino]-4,6-dimethoxy-1,3-pyrimidin-5-yl}furan-2-carboxamid;
5-(4,4,7-Trimethyl-4-sila-chroman-6-yloxy)-*N*-(2,6-dimethoxyphenyl)furan-2-carboxamid;
5-(4,4,7-Trimethyl-4-sila-chroman-6-yloxy)-*N*-(2,4,6-trimethoxyphenyl)furan-2-carboxamid;
5-(4,4,7-Trimethyl-4-sila-chroman-6-yloxy)-*N*-(2,4,6-trimethoxy-1,3-pyrimidin-5-yl)furan-2-carboxamid;
5-(4,4,7-Trimethyl-4-sila-chroman-6-yloxy)-*N*-(2-methylamino-4,6-dimethoxy-1,3-pyrimidin-5-yl)furan-2-carboxamid;
5-(4,4,7-Trimethyl-4-sila-chroman-6-yloxy)-*N*-{2-[3-(4-methylpiperazin-1-yl)propylamino]-4,6-dimethoxy-1,3-pyrimidin-5-yl}furan-2-carboxamid;
5-(4,4,7-Trimethyl-4-sila-chroman-6-yloxy)-*N*-{2-[3-(*N,N-*dimethylamino)propylamino]-4,6-dimethoxy-1,3-pyrimidin-5-yl}furan-2-carboxamid;
5-(4,4,7-Trimethyl-4-sila-chroman-6-yloxy)-*N*-{2-[3-(morpholin-4-yl)propylamino]4,6-dimethoxy-1,3-pyrimidin-5-yl}furan-2-carboxamid;
5-(1,2,3,4-Tetrahydro-4,4,7-trimethyl-4-sila-chinolin-6-yloxy)-*N*-(2,6-dimethoxyphenyl)furan-2-carboxamid;
5-(1,2,3,4-Tetrahydro-4,4,7-trimethyl-4-sila-chinolin-6-yloxy)-*N*-(2,4,6-trimethoxyphenyl)furan-2-carboxamid;
5-(1,2,3,4-Tetrahydro-4,4,7-trimethyl-4-sila-chinolin-6-yloxy)-*N*-(2,4,6-trimethoxy-1,3-pyrimidin-5-yl)furan-2-carboxamid;
5-(1,2,3,4-Tetrahydro-4,4,7-trimethyl-4-sila-chinolin-6-yloxy)-*N*-(2-methylamino-4,6-dimethoxy-1,3-pyrimidin-5-yl)furan-2-carboxamid;
5-(1,2,3,4-Tetrahydro-4,4,7-trimethy-4-sila-chinolin-6-yloxy)-*N*-{2-[3-(4-methylpiperazin-1-yl)propylamino]-4,6-dimethoxy-1,3-pyrimidin-5-yl}furan-2-carboxamid;
5-(1,2,3,4-Tetrahydro-4,4,7-trimethyl-4-sila-chinolin-6-yloxy)-*N*-{2-[3-(*N,N-*dimethylamino)propylamino]-4,6-dimethoxy-1,3-pyrimidin-5-yl}furan-2-carboxamid;
5-(1,2,3,4-Tetrahydro-4,4,7-trimethyl-4-sila-chinolin-6-yloxy)-*N*-{2-[3-(morpholin-4-yl)propylamino]-4,6-dimethoxy-1,3-pyrimidin-5-yl}furan-2-carboxamid;
5-(1,1,3,3,6-Pentamethyl-1,3-disila-2,3-dihydro-1*H*-inden-5-yloxy)-*N*-(2,6-dimethoxyphenyl)furan-2-carboxamid;
5-(1,1,3,3,6-Pentamethyl-1,3-disila-2,3-dihydro-1*H*-inden-5-yloxy)-*N*-(2,4,6-trimethoxyphenyl)furan-2-carboxamid;
5-(1,1,3,3,6-Pentamethyl-1,3-disila-2,3-dihydro-1*H*-inden-5-yloxy)-*N*-(2,4,6-trimethoxy-1,3-pyrimidin-5-yl)furan-2-carboxamid;
5-(1,1,3,3,6-Pentamethyl-1,3-disila-2,3-dihydro-1*H*-inden-5-yloxy)-*N*-(2-methylamino-4,6-dimethoxy-1,3-pyrimidin-5-yl)furan-2-carboxamid;
5-(1,1,3,3,6-Pentamethyl-1,3-disila-2,3-dihydro-1*H*-inden-5-yloxy)-*N*-{2-[3-(4-methylpiperazin-1-yl)propylamino]-4,6-dimethoxy-1,3-pyrimidin-5-yl}furan-2-carboxamid;
5-(1,1,3,3,6-Pentamethyl-1,3-disila-2,3-dihydro-1*H*-inden-5-yloxy)-*N*-{2-[3-(*N,N-*dimethylamino)propylamino]-4,6-dimethoxy-1,3-pyrimidin-5-yl}furan-2-carboxamid;
5-(1,1,3,3,6-Pentamethyl-1,3-disila-2,3-dihydro-1*H*-inden-5-yloxy)-*N*-{2-[3-(morpholin-4-yl)propylamino]-4,6-dimethoxy-1,3-pyrimidin-5-yl}furan-2-carboxamid;
5-(1,1,5-Trimethyl-1-sila-2,3-dihydro-1*H*-inden-6-yloxy)-*N*-(2,6-dimethoxyphenyl)furan-2-carboxamid;
5-(1,1,5-Trimethyl-1-sila-2,3-dihydro-1*H*-inden-6-yloxy)-*N*-(2,4,6-trimethoxyphenyl)furan-2-carboxamid;
5-(1,1,5-Trimethyl-1-sila-2,3-dihydro-1*H*-inden-6-yloxy)-*N*-(2,4,6-trimethoxy-1,3-pyrimidin-5-yl)furan-2-carboxamid;
5-(1,1,5-Trimethyl-1-sila-2,3-dihydro-1*H*-inden-6-yloxy)-*N*-(2-methylamino-4,6-dimethoxy-1,3-pyrimidin-5-yl)furan-2-carboxamid;
5-(1,1,5-Trimethyl-1-sila-2,3-dihydro-1*H*-inden-6-yloxy)-*N*-{2-[3-(4-methylpiperazin-1-yl)propylamino]-4,6-dimethoxy-1,3-pyrimidin-5-yl}furan-2-carboxamid;
5-(1,1,5-Trimethyl-1-sila-2,3-dihydro-1*H*-inden-6-yloxy)-*N*-{2-[3-(*N*,*N-*dimethylamino)propylamino]-4,6-dimethoxy-1,3-pyrimidin-5-yl}furan-2-carboxamid;
5-(1,1,5-Trimethyl-1-sila-2,3-dihydro-1*H*-inden-6-yloxy)-*N*-{2-[3-(morpholin-4-yl)propylamino]-4,6-dimethoxy-1,3-pyrimidin-5-yl}furan-2-carboxamid;
5-(1,1,5-Trimethyl-1-sila-2,3-dihydro-1*H*-inden-6-yloxy)-*N*-{2-[(pyridin-2-yl)methylamino]-4,6-dimethoxy-1,3-pyrimidin-5-yl}furan-2-carboxamid;
5-(1,1,6-Trimethyl-1-sila-2,3-dihydro-1*H*-inden-5-yloxy)-*N*-(2,6-dimethoxyphenyl)furan-2-carboxamid;
5-(1,1,6-Trimethyl-1-sila-2,3-dihydro-1*H*-inden-5-yloxy)-*N*-(2,4,6-trimethoxyphenyl)furan-2-carboxamid;
5-(1,1,6-Trimethyl-1-sila-2,3-dihydro-1*H*-inden-5-yloxy)-*N*-(2,4,6-trimethoxy-1,3-pyrimidin-5-yl)furan-2-carboxamid;
5-(1,1,6-Trimethyl-1-sila-2,3-dihydro-1*H*-inden-5-yloxy)-*N*-(2-methylamino-4,6-dimethoxy-1,3-pyrimidin-5-yl)furan-2-carboxamid;
5-(1,1,6-Trimethyl-1-sila-2,3-dihydro-1*H*-inden-5-yloxy)-*N*-{2-[3-(4-methylpiperazin-1-yl)propylamino]-4,6-dimethoxy-1,3-pyrimidin-5-yl}furan-2-carboxamid;
5-(1,1,6-Trimethyl-1-sila-2,3-dihydro-1*H*-inden-5-yloxy)-*N*-{2-[3-(*N,N-*dimethylamino)propylamino]-4,6-dimethoxy-1,3-pyrimidin-5-yl}furan-2-carboxamid;
5-(1,1,6-Trimethyl-1-sila-2,3-dihydro-1*H*-inden-5-yloxy)-*N*-{2-[3-(morpholin-4-yl)propylamino]-4,6-dimethoxy-1,3-pyrimidin-5-yl}furan-2-carboxamid;
5-(1,1,5-Trimethyl-1-sila-1*H*-inden-6-yloxy)-*N*-(2,6-dimethoxyphenyl)furan-2-carboxamid;
5-(1,1,5-Trimethyl-1-sila-1*H*-inden-6-yloxy)-*N*-(2,4,6-trimethoxyphenyl)furan-2-carboxamid;
5-(1,1,5-Trimethyl-1-sila-1*H*-inden-6-yloxy)-*N*-(2,4,6-trimethoxy-1,3-pyrimidin-5-yl)furan-2-carboxamid;
5-(1,1,5-Trimethyl-1-sila-1*H*-inden-6-yloxy)-*N*-(2-methylamino-4,6-dimethoxy-1,3-pyrimidin-5-yl)furan-2-carboxamid;
5-(1,1,5-Trimethyl-1-sila-1*H*-inden-6-yloxy)-*N*-{2-[3-(4-methylpiperazin-1-yl)propylamino]-4,6-dimethoxy-1,3-pyrimidin-5-yl}furan-2-carboxamid;
5-(1,1,5-Trimethyl-1-sila-1*H*-inden-6-yloxy)-*N*-{2-[3-(*N,N-*dimethylamino)propylamino]-4,6-dimethoxy-1,3-pyrimidin-5-yl}furan-2-carboxamid;
5-(1,1,5-Trimethyl-1-sila-1*H*-inden-6-yloxy)-*N*-{2-[3-(morpholin-4-yl)propylamino]-4,6-dimethoxy-1,3-pyrimidin-5-yl}furan-2-carboxamid;
5-(5-Methoxy-1,1-dimethyl-1-sila-1*H*-inden-6-yloxy)-*N*-(2,6-dimethoxyphenyl)furan-2-carboxamid;
5-(5-Methoxy-1,1-dimethyl-1-sila-1*H*-inden-6-yloxy)-*N*-(2,4,6-trimethoxyphenyl)furan-2-carboxamid;
5-(5-Methoxy-1,1-dimethyl-1-sila-1*H*-inden-6-yloxy)-*N*-(2,4,6-trimethoxy-1,3-pyrimidin-5-yl)furan-2-carboxamid;
5-(5-Methoxy-1,1-dimethyl-1-sila-1*H*-inden-6-yloxy)-*N*-(2-methylamino-4,6-dimethoxy-1,3-pyrimidin-5-yl)furan-2-carboxamid;
5-(5-Methoxy-1,1-dimethyl-1-sila-1*H*-inden-6-yloxy)-*N*-{2-[3-(4-methylpiperazin-1-yl)propylamino]-4,6-dimethoxy-1,3-pyrimidin-5-yl}furan-2-carboxamid;
5-(5-Methoxy-1,1-dimethyl-1-sila-1*H*-inden-6-yloxy)-*N*-{2-[3-(*N,N-*dimethylamino)propylamino]-4,6-dimethoxy-1,3-pyrimidin-5-yl}furan-2-carboxamid;
5-(5-Methoxy-1,1-dimethyl-1-sila-1*H*-inden-6-yloxy)-*N*-{2-[3-(morpholin-4-yl)propylamino]-4,6-dimethoxy-1,3-pyrimidin-5-yl}furan-2-carboxamid;
5-[1,1,6-Trimethyl-3-(2-propyl)-1-sila-2,3-dihydro-1*H*-inden-5-yloxy]-*N*-(2,6-dimethoxyphenyl)furan-2-carboxamid;
5-[1,1,6-Trimethyl-3-(2-propyl)-1-sila-2,3-dihydro-1*H*-inden-5-yloxy]-*N*-(2,4,6-trimethoxyphenyl)furan-2-carboxamid;
5-[1,1,6-Trimethyl-3-(2-propyl)-1-sila-2,3-dihydro-1*H*-inden-5-yloxy]-*N-*(2,4,6-trimethoxy-1,3-pyrimidin-5-yl)furan-2-carboxamid;
5-[1,1,6-Trimethyl-3-(2-propyl)-1-sila-2,3-dihydro-1*H*-inden-5-yloxy]-*N*-(2-methylamino-4,6-dimethoxy-1,3-pyrimidin-5-yl)furan-2-carboxamid;
5-[1,1,6-Trimethyl-3-(2-propyl)-1-sila-2,3-dihydro-1*H*-inden-5-yloxy]-*N*-{2-[3-(4-methylpiperazin-1-yl)propylamino]-4,6-dimethoxy-1,3-pyrimidin-5-yl}furan-2-carboxamid;
5-[1,1,6-Trimethyl-3-(2-propyl)-1-sila-2,3-dihydro-1*H*-inden-5-yloxy]-*N*-(2-[3-*(N,N*-dimethylamino)propylamino]-4,6-dimethoxy-1,3-pyrimidin-5-yl}furan-2-carboxamid;
5-[1,1,6-Trimethyl-3-(2-propyl)-1-sila-2,3-dihydro-1*H*-inden-5-yloxy]-*N*-{2-[3-(morpholin-4-yl)propylamino]-4,6-dimethoxy-1,3-pyrimidin-5-yl}furan-2-carboxamid;
5-(5-Chlor-1,1-dimethyl-1-sila-2,3-dihydro-1*H*-inden-6-yloxy)-*N*-(2,6-dimethoxyphenyl)furan-2-carboxamid;
5-(5-Chlor-1,1-dimethyl-1-sila-2,3-dihydro-1*H*-inden-6-yloxy)-*N*-(2,4,6-trimethoxyphenyl)furan-2-carboxamid;
5-(5-Chlor-1,-dimethyl-1-sila-2,3-dihydro-1*H*-inden-6-ytoxy)-*N*-(2,4,6-trimethoxy-1,3-pyrimidin-5-yl)furan-2-carboxamid;
5-(5-Chlor-1,1-dimethyl-1-sila-2,3-dihydro-1*H*-inden-6-yloxy)-*N*-(2-methylamino-4,6-dimethoxy-1,3-pyrimidin-5-yl)furan-2-carboxamid;
5-(5-Chlor-1,1-dimethyl-1-sila-2,3-dihydro-1*H*-inden-6-yloxy)-*N*-{2-[3-(4-methylpiperazin-1-yl)propylamino]-4,6-dimethoxy-1,3-pyrimidin-5-yl}furan-2-carboxamid;
5-(5-Chlor-1,1-dimethyl-1-sila-2,3-dihydro-1*H*-inden-6-yloxy)-*N*-{2-[3-(*N,N-*dimethylamino)propylamino]-4,6-dimethoxy-1,3-pyrimidin-5-yl}furan-2-carboxamid;
5-(5-Chlor-1,1-dimethyl-1-sila-2,3-dihydro-1*H*-inden-6-yloxy)-*N*-{2-[3-(morpholin-4-yl)propylamino]-4,6-dimethoxy-1,3-pyrimidin-5-yl}furan-2-carboxamid;
5-(5-Chlor-1,1,7-trimethyl-1-sila-2,3-dihydro-1*H*-inden-4-yloxy)-*N*-(2,6-dimethoxyphenyl)furan-2-carboxamid;
5-(5-Chlor-1,1,7-trimethyl-1-sila-2,3-dihydro-1*H*-inden-4-yloxy)-*N*-(2,4,6-trimethoxyphenyl)furan-2-carboxamid;
5-(5-Chlor-1,1,7-trimethyl-1-sila-2,3-dihydro-1*H*-inden-4-yloxy)-*N*-(2,4,6-trimethoxy-1,3-pyrimidin-5-yl)furan-2-carboxamid;
5-(5-Chlor-1,1,7-trimethyl-1-sila-2,3-dihydro-1*H*-inden-4-yloxy)-*N*-(2-methylamino-4,6-dimethoxy-1,3-pyrimidin-5-yl)furan-2-carboxamid;
5-(5-Chlor-1,1,7-trimethyl-1-sila-2,3-dihydro-1*H*-inden-4-yloxy)-*N*-{2-[3-(4-methylpiperazin-1-yl)propylamino]-4,6-dimethoxy-1,3-pyrimidin-5-yl}furan-2-carboxamid;
5-(5-Chlor-1,1,7-trimethyl-1-sila-2,3-dihydro-1*H*-inden-4-yloxy)-*N*-{2-[3-(*N,N-*dimethylamino)propylamino]-4,6-dimethoxy-1,3-pyrimidin-5-yl}furan-2-carboxamid;
5-(5-Chlor-1,1,7-trimethyl-1-sila-2,3-dihydro-1*H*-inden-4-yloxy)-*N*-{2-[3-(morpholin-4-yl)propylamino]-4,6-dimethoxy-1,3-pyrimidin-5-yl}furan-2-carboxamid;
5-(5-Methoxy-1,1-dimethyl-1-sila-2,3-dihydro-1*H*-inden-6-yloxy)-*N*-(2,6-dimethoxyphenyl)furan-2-carboxamid;
5-(5-Methoxy-1,1-dimethyl-1-sila-2,3-dihydro-1*H*-inden-6-yloxy)-*N*-(2,4,6-trimethoxyphenyl)furan-2-carboxamid;
5-(5-Methoxy-1,1-dimethyl-1-sila-2,3-dihydro-1*H*-inden-6-yloxy)-*N*-(2,4,6-trimethoxy-1,3-pyrimidin-5-yl)furan-2-carboxamid;
5-(5-Methoxy-1,1-dimethyl-1-sila-2,3-dihydro-1*H*-inden-6-yloxy)-*N*-(2-methylamino-4,6-dimethoxy-1,3-pyrimidin-5-yl)furan-2-carboxamid;
5-(5-Methoxy-1,1-dimethyl-1-sila-2,3-dihydro-1*H*-inden-6-yloxy)-*N*-{2-[3-(4-methylpiperazin-1-yl)propylamino]-4,6-dimethoxy-1,3-pyrimidin-5-yl}furan-2-carboxamid;
5-(5-Methoxy-1,1-dimethyl-1-sila-2,3-dihydro-1*H*-inden-6-yloxy)-*N*-{2-[3-(*N,N-*dimethylamino)propylamino]-4,6-dimethoxy-1,3-pyrimidin-5-yl}furan-2-carboxamid;
5-(5-Methoxy-1,1-dimethyl-1-sila-2,3-dihydro-1*H*-inden-6-yloxy)-*N*-{2-[3-(morpholin-4-yl)propylamino]-4,6-dimethoxy-1,3-pyrimidin-5-yl}furan-2-carboxamid;
5-(5-Methoxy-1,1-dimethyl-1-sila-2,3-dihydro-1*H*-inden-6-yloxy)-*N*-{2-[(pyridin-2-yl)methylamino]-4,6-dimethoxy-1,3-pyrimidin-5-yl}furan-2-carboxamid;
5-[(1,1,4,4,7-Pentamethyl-1,4-disila-1,2,3,4-tetrahydronaphthalen-6-yl)methyl]-*N*-{2-[3-(4-methylpiperazin-1-yl)propylamino]-4,6-dimethoxy-1,3-pyrimidin-5-yl}furan-2-carboxamid;
5-[(1,1,4,4,7-Pentamethyl-1,4-disila-1,2,3,4-tetrahydronaphthalen-6-yl)methyl]-*N*-{2-[3-(*N,N*-dimethylamino)propylamino]-4,6-dimethoxy-1,3-pyrimidin-5-yl}furan-2-carboxamid;
5-[(1,1,4,4,7-Pentamethyl-1,4-disila-1,2,3,4-tetrahydronaphthalen-6-yl)methyl]-*N*-{2-[3-(morpholin-4-yl)propylamino]-4,6-dimethoxy-1,3-pyrimidin-5-yl}furan-2-carboxamid;
5-[(1,1,3,3,6-Pentamethyl-1,3-disila-2,3-dihydro-1*H*-inden-5-yl)methyl]-*N*-(2,6-dimethoxyphenyl)furan-2-carboxamid;
5-[(1,1,3,3,6-Pentamethyl-1,3-disila-2,3-dihydro-1*H*-inden-5-yl)methyl]-*N*-(2,4,6-trimethoxyphenyl)furan-2-carboxamid;
5-[(1,1,3,3,6-Pentamethyl-1,3-disila-2,3-dihydro-1*H*-inden-5-yl)methyl]-*N*-(2,4,6-trimethoxy-1,3-pyrimidin-5-yl)furan-2-carboxamid;
5-[(1,1,3,3,6-Pentamethyl-1,3-disila-2,3-dihydro-1*H*-inden-5-yl)methyl]-*N*-(2-methylamino-4,6-dimethoxy-1,3-pyrimidin-5-yl)furan-2-carboxamid;
5-[(1,1,3,3,6-Pentamethyl-1,3-disila-2,3-dihydro-1*H*-inden-5-yl)methyl]-*N*-{2-[3-(4-methylpiperazin-1-yl)propylamino]-4,6-dimethoxy-1,3-pyrimidin-5-yl}furan-2-carboxamid;
5-[(1,1,3,3,6-Pentamethyl-1,3-disita-2.3-dihydro-1*H*-inden-5-yl)methyl]-*N*-{2-[3-(*N,N*-dimethylamino)propylamino]-4,6-dimethoxy-1,3-pyrimidin-5-yl}furan-2-carboxamid;
5-[(1,1,3,3,6-Pentamethyl-1,3-disila-2,3-dihydro-1*H*-inden-5-yl)methyl]-*N*-{2-[3-(morpholin-4-yl)propylamino]-4,6-dimethoxy-1,3-pyrimidin-5-yl}furan-2-carboxamid;
5-[(1,1,5-Trimethyl-1-sila-2,3-dihydro-1*H*-inden-6-yl)methyl]-*N*-(2,6-dimethoxyphenyl)furan-2-carboxamid;
5-[(1,1,5-Trimethyl-1-sila-2,3-dihydro-1*H*-inden-6-yl)methyl]-*N*-(2,4,6-trimethoxyphenyl)furan-2-carboxamid;
5-[(1,1,5-Trimethyl-1-sila-2,3-dihydro-1*H*-inden-6-yl)methyl]-*N*-(2,4,6-trimethoxy-1,3-pyrimid in-5-yl)furan-2-carboxamid;
5-[(1,1,5-Trimethyl-1-sila-2,3-dihydro-1*H*-inden-6-yl)methyl]-*N*-(2-methylamino-4,6-dimethoxy-1,3-pyrimidin-5-yl)furan-2-carboxamid;
5-[(1,1,5-Trimethyl-1-sila-2,3-dihydro-1*H*-inden-6-yl)methyl]-*N*-{2-[3-(4-methylpiperazin-1-yl)propylamino]-4,6-dimethoxy-1,3-pyrimidin-5-yl}furan-2-carboxamid;
5-[(1,1,5-Trimethyl-1-sila-2,3-dihydro-1*H*-inden-6-yl)methyl]-*N*-{2-[3-(*N,N-*dimethylamino)propylamino]-4,6-dimethoxy-1,3-pyrimidin-5-yl}furan-2-carboxamid;
5-[(1,1,5-Trimethyl-1-sila-2,3-dihydro-1*H*-inden-6-yl)methyl]-*N*-[2-(3-morpholinopropylamino)-4,6-dimethoxy-1,3-pyrimidin-5-yl]furan-2-carboxamid;
5-[(1,1,5-Trimethyl-1-sila-2,3-dihydro-1*H*-inden-6-yl)methyl]-*N*-{2-[(pyridin-2-yl)methylamino]-4,6-dimethoxy-1,3-pyrimidin-5-yl)furan-2-carboxamid; 5-[(1,1,5-Trimethyl-1-sila-2,3-dihydroinden-6-yl)methyl]-*N*-(4-chlor-2,6-dimethoxyphenyl)furan-2-carboxamid;
5-(1,1,3,4,4,6-Hexamethyl-1-sila-1,2,3,4-tetrahydronaphthalen-7-yloxy)-*N*-{[2-(2-(morpholin-4-yl)-ethylamino)-4,6-dimethoxy-1,3-pyrimidin-5-yl]furan-2-carboxamid;
5-(1,1,4,4,6-Pentamethyl-1,4-disila-1,2,3,4-tetrahydronaphthalen-5-oxy)-*N-*(2,4,6-trimethoxyphenyl)furan-2-carboxamid;
5-(7'-Hydroxy-1,1,1',1',6,6'-hexamethyl-3,3'-spiro-1,1'-disila-4,4'-dioxo-1,1',2,2',3,3',4,4'-octahydrodinaphthalen-7-ytoxy)-*N*-(2,6-dimethoxyphenyl)furan-2-carboxamid;
5-[1,1,2,6-Tetramethyl-3-(2-propyl)-1-sila-2,3-dihydroinden-5-yloxy]-*N*-(2,4,6-trimethoxyphenyl)furan-2-carboxamid;
5-(1,1,3,4,4-Pentamethyl-1-sila-1,2,3,4-tetrahydronaphthalen-7-yloxy)-*N*-(2,4,6-trimethoxy-1,3-pyrimidin-5-yl)furan-2-carboxamid;
5-(1,1,5-Trimethyl-1-sila-2,3-dihydroinden-6-yloxy)-*N*-(4-chlor-2,6-dimethoxyphenyl)furan-2-carboxamid;
5-(1,1,3,3,5-Pentamethyl-1-sila-2,3-dihydroinden-6-yloxy)-*N*-(2,6-dimethoxyphenyl)furan-2-carboxamid;
5-(1,1,3,3,6-Pentamethyl-1-sila-2,3-dihydroinden-5-yloxy)-*N*-(2,6-dimethoxyphenyl)furan-2-carboxamid;
5-(8-Methoxy-1,1,4,4,6-pentamethyl-1-sila-1,2,3,4-tetrahydronaphthalen-7-yloxy)-*N*-{2-[3-(morpholin-4-yl)-*N*-propylamino]-4,6-dimethoxy-1,3-pyrimidin-5-yl}furan-2-carboxamid;
5-(1,1,6-Trimethyl-1-sila-1,2,3,4-tetrahydronaphthalen-7-yloxy)-*N*-(2,4-dimethoxypyridin-3-yl)furan-2-carboxamid;
5-(1,1,3,4,4,6-Hexamethyl-1-sila-1,2,3,4-tetrahydronaphthalen-7-yloxy)-*N-*(2,4,6-trimethoxy-1,3-pyrimidin-5-yl)furan-2-carboxamid und
5-(1,1,3,4,4,6-Hexamethyl-1-sila-1,2,3,4-tetrahydronaphthalen-7-yloxy)-*N*-[2-(2-*N,N*-dimethylaminoethyl)-4,6-trimethoxy-1,3-pyrimidin-5-yl]furan-2-carboxamid.

21. Verbindung gemäß einem der vorhergehenden Ansprüche, die in der Form eines einzigen Enantiomers oder Diastereomers oder Tautomers vorliegt.

22. Verbindung gemäß einem der vorhergehenden Ansprüche, für die therapeutische Verwendung.

23. Pharmazeutische Zusammensetzung, die eine Verbindung nach einem der Ansprüche 1 bis 21 aufweist, und ein/einen pharmazeutisch verträgliches/en Verdünnungsmittel oder Träger.

24. Verwendung einer Verbindung nach einem der Ansprüche 1 bis 21 für die Herstellung eines Arzneimittels für die Krebstherapie.

25. Verwendung einer Verbindung nach einem der Ansprüche 1 bis 21 für die Herstellung eines Arzneimittels für die Behandlung oder Verhinderung von Endometriose, Gebärmuttermyom, einer Eierstockerkrankung, einer zystischen Brusterkrankung, Prostata Hypertrophie, Amenorrhoe, vorzeitiger Pubertät, prämenstruellem Syndrom, einer Sexualsteroid abhängigen Pathophysiologie oder gutartiger Prostata Hyperplasie oder um die Spermatogenese anzuhalten.

26. Verwendung gemäß Anspruch 25 für die Behandlung oder Verhinderung von schmerzhafter Endometriose, polycystischer Eierstockerkrankung oder sekundäre Amenorrhoe.

27. Verwendung einer Verbindung nach einem der Ansprüche 1 bis 21 für die Herstellung eines Arzneimittels für die Behandlung oder Verhinderung von Alzheimer'scher Erkrankung.

28. Verwendung einer Verbindung nach einem der Ansprüche 1 bis 21 für die Herstellung eines Arzneimittels für die Behandlung oder Verhinderung von HIV-Infektion oder AIDS.

29. Verwendung einer Verbindung nach einem der Ansprüche 1 bis 21 für die Herstellung eines Arzneimittels für die Behandlung oder Verhinderung einer Erkrankung, die durch eine Fehlfunktion des Thymus verursacht wird.

30. Verwendung gemäß Anspruch 29 für die Behandlung oder Verhinderung von multipler Sklerose, rheumatoider Arthritis oder Typ 1 Diabetes.

## Revendications

1. Composé de formule (I) ou de formule (II) 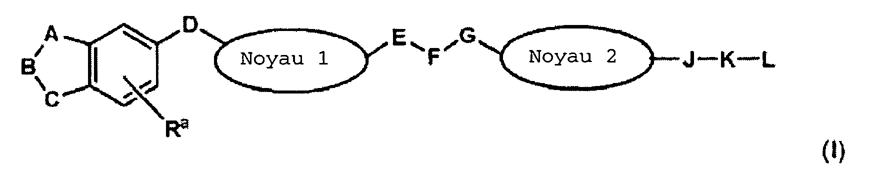 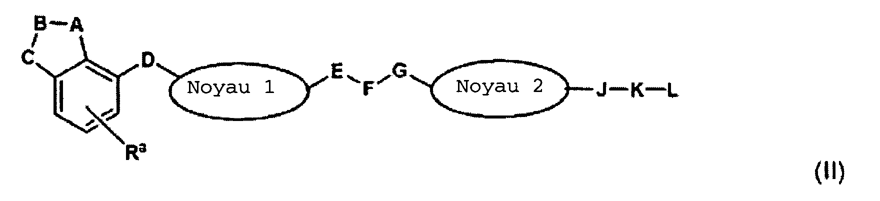 dans lesquelles
A et C sont identiques ou différents et représentent chacun une liaison, un groupe -(CH₂)ₙ-, -C(R^{b})₂-, -Si(R^{c})₂-, -O-, -S(O)ₘ-, -N=, -N(R^{b})-, -N(R^{e})C(=X)- ou -C(=X)- ;
B représente un groupe -(CH₂)ₙ-, -O-, -C(R^{b})₂-, -Si(R^{c})₂-, -C(R^{b})=C(R^{b})-, -C(R^{b})=, -(CH₂)ₙC(R^{g})₂-, -C(R^{g})₂(CH₂)ₙ- OU -CH(R^{b})CH(R^{b})- ;
dans lesquels n'importe lequel de A, B et C est facultativement substitué avec un substituant -Si(R^{c})₃ ;
D représente un groupe -(CH₂)ₙ-, -C(=X)-, -O-, -S(O)ₘ-, -C(=X)N(R^{e})-, -C(R^{b})₂-, -C(R^{b})=C(R^{b})-, -CH(R^{b})CH(R^{b})- ;
E est facultativement présent et représente un groupe -(CH₂)ₙ-, -N(R^{d})-, -(CH₂)ₙN(R^{d})- ou -N(R^{d})(CH₂)ₙ- ;
F représente un groupe -C(=X)- ou -N(R^{d})- ;
G représente un groupe -(CH₂)ₙ-, -N(R^{d})-, -(CH₂)ₙN(R^{d})- ou -N(R^{d})(CH₂)ₙ ;
J est facultativement présent et représente un groupe -O-, -N(R^{c})C(=X)-, -C(=X)N(R^{c})-, -S(O)ₘ-, -N(R^{c})S(O)ₘ-, -S(O)ₘN(R^{c})- ou -N(R^{e})-;
K est facultativement présent et représente un groupe alkylène facultativement substitué avec R^{b} ; ou bien K représente un groupe cycloalkylène, cycloalcénylène, arylène, hétérocycloalkylène, hétérocycloalkylène ou hétéroarylène, n'importe lequel de ces groupes étant facultativement substitué avec R^{a} ;
L représente un atome d'hydrogène, un atome d'halogène, un groupe -N(R^{f})₂, cycloalkyle, cycloalcényle, aryle, hétérocycloalkyle, hétérocycloalcényle ou hétéroaryle, n'importe lequel de ces groupes étant facultativement substitué avec un substituant R^{a}, -C(=X)OR^{d}, -OH, -OR^{c}, -C(=X)N(R^{b})(R^{c}), S(O)ₘN(R^{b})(R^{c}) ou -CN ;
les groupes R^{a} sont identiques ou différents et représentent chacun un atome d'hydrogène, un atome d'halogène, un groupe alkyle, aryle, hydroxy, alkoxy, -alkoxy-(CH₂)ₙC(O)₂R^{b}, -O-aryle, -C(=X)R^{c}, -NO₂, -CN, -N(R^{c})C(=X)R^{c}, -C(=X)N(R^{c})₂, -S(O)₂N(R^{c})₂ ou -N(R^{e})₂ ;
les groupes R^{b} sont identiques ou différents et représentent chacun un atome d'hydrogène ou un groupe alkyle ;
les groupes R^{c} sont identiques ou différents et représentent chacun un groupe alkyle, cycloalkyle, -alkyl-aryle, -alkyl-cycloalkyle ou aryle facultativement substitué avec R^{a} ;
les groupes R^{d} sont identiques ou différents et représentent chacun un atome d'hydrogène, un groupe alkyle ou aryle facultativement substitué avec R^{a} ;
les groupes R^{e} sont identiques ou différents et représentent chacun un atome d'hydrogène ou un groupe alkyle ; ou bien R^{e} représente un groupe aryle ou hétéroaryle, chacun de ces groupes étant facultativement substitué avec R^{a} ;
les groupes R^{f} sont identiques ou différents et représentent chacun un atome d'hydrogène ou un groupe alkyle ; ou bien R^{f}-N-R^{f}, dont les composants sont pris conjointement, forme un groupe hétérocycloalkyle, hétérocycloalcényle ou hétéroaryle ;
R^{g}-C-R^{g}, dont les composants sont pris conjointement, forme un groupe hétérocyclyle ;
les groupes X sont identiques ou différents et représentent chacun un atome d'oxygène ou de soufre ;
les Noyaux 1 et 2 sont identiques ou différents et représentent chacun un groupe arylène ou hétéroarylène, chacun de ces groupes étant facultativement substitué avec R^{a} ;
les indices m sont identiques ou différents et sont chacun égaux à 0, 1 ou 2 ; et
les indices n sont identiques ou différents et sont chacun égaux à 0, 1, 2 ou 3 ;
sous réserve qu'au moins un de A, B et C comprenne un atome de silicium ; A et C ne représentent pas chacun une liaison et le composé ne comprend pas une liaison Si-Si, une liaison simple N-N, une liaison Si-O, une liaison Si-N ou un liaison N-O-N ;
sous réserve en outre que le composé ne réponde pas à la formule 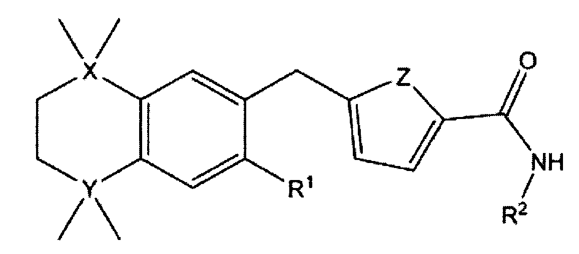 dans laquelle
un de X et Y représente le silicium, et l'autre représente le carbone ou le silicium ;
Z représente l'oxygène, le soufre ou un groupe -N(R)-, dans lequel R représente l'hydrogène ou un groupe alkyle ;
R¹ représente l'hydrogène, un atome d'halogène, un groupe alkyle, alcényle, alcynyle, alkoxy ou cycloalkyle ; et
R² représente un groupe alkyle, alcényle, alcynyle, cycloalkyle, hétérocycloalkyle, aryle, hétéroaryle, -alkyl-cycloalkyle, -alkyl-hétérocycloalkyle, -alkyl-aryle ou -alkyl-hétéroaryle ;
ou un de ses sels pharmaceutiquement acceptables.

2. Composé suivant la revendication 1, qui est un composé de formule (I).

3. Composé suivant la revendication 1 ou la revendication 2, dans lequel A et/ou C représentent un groupe -Si(R^{c})₂-.

4. Composé suivant la revendication 3, dans lequel les groupes R^{c} sont identiques ou différents et représentent chacun un groupe alkyle.

5. Composé suivant la revendication 1 ou la revendication 2, dans lequel A et/ou C représentent un groupe -C(R^{b})₂-, -(CH₂)ₙ-, -N(R^{b})- ou -O-.

6. Composé suivant la revendication 5, dans lequel les groupes R^{b} sont identiques ou différents et représentent chacun un atome d'hydrogène ou un groupe alkyle.

7. Composé suivant l'une quelconque des revendications précédentes, dans lequel B représente un groupe -(CH₂)ₙ-, -C(R^{b})₂-, -CH(R^{b})CH(R^{b})-, -C(R^{b})=C(R^{b})- ou -CH₂-C(R^{g})₂-.

8. Composé suivant la revendication 7, dans lequel les groupes R^{b} sont identiques ou différents et représentent chacun un atome d'hydrogène ou un groupe alkyle.

9. Composé suivant l'une quelconque des revendications précédentes, dans lequel D représente un groupe -O-, -S- ou -CH₂-.

10. Composé suivant l'une quelconque des revendications précédentes, dans lequel E est absent.

11. Composé suivant l'une quelconque des revendications précédentes, dans lequel F représente un groupe -C(O)-.

12. Composé suivant l'une quelconque des revendications précédentes, dans lequel G représente un groupe -N(R^{d})-.

13. Composé suivant la revendication 12, dans lequel R^{d} représente un atome d'hydrogène.

14. Composé suivant l'une quelconque des revendications précédentes, dans lequel J et K sont absents et L représente un atome d'hydrogène ou un groupe -N(R^{f})₂.

15. Composé suivant l'une quelconque des revendications 1 à 13, dans lequel J représente un groupe -NH-, K représente un groupe alkylène et L représente un groupe hétérocycloalkyle.

16. Composé suivant l'une quelconque des revendications précédentes, dans lequel le Noyau 1 est un noyau hétéroarylène.

17. Composé suivant la revendication 16, dans lequel le Noyau 1 est un noyau furannylène.

18. Composé suivant l'une quelconque des revendications précédentes, dans lequel le Noyau 2 est un noyau phényle, pyrimidinyle ou pyridinyle, chacun étant facultativement substitué.

19. Composé suivant la revendication 18, dans lequel le Noyau 2 est substitué 1, 2 ou 3 fois, les substituants étant identiques ou différents à chaque occurrence et étant choisis entre des substituants alkoxy et halogéno.

20. Composé suivant la revendication 1, choisi entre :
5-(1,1,4,4,7-pentaméthyl-1,4-disila-1,2,3,4-tétrahydronaphtalène-6-yloxy)-*N*-(2,6-diméthoxyphényl)furanne-2-carboxamide ;
5-(1,1,4,4,7-pentaméthyl-1,4-disila-1,2,3,4-tétrahydronaphtalène-6-yloxy)-*N*-(2,4,6-triméthoxyphényl)furanne-2-carboxamide ;
5-(1,1,4,4,7-pentaméthyl-1,4-disila-1,2,3,4-tétrahydronaphtalène-6-yloxy)-*N*-(2,4,6-triméthoxy-1,3-pyrimidine-5-yl)furanne-2-carboxamide ;
5-(1,1,4,4,7-pentaméthyl-1,4-disila-1,2,3,4-tétrahydronaphtalène-6-yloxy)-*N*-(2-méthylamino-4,6-diméthoxy-1,3-pyrimidine-5-yl)furanne-2-carboxamide ;
5-(1,1,4,4,7-pentaméthyl-1,4-disila-1,2,3,4-tétrahydronaphtalène-6-yloxy)-*N*-{2-[3-(4-méthylpipérazine-1-yl)propylamino]-4,6-diméthoxy-1,3-pyrimidine-5-yl}furanne-2-carboxamide ;
5-(1,1,4,4,7-pentaméthyl-1,4-disila-1,2,3,4-tétrahydronaphtalène-6-yloxy)-*N*-{2-[3-(*N,N*-diméthylamino)propylamino]-4,6-diméthoxy-1,3-pyrimidine-5-yl}furanne-2-carboxamide ;
5-(1,1,4,4,7-pentaméthyl-1,4-disila-1,2,3,4-tétrahydronaphtalène-6-yloxy)-*N*-{2-[3-(morpholine-4-yl)propylamino]-4,6-diméthoxy-1,3-pyrimidine-5-yl}furanne-2-carboxamide ;
5-(1,1,6-triméthyl-1-sila-1,2,3,4-tétrahydronaphtalène-7-yloxy)-*N*-(2,6-diméthoxyphényl)furanne-2-carboxamide ;
5-(1,1,6-triméthyl-1-sila-1,2,3,4-tétrahydronaphtalène-7-yloxy)-*N*-(2,4,6-triméthoxyphényl)furanne-2-carboxamide ;
5-(1,1,6-triméthyl-1-sila-1,2,3,4-tétrahydronaphtalène-7-yloxy)-*N*-(2,4,6-triméthoxy-1,3-pyrimidine-5-yl)furanne-2-carboxamide ;
5-(1,1,6-triméthyl-1-sila-1,2,3,4-tétrahydronaphtalène-7-yloxy)-*N*-(2-méthylamino-4,6-diméthoxy-1,3-pyrimidine-5-yl)furanne-2-carboxamide ;
5-(1,1,6-triméthyl-1-sila-1,2,3,4-tétrahydronaphtalène-7-yloxy)-*N*-{2-[3-(4-méthylpipérazine-1-yl)propylamino]-4,6-diméthoxy-1,3-pyrimidine-5-yl}furanne-2-carboxamide ;
5-(1,1,6-triméthyl-1-sila-1,2,3,4-tétrahydronaphtalène-7-yloxy)-*N*-{2-[3-(*N,N*-diméthylamino)propylamino]-4,6-diméthoxy-1,3-pyrimidine-5-yl}furanne-2-carboxamide ;
5-(1,1,6-triméthyl-1-sila-1,2,3,4-tétrahydronaphtalène-7-yloxy)-*N*-{2-[3-(morpholine-4-yl)propylamino]-4,6-diméthoxy-1,3-pyrimidine-5-yl}furanne-2-carboxamide ;
5-(1,1,7-triméthyl-1-sila-1,2,3,4-tétrahydronaphtalène-6-yloxy)-*N*-(2,6-diméthoxyphényl)furanne-2-carboxamide ;
5-(1,1,7-triméthyl-1-sila-1,2,3,4-tétrahydronaphtalène-6-yloxy)-*N*-(2,4,6-triméthoxyphényl)furanne-2-carboxamide ;
5-(1,1,7-triméthyl-1-sila-1,2,3,4-tétrahydronaphtalène-6-yloxy)-*N*-(2,4,6-triméthoxy-1,3-pyrimidine-5-yl)furanne-2-carboxamide ;
5-(1,1,7-triméthyl-1-sila-1,2,3,4-tétrahydronaphtalène-6-yloxy)-*N*-(2-méthylamino-4,6-diméthoxy-1,3-pyrimidine-5-yl)furanne-2-carboxamide ;
5-(1,1,7-triméthyl-1-sila-1,2,3,4-tétrahydronaphtalène-6-yloxy)-*N*-{2-[3-(4-méthylpipérazine-1-yl)propylamino]-4,6-diméthoxy-1,3-pyrimidine-5-yl}furanne-2-carboxamide ;
5-(1,1,7-triméthyl-1-sila-1,2,3,4-tétrahydronaphtalène-6-yloxy)-*N*-{2-[3-(*N,N*-diméthylamino)propylamino]-4,6-diméthoxy-1,3-pyrimidine-5-yl}furanne-2-carboxamide ;
5-(1,1,7-triméthyl-1-sila-1,2,3,4-tétrahydronaphtalène-6-yloxy)-*N*-{2-[3-(morpholine-4-yl)propylamino]-4,6-diméthoxy-1,3-pyrimidine-5-yl}furanne-2-carboxamide ;
5-(1,1,2,4,4,7-hexaméthyl-1,4-disila-1,2,3,4-tétrahydronaphtalène-6-yloxy)-*N*-(2,6-diméthoxyphényl)furanne-2-carboxamide ;
5-(1,1,2,4,4,7-hexaméthyl-1,4-disila-1,2,3,4-tétrahydronaphtalène-6-yloxy)-*N*-(2,4,6-triméthoxyphényl)furanne-2-carboxamide ;
5-(1,1,2,4,4,7-hexaméthyl-1,4-disila-1,2,3,4-tétrahydronaphtalène-6-yloxy)-*N*-(2,4,6-triméthoxy-1,3-pyrimidine-5-yl)furanne-2-carboxamide ;
5-(1,1,2,4,4,7-hexaméthyl-1,4-disila-1,2,3,4-tétrahydronaphtalène-6-yloxy)-*N*-(2-méthylamino-4,6-diméthoxy-1,3-pyrimidine-5-yl)furanne-2-carboxamide ;
5-(1,1,2,4,4,7-hexaméthyl-1,4-disila-1,2,3,4-tétrahydronaphtalène-6-yloxy)-*N*-{2-[3-(4-méthylpipérazine-1-yl)propylamino]-4,6-diméthoxy-1,3-pyrimidine-5-yl}furanne-2-carboxamide ;
5-(1,1,2,4,4,7-hexaméthyl-1,4-disila-1,2,3,4-tétrahydronaphtalène-6-yloxy)-*N*-{2-[3-(*N,N*-diméthylamino)propylamino]-4,6-diméthoxy-1,3-pyrimidine-5-yl}furanne-2-carboxamide ;
5-(1,1,2,4,4,7-hexaméthyl-1,4-disila-1,2,3,4-tétrahydronaphtalène-6-yloxy)-*N*-{2-[3-(morpholine-4-yl)propylamino]-4,6-diméthoxy-1,3-pyrimidine-5-yl}furanne-2-carboxamide ;
5-(5-méthoxy-1,1,4,4,7-pentaméthyl-1,4-disila-1,2,3,4-tétrahydronaphtalène-6-yloxy)-*N*-(2,6-diméthoxyphényl)furanne-2-carboxamide ;
5-(5-méthoxy-1,1,4,4,7-pentaméthyl-1,4-disila-1,2,3,4-tétrahydronaphtalène-6-yloxy)-*N*-(2,4,6-triméthoxyphényl)furanne-2-carboxamide ;
5-(5-méthoxy-1,1,4,4,7-pentaméthyl-1,4-disila-1,2,3,4-tétrahydronaphtalène-6-yloxy)-*N*-(2,4,6-triméthoxy-1,3-pyrimidine-5-yl)furanne-2-carboxamide ;
5-(5-méthoxy-1,1,4,4,7-pentaméthyl-1,4-disila-1,2,3,4-tétrahydronaphtalène-6-yloxy)-*N*-(2-méthylamino-4,6-diméthoxy-1,3-pyrimidine-5-yl)furanne-2-carboxamide ;
5-(5-méthoxy-1,1,4,4,7-pentaméthyl-1,4-disila-1,2,3,4-tétrahydronaphtalène-6-yloxy)-*N*-{2-[3-(4-méthylpipérazine-1-yl)propylamino]-4,6-triméthoxy-1,3-pyrimidine-5-yl}furanne-2-carboxamide ;
5-(5-méthoxy-1,1,4,4,7-pentaméthyl-1,4-disila-1,2,3,4-tétrahydronaphtalène-6-yloxy)-*N*-{2-[3-(*N,N*-diméthylamino)-propylamino]-4,6-diméthoxy-1,3-pyrimidine-5-yl}furanne-2-carboxamide ;
5-(5-méthoxy-1,1,4,4,7-pentaméthyl-1,4-disila-1,2,3,4-tétrahydronaphtalène-6-yloxy)-*N*-{2-[3-(morpholine-4-yl)propylamino]-4,6-diméthoxy-1,3-pyrimidine-5-yl}furanne-2-carboxamide ;
5-(8-méthoxy-1,1,6-triméthyl-1-sila-1,2,3,4-tétrahydronaphtalène-7-yloxy)-*N*-(2,6-diméthoxyphényl)furanne-2-carboxamide ;
5-(8-méthoxy-1,1,6-triméthyl-1-sila-1,2,3,4-tétrahydronaphtalène-7-yloxy)-*N*-(2,4,6-triméthoxyphényl)furanne-2-carboxamide ;
5-(8-méthoxy-1,1,6-triméthyl-1-sila-1,2,3,4-tétrahydronaphtalène-7-yloxy)-*N*-(2,4,6-triméthoxy-1,3-pyrimidine-5-yl)furanne-2-carboxamide ;
5-(8-méthoxy-1,1,6-triméthyl-1-sila-1,2,3,4-tétrahydronaphtalène-7-yloxy)-*N*-(2-méthylamino-4,6-diméthoxy-1,3-pyrimidine-5-yl)furanne-2-carboxamide ;
5-(8-méthoxy-1,1,6-triméthyl-1-sila-1,2,3,4-tétrahydronaphtalène-7-yloxy)-*N*-{2-[3-(4-méthylpipérazine-1-yl)propylamino]-4,6-diméthoxy-1,3-pyrimidine-5-yl}furanne-2-carboxamide ;
5-(8-méthoxy-1,1,6-triméthyl-1-sila-1,2,3,4-tétrahydronaphtalène-7-yloxy)-*N*-{2-[3-(*N,N*-diméthylamino)propylamino]-4,6-diméthoxy-1,3-pyrimidine-5-yl}furanne-2-carboxamide ;
5-(8-méthoxy-1,1,6-triméthyl-1-sila-1,2,3,4-tétrahydronaphtalène-7-yloxy)-*N*-{2-[3-(morpholine-4-yl)propylamino]-4,6-diméthoxy-1,3-pyrimidine-5-yl}furanne-2-carboxamide ;
5-(1,1,4,4,5,7-hexaméthyl-1,4-disila-1,2,3,4-tétrahydronaphtalène-6-yloxy)-*N*-(2,6-diméthoxyphényl)furanne-2-carboxamide ;
5-(1,1,4,4,5,7-hexaméthyl-1,4-disila-1,2,3,4-tétrahydronaphtalène-6-yloxy)-*N*-(2,4,6-triméthoxyphényl)furanne-2-carboxamide ;
5-(1,1,4,4,5,7-hexaméthyl-1,4-disila-1,2,3,4-tétrahydronaphtalène-6-yloxy)-*N*-(2,4,6-triméthoxy-1,3-pyrimidine-5-yl)furanne-2-carboxamide ;
5-(1,1,4,4,5,7-hexaméthyl-1,4-disila-1,2,3,4-tétrahydronaphtalène-6-yloxy)-*N*-(2-méthylamino-4,6-diméthoxy-1,3-pyrimidine-5-yl)furanne-2-carboxamide ;
5-(1,1,4,4,5,7-hexaméthyl-1,4-disila-1,2,3,4-tétrahydronaphtalène-6-yloxy)-*N*-{2-[3-(4-méthylpipérazine-1-yl)propylamino]-4,6-diméthoxy-1,3-pyrimidine-5-yl}furanne-2-carboxamide ;
5-(1,1,4,4,5,7-hexaméthyl-1,4-disila-1,2,3,4-tétrahydronaphtalène-6-yloxy)-*N*-{2-[3-(*N*,*N*-diméthylamino)propylamino]-4,6-diméthoxy-1,3-pyrimidine-5-yl}furanne-2-carboxamide ;
5-(1,1,4,4,5,7-hexaméthyl-1,4-disila-1,2,3,4-tétrahydronaphtalène-6-yloxy)-*N*-{2-[3-(morpholine-4-yl)propylamino]-4,6-diméthoxy-1,3-pyrimidine-5-yl}furanne-2-carboxamide ;
5-(6-méthoxy-1,1,4,4,8-pentaméthyl-1,4-disila-1,2,3,4-tétrahydronaphtalène-7-yloxy)-*N*-(2,6-diméthoxyphényl)furanne-2-carboxamide ;
5-(6-méthoxy-1,1,4,4,8-pentaméthyl-1,4-disila-1,2,3,4-tétrahydronaphtalène-7-yloxy)-*N*-(2,4,6-triméthoxyphényl)furanne-2-carboxamide ;
5-(6-méthoxy-1,1,4,4,8-pentaméthyl-1,4-disila-1,2,3,4-tétrahydronaphtalène-7-yloxy)-*N*-(2,4,6-triméthoxy-1,3-pyrimidine-5-yl)furanne-2-carboxamide ;
5-(6-méthoxy-1,1,4,4,8-pentaméthyl-1,4-disila-1,2,3,4-tétrahydronaphtalène-7-yloxy)-*N*-(2-méthylamino-4,6-diméthoxy-1,3-pyrimidine-5-yl)furanne-2-carboxamide ;
5-(6-méthoxy-1,1,4,4,8-pentaméthyl-1,4-disila-1,2,3,4-tétrahydronaphtalène-7-yloxy)-*N*-{2-[3-(4-méthylpipérazine-1-yl)propylamino]-4,6-diméthoxy-1,3-pyrimidine-5-yl}furanne-2-carboxamide ;
5-(6-méthoxy-1,1,4,4,8-pentaméthyl-1,4-disila-1,2,3,4-tétrahydronaphtalène-7-yloxy)-*N*-{2-[3-(*N,N*-diméthylamino)-propylamino]-4,6-diméthoxy-1,3-pyrimidine-5-yl}furanne-2-carboxamide ;
5-(6-méthoxy-1,1,4,4,8-pentaméthyl-1,4-disila-1,2,3,4-tétrahydronaphtalène-7-yloxy)-*N*-{2-[3-(morpholine-4-yl)propylamino]-4,6-diméthoxy-1,3-pyrimidine-5-yl}furanne-2-carboxamide ;
5-(6-chloro-1,1,4,4-tétraméthyl-1,4-disila-1,2,3,4-tétrahydronaphtalène-7-yloxy)-*N*-(2,6-diméthoxyphényl)furanne-2-carboxamide ;
5-(6-chloro-1,1,4,4-tétraméthyl-1,4-disila-1,2,3,4-tétrahydronaphtalène-7-yloxy)-*N*-(2,4,6-triméthoxyphényl)furanne-2-carboxamide ;
5-(6-chloro-1,1,4,4-tétraméthyl-1,4-disila-1,2,3,4-tétrahydronaphtalène-7-yloxy)-*N*-(2,4,6-triméthoxy-1,3-pyrimidine-5-yl)furanne-2-carboxamide ;
5-(6-chloro-1,1,4,4-tétraméthyl-1,4-disila-1,2,3,4-tétrahydronaphtalène-7-yloxy)-*N*-(2-méthylamino-4,6-diméthoxy-1,3-pyrimidine-5-yl)furanne-2-carboxamide ;
5-(6-chloro-1,1,4,4-tétraméthyl-1,4-disila-1,2,3,4-tétrahydronaphtalène-7-yloxy)-*N*-{2-[3-(4-méthylpipérazine-1-yl)-propylamino]-4,6-diméthoxy-1,3-pyrimidine-5-yl}furanne-2-carboxamide ;
5-(6-chloro-1,1,4,4-tétraméthyl-1,4-disila-1,2,3,4-tétrahydronaphtalène-7-yloxy)-*N*-{2-[3-(*N,N*-diméthylamino)propylamino]-4,6-diméthoxy-1,3-pyrimidine-5-yl}furanne-2-carboxamide ;
5-(6-chloro-1,1,4,4-tétraméthyl-1,4-disila-1,2,3,4-tétrahydronaphtalène-7-yloxy)-*N*-{2-(3-morpholine-4-yl)propylamino]-4,6-diméthoxy-1,3-pyrimidine-5-yl}furanne-2-carboxamide ;
5-(6-chloro-1,1-diméthyl-1-sila-1,2,3,4-tétrahydronaphtalène-7-yloxy)-*N*-(2,6-diméthoxyphényl)furanne-2-carboxamide ;
5-(6-chloro-1,1-diméthyl-1-sila-1,2,3,4-tétrahydronaphtalène-7-yloxy)-*N*-(2,4,6-triméthoxyphényl)furanne-2-carboxamide ;
5-(6-chloro-1,1-diméthyl-1-sila-1,2,3,4-tétrahydronaphtalène-7-yloxy)-*N*-(2,4,6-triméthoxy-1,3-pyrimidine-5-yl)furanne-2-carboxamide ;
5-(6-chloro-1,1-diméthyl-1-sila-1,2,3,4-tétrahydronaphtalène-7-yloxy)-*N*-(2-méthylamino-4,6-diméthoxy-1,3-pyrimidine-5-yl)furanne-2-carboxamide ;
5-(6-chloro-1,1-diméthyl-1-sila-1,2,3,4-tétrahydronaphtalène-7-yloxy)-*N*-{2-[3-(4-méthylpipérazine-1-yl)propylamino]-4,6-diméthoxy-1,3-pyrimidine-5-yl}furanne-2-carboxamide ;
5-(6-chloro-1,1-diméthyl-1-sila-1,2,3,4-tétrahydronaphtalène-7-yloxy)-*N*-{2-[3-(*N,N*-diméthylamino)propylamino]-4,6-diméthoxy-1,3-pyrimidine-5-yl}furanne-2-carboxamide ;
5-(6-chloro-1,1-diméthyl-1-sila-1,2,3,4-tétrahydronaphtalène-7-yloxy)-*N*-{2-[3-(morpholine-4-yl)propylamino]-4,6-diméthoxy-1,3-pyrimidine-5-yl}furanne-2-carboxamide ;
5-(4,4,7-triméthyl-4-sila-chromane-6-yloxy)-*N*-(2,6-diméthoxyphényl)furanne-2-carboxamide ;
5-(4,4,7-triméthyl-4-sila-chromane-6-yloxy)-*N*-(2,4,6-triméthoxyphényl)furanne-2-carboxamide ;
5-(4,4,7-triméthyl-4-sila-chromane-6-yloxy)-*N*-(2,4,6-triméthoxy-1,3-pyrimidine-5-yl)furanne-2-carboxamide ;
5-(4,4,7-triméthyl-4-sila-chromane-6-yloxy)-*N*-(2-méthylamino-4,6-diméthoxy-1,3-pyrimidine-5-yl)furanne-2-carboxamide ;
5-(4,4,7-triméthyl-4-sila-chromane-6-yloxy)-*N*-{2-[3-(4-méthylpipérazine-1-yl)propylamino]-4,6-diméthoxy-1,3-pyrimidine-5-yl}furanne-2-carboxamide ;
5-(4,4,7-triméthyl-4-sila-chromane-6-yloxy)-*N*-{2-[3-(*N,N-*diméthylamino)propylamino]-4,6-diméthoxy-1,3-pyrimidine-5-yl}furanne-2-carboxamide ;
5-(4,4,7-triméthyl-4-sila-chromane-6-yloxy)-*N*-{2-[3-(morpholine-4-yl)propylamino]-4,6-diméthoxy-1,3-pyrimidine-5-yl}furanne-2-carboxamide ;
5-(1,2,3,4-tétrahydro-4,4,7-triméthyl-4-sila-quinoléine-6-yloxy)-*N*-(2,6-diméthoxyphényl)furanne-2-carboxamide ;
5-(1,2,3,4-tétrahydro-4,4,7-triméthyl-4-sila-quinoléine-6-yloxy)-*N*-(2,4,6-triméthoxyphényl)furanne-2-carboxamide ;
5-(1,2,3,4-tétrahydro-4,4,7-triméthyl-4-sila-quinoléine-6-yloxy)-*N*-(2,4,6-triméthoxy-1,3-pyrimidine-5-yl)furanne-2-carboxamide ;
5-(1,2,3,4-tétrahydro-4,4,7-triméthyl-4-sila-quinoléine-6-yloxy)-*N*-(2-méthylamino-4,6-diméthoxy-1,3-pyrimidine-5-yl)-furanne-2-carboxamide ;
5-(1,2,3,4-tétrahydro-4,4,7-triméthyl-4-sila-quinoléine-6-yloxy)-*N*-{2-[3-(4-méthylpipérazine-1-yl)propylamino]-4,6-diméthoxy-1,3-pyrimidine-5-yl}furanne-2-carboxamide ;
5-(1,2,3,4-tétrahydro-4,4,7-triméthyl-4-sila-quinoléine-6-yloxy)-*N*-{2-[3-(*N,N*-diméthylamino)propylamino]-4,6-diméthoxy-1,3-pyrimidine-5-yl}furanne-2-carboxamide ;
5-(1,2,3,4-tétrahydro-4,4,7-triméthyl-4-sila-quinoléine-6-yloxy)-*N*-{2-[3-(morpholine-4-yl)propylamino]-4,6-diméthoxy-1,3-pyrimidine-5-yl}furanne-2-carboxamide ;
5-(1,1,3,3,6-pentaméthyl-1,3-disila-2,3-dihydro-1*H*-indène-5-yloxy)-*N*-(2,6-diméthoxyphényl)furanne-2-carboxamide ;
5-(1,1,3,3,6-pentaméthyl-1,3-disila-2,3-dihydro-1*H*-indène-5-yloxy)-*N*-(2,4,6-triméthoxyphényl)furanne-2-carboxamide ;
5-(1,1,3,3,6-pentaméthyl-1,3-disila-2,3-dihydro-1*H*-indène-5-yloxy)-*N*-(2,4,6-triméthoxy-1,3-pyrimidine-5-yl)furanne-2-carboxamide ;
5-(1,1,3,3,6-pentaméthyl-1,3-disila-2,3-dihydro-1*H*-indène-5-yloxy)-*N*-(2-méthylamino-4,6-diméthoxy-1,3-pyrimidine-5-yl)-furanne-2-carboxamide ;
5-(1,1,3,3,6-pentaméthyl-1,3-disila-2,3-dihydro-1*H*-indène-5-yloxy)-*N*-{2-[3-(4-méthylpipérazine-1-yl)propylamino]-4,6-diméthoxy-1,3-pyrimidine-5-yl}furanne-2-carboxamide ;
5-(1,1,3,3,6-pentaméthyl-1,3-disila-2,3-dihydro-1*H*-indène-5-yloxy)-*N*-{2-[3-(*N,N*-diméthylamino)propylamino]-4,6-diméthoxy-1,3-pyrimidine-5-yl}furanne-2-carboxamide ;
5-(1,1,3,3,6-pentaméthyl-1,3-disila-2,3-dihydro-1*H*-indène-5-yloxy)-*N*-{2-[3-(morpholine-4-yl)propylamino]-4,6-diméthoxy-1,3-pyrimidine-5-yl}furanne-2-carboxamide ;
5-(1,1,5-triméthyl-1-sila-2,3-dihydro-1*H*-indène-6-yloxy)-*N*-(2,6-diméthoxyphényl)furanne-2-carboxamide ;
5-(1,1,5-triméthyl-1-sila-2,3-dihydro-1*H*-indène-6-yloxy)-*N*-(2,4,6-triméthoxyphényl)furanne-2-carboxamide ;
5-(1,1,5-triméthyl-1-sila-2,3-dihydro-1*H*-indène-6-yloxy)-*N*-(2,4,6-triméthoxy-1,3-pyrimidine-5-yl)furanne-2-carboxamide ;
5-(1,1,5-triméthyl-1-sila-2,3-dihydro-1*H*-indène-6-yloxy)-*N*-(2-méthylamino-4,6-diméthoxy-1,3-pyrimidine-5-yl)furanne-2-carboxamide ;
5-(1,1,5-triméthyl-1-sila-2,3-dihydro-1*H*-indène-6-yloxy)-*N*-{2-[3-(4-méthylpipérazine-1-yl)propylamino]-4,6-diméthoxy-1,3-pyrimidine-5-yl}furanne-2-carboxamide ;
5-(1,1,5-triméthyl-1-sila-2,3-dihydro-1*H*-indène-6-yloxy)-*N*-{2-[3-(*N,N*-diméthylamino)propylamino]-4,6-diméthoxy-1,3-pyrimidine-5-yl}furanne-2-carboxamide ;
5-(1,1,5-triméthyl-1-sila-2,3-dihydro-1*H*-indène-6-yloxy)-*N*-{2-[3-(morpholine-4-yl)propylamino]-4,6-diméthoxy-1,3-pyrimidine-5-yl}furanne-2-carboxamide ;
5-(1,1,5-triméthyl-1-sila-2,3-dihydro-1*H*-indène-6-yloxy)-*N*-{2-[(pyridine-2-yl)méthylamino]-4,6-diméthoxy-1,3-pyrimidine-5-yl}furanne-2-carboxamide ;
5-(1,1,6-triméthyl-1-sila-2,3-dihydro-1*H*-indène-5-yloxy)-*N*-(2,6-diméthoxyphényl)furanne-2-carboxamide ;
5-(1,1,6-triméthyl-1-sila-2,3-dihydro-1*H*-indène-5-yloxy)-*N*-(2,4,6-triméthoxyphényl)furanne-2-carboxamide ;
5-(1,1,6-triméthyl-1-sila-2,3-dihydro-1*H*-indène-5-yloxy)-*N*-(2,4,6-triméthoxy-1,3-pyrimidine-5-yl)furanne-2-carboxamide ;
5-(1,1,6-triméthyl-1-sila-2,3-dihydro-1*H*-indène-5-yloxy)-*N*-(2-méthylamino-4,6-diméthoxy-1,3-pyrimidine-5-yl)furanne-2-carboxamide ;
5-(1,1,6-triméthyl-1-sila-2,3-dihydro-1*H*-indène-5-yloxy)-*N*-{2-[3-(4-méthylpipérazine-1-yl)propylamino]-4,6-diméthoxy-1,3-pyrimidine-5-yl}furanne-2-carboxamide ;
5-(1,1,6-triméthyl-1-sila-2,3-dihydro-1*H*-indène-5-yloxy)-*N*-{2-[3-(*N,N*-diméthylamino)propylamino]-4,6-diméthoxy-1,3-pyrimidine-5-yl}furanne-2-carboxamide ;
5-(1,1,6-triméthyl-1-sila-2,3-dihydro-1*H*-indène-5-yloxy)-*N*-{2-[3-(morpholine-4-yl)propylamino]-4,6-diméthoxy-1,3-pyrimidine-5-yl}furanne-2-carboxamide ;
5-(1,1,5-triméthyl-1-sila-1*H*-indène-6-yloxy)-*N*-(2,6-diméthoxyphényl)furanne-2-carboxamide ;
5-(1,1,5-triméthyl-1-sila-1*H*-indène-6-yloxy)-*N*-(2,4,6-triméthoxyphényl)furanne-2-carboxamide ;
5-(1,1,5-triméthyl-1-sila-1*H*-indène-6-yloxy)-*N*-(2,4,6-triméthoxy-1,3-pyrimidine-5-yl)furanne-2-carboxamide ;
5-(1,1,5-triméthyl-1-sila-1*H*-indène-6-yloxy)-*N*-(2-méthylamino-4,6-diméthoxy-1,3-pyrimidine-5-yl)furanne-2-carboxamide ;
5-(1,1,5-triméthyl-1-sila-1*H*-indène-6-yloxy)-*N*-{2-[3-(4-méthylpipérazine-1-yl)propylamino]-4,6-diméthoxy-1,3-pyrimidine-5-yl}furanne-2-carboxamide ;
5-(1,1,5-triméthyl-1-sila-1*H*-indène-6-yloxy)-*N*-{2-[3-(*N,N-*diméthylamino)propylamino]-4,6-diméthoxy-1,3-pyrimidine-5-yl}furanne-2-carboxamide ;
5-(1,1,5-triméthyl-1-sila-1*H*-indène-6-yloxy)-*N*-{2-[3-(morpholine-4-yl)propylamino]-4,6-diméthoxy-1,3-pyrimidine-5-yl}furanne-2-carboxamide ;
5-(5-méthoxy-1,1-diméthyl-1-sila-1*H*-indène-6-yloxy)-*N-*(2,6-diméthoxyphényl)furanne-2-carboxamide ;
5-(5-méthoxy-1,1-diméthyl-1-sila-1*H*-indène-6-yloxy)-*N-*(2,4,6-triméthoxyphényl)furanne-2-carboxamide ;
5-(5-méthoxy-1,1-diméthyl-1-sila-1*H*-indène-6-yloxy)-*N-*(2,4,6-triméthoxy-1,3-pyrimidine-5-yl)furanne-2-carboxamide ;
5-(5-méthoxy-1,1-diméthyl-1-sila-1*H*-indène-6-yloxy)-*N-*(2-méthylamino-4,6-diméthoxy-1,3-pyrimidine-5-yl)furanne-2-carboxamide ;
5-(5-méthoxy-1,1-diméthyl-1-sila-1*H*-indène-6-yloxy)-*N-*{2-[3-(4-méthylpipérazine-1-yl)propylamino]-4,6-diméthoxy-1,3-pyrimidine-5-yl}furanne-2-carboxamide ;
5-(5-méthoxy-1,1-diméthyl-1-sila-1*H*-indène-6-yloxy)-*N-*{2-[3-(*N,N*-diméthylamino)propylamino]-4,6-diméthoxy-1,3-pyrimidine-5-yl}furanne-2-carboxamide ;
5-(5-méthoxy-1,1-diméthyl-1-sila-1*H*-indène-6-yloxy)-*N-*{2-[3-(morpholine-4-yl)propylamino]-4,6-diméthoxy-1,3-pyrimidine-5-yl}furanne-2-carboxamide ;
5-[1,1,6-triméthyl-3-(2-propyl)-1-sila-2,3-dihydro-1*H-*indène-5-yloxy]-*N*-(2,6-diméthoxyphényl)furanne-2-carboxamide ;
5-[1,1,6-triméthyl-3-(2-propyl)-1-sila-2,3-dihydro-1*H-*indène-5-yloxy]-*N*-(2,4,6-triméthoxyphényl)furanne-2-carboxamide ;
5-[1,1,6-triméthyl-3-(2-propyl)-1-sila-2,3-dihydro-1*H-*indène-5-yloxy]-*N*-(2,4,6-triméthoxy-1,3-pyrimidine-5-yl)furanne-2-carboxamide ;
5-[1,1,6-triméthyl-3-(2-propyl)-1-sila-2,3-dihydro-1*H-*indène-5-yloxy]-*N*-(2-méthylamino-4,6-diméthoxy-1,3-pyrimidine-5-yl)furanne-2-carboxamide ;
5-[1,1,6-triméthyl-3-(2-propyl)-1-sila-2,3-dihydro-1*H-*indène-5-yloxy]-*N*-{2-[3-(4-méthylpipérazine-l-yl)propylamino]-4,6-diméthoxy-1,3-pyrimidine-5-yl}furanne-2-carboxamide ;
5-[1,1,6-triméthyl-3-(2-propyl)-1-sila-2,3-dihydro-1*H-*indène-5-yloxy]-*N*-{2-[3-(*N,N*-diméthylamino)propylamino]-4,6-diméthoxy-1,3-pyrimidine-5-yl}furanne-2-carboxamide ;
5-[1,1,6-triméthyl-3-(2-propyl)-1-sila-2,3-dihydro-1*H-*indène-5-yloxy]-*N*-{2-[3-(morpholine-4-yl)propylamino]-4,6-diméthoxy-1,3-pyrimidine-5-yl}furanne-2-carboxamide ;
5-(5-chloro-1,1-diméthyl-1-sila-2,3-dihydro-1*H*-indène-6-yloxy)-*N*-(2,6-diméthoxyphényl)furanne-2-carboxamide ;
5-(5-chloro-1,1-diméthyl-1-sila-2,3-dihydro-1*H*-indène-6-yloxy)-*N*-(2,4,6-triméthoxyphényl)furanne-2-carboxamide ;
5-(5-chloro-1,1-diméthyl-1-sila-2,3-dihydro-1*H*-indène-6-yloxy)-*N*-(2,4,6-triméthoxy-1,3-pyrimidine-5-yl)furanne-2-carboxamide ;
5-(5-chloro-1,1-diméthyl-1-sila-2,3-dihydro-1*H*-indène-6-yloxy)-*N*-(2-méthylamino-4,6-diméthoxy-1,3-pyrimidine-5-yl)-furanne-2-carboxamide ;
5-(5-chloro-1,1-diméthyl-1-sila-2,3-dihydro-1*H*-indène-6-yloxy)-*N*-{2-[3-(4-méthylpipérazine-1-yl)propylamino]-4,6-diméthoxy-1,3-pyrimidine-5-yl}furanne-2-carboxamide ;
5-(5-chloro-1,1-diméthyl-1-sila-2,3-dihydro-1*H*-indène-6-yloxy)-*N*-{2-[3-(*N,N*-diméthylamino)propylamino]-4,6-diméthoxy-1,3-pyrimidine-5-yl}furanne-2-carboxamide ;
5-(5-chloro-1,1-diméthyl-1-sila-2,3-dihydro-1*H*-indène-6-yloxy)-*N*-{2-[3-(morpholine-4-yl)propylamino]-4,6-diméthoxy-1,3-pyrimidine-5-yl}furanne-2-carboxamide ;
5-(5-chloro-1,1,7-triméthyl-1-sila-2,3-dihydro-1*H*-indène-4-yloxy)-*N*-(2,6-diméthoxyphényl)furanne-2-carboxamide ;
5-(5-chloro-1,1,7-triméthyl-1-sila-2,3-dihydro-1*H*-indène-4-yloxy)-*N*-(2,4,6-triméthoxyphényl)furanne-2-carboxamide ;
5-(5-chloro-1,1,7-triméthyl-1-sila-2,3-dihydro-1*H*-indène-4-yloxy)-*N*-(2,4,6-triméthoxy-1,3-pyrimidine-5-yl)furanne-2-carboxamide ;
5-(5-chloro-1,1,7-triméthyl-1-sila-2,3-dihydro-1*H*-indène-4-yloxy)-*N*-(2-méthylamino-4,6-diméthoxy-1,3-pyrimidine-5-yl)-furanne-2-carboxamide ;
5-(5-chloro-1,1,7-triméthyl-1-sila-2,3-dihydro-1*H*-indène-4-yloxy)-*N*-{2-[3-(4-méthylpipérazine-1-yl)propylamino]-4,6-diméthoxy-1,3-pyrimidine-5-yl}furanne-2-carboxamide ;
5-(5-chloro-1,1,7-triméthyl-1-sila-2,3-dihydro-1*H*-indène-4-yloxy)-*N*-{2-[3-(*N,N*-diméthylamino)propylamino]-4,6-diméthoxy-1,3-pyrimidine-5-yl}furanne-2-carboxamide ;
5-(5-chloro-1,1,7-triméthyl-1-sila-2,3-dihydro-1*H*-indène-4-yloxy)-*N*-{2-[3-(morpholine-4-yl)propylamino]-4,6-diméthoxy-1,3-pyrimidine-5-yl}furanne-2-carboxamide ;
5-(5-méthoxy-1,1-diméthyl-1-sila-2,3-dihydro-1*H*-indène-6-yloxy)-*N*-(2,6-diméthoxyphényl)furanne-2-carboxamide ;
5-(5-méthoxy-1,1-diméthyl-1-sila-2,3-dihydro-1*H*-indène-6-yloxy)-*N*-(2,4,6-triméthoxyphényl)furanne-2-carboxamide ;
5-(5-méthoxy-1,1-diméthyl-1-sila-2,3-dihydro-1*H*-indène-6-yloxy)-*N*-(2,4,6-triméthoxy-1,3-pyrimidine-5-yl)furanne-2-carboxamide ;
5-(5-méthoxy-1,1-diméthyl-1-sila-2,3-dihydro-1*H*-indène-6-yloxy)-*N*-(2-méthylamino-4,6-diméthoxy-1,3-pyrimidine-5-yl)-furanne-2-carboxamide ;
5-(5-méthoxy-1,1-diméthyl-1-sila-2,3-dihydro-1*H*-indène-6-yloxy)-*N*-{2-[3-(4-méthylpipérazine-1-yl)propylamino]-4,6-diméthoxy-1,3-pyrimidine-5-yl}furanne-2-carboxamide ;
5-(5-méthoxy-1,1-diméthyl-1-sila-2,3-dihydro-1*H*-indène-6-yloxy)-*N*-{2-[3-(*N,N*-diméthylamino)propylamino]-4,6-diméthoxy-1,3-pyrimidine-5-yl}furanne-2-carboxamide ;
5-(5-méthoxy-1,1-diméthyl-1-sila-2,3-dihydro-1*H*-indène-6-yloxy)-*N*-{2-[3-(morpholine-4-yl)propylamino]-4,6-diméthoxy-1,3-pyrimidine-5-yl}furanne-2-carboxamide ;
5-(5-méthoxy-1,1-diméthyl-1-sila-2,3-dihydro-1*H*-indène-6-yloxy)-*N*-{2-[(pyridine-2-yl)méthylamino]-4,6-diméthoxy-1,3-pyrimidine-5-yl}furanne-2-carboxamide ;
5-[(1,1,4,4,7-pentaméthyl-1,4-disila-1,2,3,4-tétrahydronaphatalène-6-yl)méthyl]-*N*-{2-[3-(4-méthylpipérazine-1-yl)-propylamino]-4,6-diméthoxy-1,3-pyrimidine-5-yl}furanne-2-carboxamide ;
5-[(1,1,4,4,7-pentaméthyl-1,4-disila-1,2,3,4-tétrahydronaphatalène-6-yl)méthyl]-*N*-{2-[3-(*N,N*-diméthylamino)propylamino]-4,6-diméthoxy-1,3-pyrimidine-5-yl}furanne-2-carboxamide ;
5-[(1,1,4,4,7-pentaméthyl-1,4-disila-1,2,3,4-tétrahydronaphatalène-6-yl)méthyl]-*N*-{2-[3-(morpholine-4-yl)propylamino]-4,6-diméthoxy-1,3-pyrimidine-5-yl}furanne-2-carboxamide ;
5-[(1,1,3,3,6-pentaméthyl-1,3-disila-2,3-dihydro-1*H-*indène-5-yl)méthyl]-*N*-(2,6-diméthoxyphényl)furanne-2-carboxamide ;
5-[(1,1,3,3,6-pentaméthyl-1,3-disila-2,3-dihydro-1*H-*indène-5-yl)méthyl]-*N*-(2,4,6-triméthoxyphényl)furanne-2-carboxamide ;
5-[(1,1,3,3,6-pentaméthyl-1,3-disila-2,3-dihydro-1*H-*indène-5-yl)méthyl]-*N*-(2,4,6-triméthoxy-1,3-pyrimidine-5-yl)furanne-2-carboxamide ;
5-[(1,1,3,3,6-pentaméthyl-1,3-disila-2,3-dihydro-1*H-*indène-5-yl)méthyl]-*N*-(2-méthylamino-4,6-diméthoxy-1,3-pyrimidine-5-yl)furanne-2-carboxamide ;
5-[(1,1,3,3,6-pentaméthyl-1,3-disila-2,3-dihydro-1*H-*indène-5-yl)méthyl]-*N*-{2-[3-(4-méthylpipérazine-1-yl)propylamino]-4,6-diméthoxy-1,3-pyrimidine-5-yl}furanne-2-carboxamide ;
5-[(1,1,3,3,6-pentaméthyl-1,3-disila-2,3-dihydro-1*H-*indène-5-yl)méthyl]-*N*-{2-[3-(*N,N*-diméthylamino)propylamino]-4,6-diméthoxy-1,3-pyrimidine-5-yl}furanne-2-carboxamide ;
5-[(1,1,3,3,6-pentaméthyl-1,3-disila-2,3-dihydro-1*H-*indène-5-yl)méthyl]-*N*-{2-[3-(morpholine-4-yl)propylamino]-4,6-diméthoxy-1,3-pyrimidine-5-yl}furanne-2-carboxamide ;
5-[(1,1,5-triméthyl-1-sila-2,3-dihydro-1*H*-indène-6-yl)-méthyl]-*N*-(2,6-diméthoxyphényl)furanne-2-carboxamide ;
5-[(1,1,5-triméthyl-1-sila-2,3-dihydro-1*H*-indène-6-yl)-méthyl]-*N*-(2,4,6-triméthoxyphényl)furanne-2-carboxamide ;
5-[(1,1,5-triméthyl-1-sila-2,3-dihydro-1*H*-indène-6-yl)-méthyl]-*N*-(2,4,6-triméthoxy-1,3-pyrimidine-5-yl)furanne-2-carboxamide ;
5-[(1,1,5-triméthyl-1-sila-2,3-dihydro-1*H*-indène-6-yl)-méthyl]-*N*-(2-méthylamino-4,6-diméthoxy-1,3-pyrimidine-5-yl)-furanne-2-carboxamide ;
5-[(1,1,5-triméthyl-1-sila-2,3-dihydro-1*H*-indène-6-yl)-méthyl]-*N*-{2-[3-(4-méthylpipérazine-1-yl)propylamino]-4,6-diméthoxy-1,3-pyrimidine-5-yl}furanne-2-carboxamide ;
5-[(1,1,5-triméthyl-1-sila-2,3-dihydro-1*H*-indène-6-yl)-méthyl]-*N*-{2-[3-(*N,N*-diméthylamino)propylamino]-4,6-diméthoxy-1,3-pyrimidine-5-yl}furanne-2-carboxamide ;
5-[(1,1,5-triméthyl-1-sila-2,3-dihydro-1*H*-indène-6-yl)-méthyl]-*N*-[2-(3-morpholinopropylamino)-4,6-diméthoxy-1,3-pyrimidine-5-yl]furanne-2-carboxamide ;
5-[(1,1,5-triméthyl-1-sila-2,3-dihydro-1*H*-indène-6-yl)-méthyl]-*N*-{2-[(pyridine-2-yl)méthylaminol-4,6-diméthoxy-1,3-pyrimidine-5-yl}furanne-2-carboxamide ;
5-[(1,1,5-triméthyl-1-sila-2,3-dihydroindène-6-yl)méthyl]-*N*-(4-chloro-2,6-diméthoxyphényl)furanne-2-carboxamide ;
5-(1,1,3,4,4,6-hexaméthyl-1-sila-1,2,3,4-tétrahydronaphtalène-7-yloxy)-*N*-{[2-(2-(morpholine-4-yl)éthylamino)-4,6-diméthoxy-1,3-pyrimidine-5-yl]furanne-2-carboxamide ;
5-(1,1,4,4,6-pentaméthyl-1,4-disila-1,2,3,4-tétrahydronaphtalène-5-oxy)-*N*-(2,4,6-triméthoxyphényl)furanne-2-carboxamide ;
5-(7'-hydroxy-1,1,1',1',6,6'-hexaméthyl-3,3'-spiro-1,1'-disila-4,4'-dioxo-1,1',2,2',3,3',4,4'-octahydrodinaphtalène-7-yloxy)-*N*-(2,6-diméthoxyphényl)furanne-2-carboxamide ;
5-[1,1,2,6-tétraméthyl-3-(2-propyl)-1-sila-2,3-dihydroindène-5-yloxy]-*N*-(2,4,6-triméthoxyphényl)furanne-2-carboxamide ;
5-(1,1,3,4,4-pentaméthyl-1-sila-1,2,3,4-tétrahydronaphtalène-7-yloxy)-*N*-(2,4,6-triméthoxy-1,3-pyrimidine-5-yl)-furanne-2-carboxamide ;
5-(1,1,5-triméthyl-1-sila-2,3-dihydroindène-6-yloxy)-*N*-(4-chloro-2,6-diméthoxyphényl)furanne-2-carboxamide ;
5-(1,1,3,3,5-pentaméthyl-1-sila-2,3-dihydroindène-6-yloxy)-*N*-(2,6-diméthoxyphényl)furanne-2-carboxamide ;
5-(1,1,3,3,6-pentaméthyl-1-sila-2,3-dihydroindène-5-yloxy)-*N*-(2,6-diméthoxyphényl)furanne-2-carboxamide ;
5-(8-méthoxy-1,1,4,4,6-pentaméthyl-1-sila-1,2,3,4-tétrahydronaphtalène-7-yloxy)-*N*-{2-[3-(morpholine-4-yl)-*N*-propylamino]-4,6-diméthoxy-1,3-pyrimidine-5-yl}furanne-2-carboxamide ;
5-(1,1,6-triméthyl-1-sila-1,2,3,4-tétrahydronaphtalène-7-yloxy)-*N*-(2,4-diméthoxypyridine-3-yl)furanne-2-carboxamide ;
5-(1,1,3,4,4,6-hexaméthyl-1-sila-1,2,3,4-tétrahydronaphtalène-7-yloxy)-*N*-(2,4,6-triméthoxy-1,3-pyrimidine-5-yl)furanne-2-carboxamide ; et
5-(1,1,3,4,4,6-hexaméthyl-1-sila-1,2,3,4-tétrahydronaphtalène-7-yloxy)-*N*-[2-(2-*N,N*-diméthylaminoéthyl)-4,6-triméthoxy-1,3-pyrimidine-5-yl]furanne-2-carboxamide.

21. Composé suivant l'une quelconque des revendications précédentes, qui est sous forme d'un énantiomère, diastéréoisomère ou tautomère unique.

22. Composé suivant l'une quelconque des revendications précédentes, à usage thérapeutique.

23. Composition pharmaceutique comprenant un composé de l'une quelconque des revendications 1 à 21 et un diluant ou support pharmaceutiquement acceptable.

24. Utilisation d'un composé de l'une quelconque des revendications 1 à 21, pour la production d'un médicament destiné à la thérapie contre le cancer.

25. Utilisation d'un composé de l'une quelconque des revendications 1 à 21, pour la production d'un médicament destiné au traitement ou à la prévention de l'endométriose, du myome utérin, d'une maladie ovarienne, d'une maladie kystique des glandes mammaires, de l'hypertrophie prostatique, de l'aménorrhée, de la puberté précoce, du syndrome prémenstruel, d'une physiopathologie dépendant de stéroïdes sexuels ou de l'hyperplasie prostatique bénigne, ou pour interrompre la spermatogenèse.

26. Utilisation suivant la revendication 25, pour le traitement ou la prévention de l'endométriose avec une douleur, de la maladie ovarienne polykystique ou de l'aménorrhée secondaire.

27. Utilisation d'un composé de l'une quelconque des revendications 1 à 21, pour la production d'un médicament destiné au traitement ou à la prévention de la maladie d'Alzheimer.

28. Utilisation d'un composé de l'une quelconque des revendications 1 à 21, pour la production d'un médicament destiné au traitement ou à la prévention de l'infection par le VIH ou du SIDA.

29. Utilisation d'un composé de l'une quelconque des revendications 1 à 21, pour la production d'un médicament destiné au traitement ou à la prévention d'une maladie provoquée par un dysfonctionnement thymique.

30. Utilisation suivant la revendication 29, pour le traitement ou la prévention de la sclérose en plaques, de la polyarthrite rhumatoïde ou du diabète du type 1.
